# EUROPEAN PATENT APPLICATION

(11) **EP 3 912 994 A1**
(43) Date of publication of application: **24.11.2021**
(21) Application number: 21305209.5
(22) Date of filing: 19.02.2021
(51) Int. Cl.: C07K 14/78, A61K 38/39

(54) **COMPOUNDS BINDING TENASCIN-C (TNC) FOR USE IN THE TREATMENT OF DISEASES**

(30) Priority: 20.05.2020 EP 20315253
(71) Applicant: Université de Strasbourg, 67000 Strasbourg (FR); INSTITUT NATIONAL DE LA SANTE ET DE LA RECHERCHE MEDICALE - INSERM, 75013 Paris (FR)
(72) Inventor: OREND, Gertraud, 77694 KEHL (DE)
(74) Representative: Novagraaf Technologies

(57) **Abstract**

The invention relates compound binding Matrix Binding Motif (M-motif) on Tenascin-C (TNC) for use in the prevention and/or treatment of disease, a pharmaceutical compositions comprising compound binding Matrix Binding Motif (M-motif) on Tenascin-C (TNC) for use in the prevention and/or treatment of disease.

The present invention finds applications in the therapeutic medical technical fields and also in veterinary technical fields.

## Description

### FIELD

The invention relates compound binding Matrix Binding Motif (M-motif) on Tenascin-C (TNC) for use in the prevention and/or treatment of disease.

The invention also relates to pharmaceutical compositions comprising compound binding Matrix Binding Motif (M-motif) on Tenascin-C (TNC) for use in the prevention and/or treatment of disease.

The present invention finds applications in the therapeutic and diagnostic medical technical fields and also in veterinary technical fields.

### BACKGROUND

Cancer remains the most common malignancy and second-most common cause of death in the Western world. Early detection is essential for curative cancer therapy and for achieving a decrease in cancer mortality.

Improved molecular understanding of cancers has resulted in identification of various cancer cell targets for use in diagnostic and therapeutic interventions. However, tumor heterogeneity (phenotypic and genetic) is the first problem that currently challenges tumor-specific diagnosis, imaging and therapy.

Many therapies and/or process for specifically identify and/or target the tumors, in particular tumorous cells have been developed in the art. In most of the case the therapies associate surgical resection to the extent that is safely feasible, followed by radiotherapy and concomitant chemotherapy. However, the therapy protocol could be completely different with regards to a patient to another.

All these therapies have to be improved since they do not allow to effectively treat all the different cancers and have varying efficiencies due to the high diversity of cancer and the diversity of patient.

There is therefore a real need to find a method and/or a compound which allows more efficient treatment and/or effective treatment of more varied cancers. In particular there is a real need to find new strategies, i.e. new targets/pathways, in the treatment of cancer.

In addition, it has been suggested/discussed that inhibition of the body's immune defense is involved in tumor growth (Turley et al., 2015, Nat Rev Immunol 15, 669-82 [39])

The extracellular matrix (ECM) molecule Tenascin-C (TNC) plays multiple roles in cancer such as promoting tumor cell survival, proliferation, invasion, angiogenesis and metastasis (Midwood et al., 2016 [10]; Sun et al., 2019 [13]).

TNC can also shape tumor immunity by regulating the behavior of several subsets of immune cells through distinct mechanisms (Deligne et al., 2020 [4]; Huang et al., 2010 [5]; Jachetti et al., 2015 [9], Hauzenberger et al., Eur J Immunol. 1999 May; 29(5):1435-47. doi: 10.1002/(SICI)1521 - 4141(199905)29:05<1435::AID-IMMU1435>3.0.CO;2-N [36])

As recently discovered, one mechanism involves dendritic cells (DC) and in particular impairing DC function (Spenlé et al., 2020, [37]).Thus the extracellular matrix molecule Tenascin-C (TNC) can promote cancer by multiple mechanisms.

TNC also binds to several other molecules amongst them fibronectin (FN) (reviewed in Midwood et al., 2016 [10]). TNC and FN are frequently co-expressed in inflamed and tumor tissue (Van Obberghen-Schilling et al., 2011 [14]). This complicity seems to be linked to intricate but poorly understood interactions between FN and TNC that regulate cell adhesion (Chiquet-Ehrismann et al., 1988 [2]; Huang et al., 2001 [6]; Orend et al., 2003 [11]). Moreover, both proteins contain recognition sites for transmembrane receptors (e.g. integrins, syndecans) through which cells receive information from these ECM molecules that shape their behavior. Furthermore, similar to FN also TNC binds soluble factors thereby potentially representing a reservoir of growth factors regulating cell behavior (De Laporte et al., 2013 [3]; Hynes, 2009 [7]; Midwood et al., 2016 [10]). A detailed analysis revealed that TNC-binding domains in FN are located inside the three heparin binding sites (HBS), in FN-Hepl (N-terminal 29KDa domain), FN-Hepll (fibronectin type III repeat (FNIII) 13, FN13) and FN-Heplll (FN4-6) (Huang et al., 2001 [5]; Ingham et al., 2004 [8]).

There is therefore a real need to find a method and/or a compound which allows more efficient treatment and/or effective treatment of varied cancers, for example by targeting molecules involved in tumor immunity and/or inflammation of tissues and/or particular biological mechanisms which appear in cancer. In particular there is a real need to find new strategies, i.e. new targets/pathways, in the treatment of cancer.

There is also a real need to find a method and/or a compound which allows to the treatment of disease, for example by reactivating this immune defense, for example by eliciting an anti-tumor immunity response.

### Description of the invention

The present invention meets these needs and overcomes the abovementioned drawbacks of the prior art by providing compounds binding the Matrix Binding Motif (M-motif) on Tenascin-C (TNC).

In particular, the present invention meets these needs and overcomes the abovementioned drawbacks of the prior art by providing compounds binding the Matrix Binding Motif (M-motif) on Tenascin-C (TNC) for use as a medicament.

In addition, the present invention meets these needs and overcomes the abovementioned drawbacks of the prior art by providing compounds binding the Matrix Binding Motif (M-motif) on Tenascin-C (TNC) for use in the prevention and/or treatment of disease.

In particular, the compound binding Matrix Binding Motif (M-motif) on Tenascin-C (TNC) of according to the invention allows to bind Tenascin-C. In particular, the inventor has demonstrated that the compound binding Matrix Binding Motif (M-motif) on Tenascin-C (TNC) of the present invention allows to modify the quaternary structure of Tenascin-C. In addition the inventors have demonstrated that the compound binding Matrix Binding Motif (M-motif) on Tenascin-C (TNC) of the present invention allows to modify the conformation of Tenascin-C and thus allow the release of molecule(s) bound and/or linked to the Tenascin-C, for example chemokines bound to Tenascin-C.

Moreover, the inventor has demonstrated and identify a common TNC binding motif, also mentioned as Matrix Binding Motif (M-Motif), on TNC, in particular inside the fifth FNIII domain of TNC (TN5) that impacted binding of chemokines. In addition, the inventors demonstrate that this Matrix Binding Motif (M-Motif) mediates the interaction with Fibronectin (FN). The inventors also surprisingly demonstrated that compound binding on Matrix Binding Motif (M-Motif) according to the invention modify the binding of chemokines to TNC in an allosteric manner.

The inventors have also clearly demonstrated that compound binding Matrix Binding Motif (M-motif) on Tenascin-C (TNC) of the present invention blocked the interaction of Tenascin-C (TNC) with Fibronectin (FN) and cell rounding by TNC on the FN/TNC substratum. The inventors have also clearly demonstrated that compound binding Matrix Binding Motif (M-motif) on Tenascin-C (TNC) compete, in an allosteric manner, chemokine binding to TNC and chemoretention of dendritic cells by TNC.

In addition, the inventors have demonstrated that compound binding Matrix Binding Motif (M-motif) on Tenascin-C (TNC) may block TNC actions, for example in forming matrix networks, and its binding to immune modulatory molecules. The inventors have also demonstrated that compound binding Matrix Binding Motif (M-motif) on Tenascin-C (TNC) may block the immuno-suppressive properties of the immuno-suppressive tumour microenvironment (TME).

The compound binding Matrix Binding Motif (M-motif) on Tenascin-C (TNC) might also inactivate the TNC drawbacks with regards to tumour immunity, metastasis, inflammatory and fibrotic diseases.

In other words, the inventors have demonstrated that compound binding Matrix Binding Motif (M-motif) on tenascin-C (TNC) according to the invention allow to activate the immune response, in particular in an immuno-suppressive tumour microenvironment. In particular, the inventors have demonstrated that compound binding Matrix Binding Motif (M-motif) on tenascin-C (TNC) according to the invention allow to activate the immune response in an immuno-suppressive tumour microenvironment such as chemoretention of dendritic cells by a TNC/CCL21 and TNC/CXCL12 complex and activation of Tumor necrosis factor (TNF)-related apoptosis-induced ligand (TRAIL)-induced tumor cell death.

In other words, the inventors have demonstrated that compound binding Matrix Binding Motif (M-motif) on Tenascin-C (TNC) according to the invention allows to inhibit matrix network formation and block TGFβ signalling. Advantageously the inventors have demonstrated that compound binding Matrix Binding Motif (M-motif) on Tenascin-C (TNC) allows to inhibit matrix network formation and block TGFβ signalling in fibrotic conditions such as Chronic Kidney Disease and other fibrotic diseases such as liver and lung fibrosis.

### Definitions

To facilitate an understanding of the present invention, a number of terms and phrases are defined below:
As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof" "a mixture thereof" and such like following a listing, the use of "and/or" as part of a listing, a listing in a table, the use of "etc." as part of a listing, the phrase "such as," and/or a listing within brackets with "e.g.," or i.e., refers to any combination (e.g., any sub-set) of a set of listed components, and combinations and/or mixtures of related species and/or embodiments described herein though not directly placed in such a listing are also contemplated. Such related and/or like genera(s), subgenera(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."

As used herein, the term "and/or" means any one of the items, any combination of the items, or all of the items with which this term is associated.

As used herein, the term "about" refers to a variation of ±5-10% of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., weight percents, proximate to the recited range that are equivalent in terms of the functionality of the individual ingredient, the composition, or the embodiment.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range (e.g., weight percents) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to," "at least," "greater than," "less than," "more than," "or more," and the like, include the number recited and such terms refer to ranges that can be subsequently broken down into subranges as discussed above. In the same manner, all ratios recited herein also include all sub-ratios falling within the broader ratio

An "effective amount" refers to an amount effective to treat a disease, disorder, and/or condition, or to bring about a recited effect. For example, an amount effective can be an amount effective to reduce the progression or severity of the condition or symptoms being treated. Determination of a therapeutically effective amount is well within the capacity of persons skilled in the art. The term "effective amount" is intended to include an amount of a compound described herein, or an amount of a combination of compounds described herein, e.g., that is effective to treat or prevent a disease or disorder, or to treat the symptoms of the disease or disorder, in a host. Thus, an "effective amount" generally means an amount that provides the desired effect.

The terms "treating", "treat" and "treatment" include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, therapeutic, and/or prophylactic administration, as appropriate.

The terms "regulating" include (i) inhibiting the disease, pathologic or medical condition or arresting its development; (ii) relieving the disease, pathologic or medical condition; and/or (iii) diminishing symptoms associated with the disease, pathologic or medical condition.

In the present "inflammation" refers to a biological response to harmful stimuli, such as pathogens, damaged cells, or irritants. As such, inflammation is a component of the nonspecific defense system, for example inflammation is one of the responses by an infected subject to a pathogen or one of the response by a subject to an abnormal biological mechanism, for example damaged cells.

In the present, in the peptide sequences disclosed herein, the amino acids are represented by their one letter code according to the following nomenclature: A: alanine; C: cysteine; D: aspartic acid; E: glutamic acid; F: phenylalanine; G: glycine; H: histidine; I: isoleucine; K: lysine; L: leucine ; M: methionine ; N: asparagine ; P: proline ; Q: glutamine ; R: arginine ; S: serine ; T: threonine ; V: valine ; W: tryptophan and Y: tyrosine.

In the present, the term "Immune response" refers to, for example lymphocytes, antigen presenting cells, phagocytic cells, granulocytes, and soluble polymer produced by the cells or liver (antibodies, cytokines and a complement) of, invasive pathogens, cells or tissues infected with a pathogen, cancer cell, or autoimmune or pathological inflammation in the case of normal human cells or tissue, and selectively injury, break, or the act of removing from the human body.

In the present, Tenascin-C (TNC) refers to a glycoprotein that in humans is encoded by the TNC gene located on chromosome 9 with location of the cytogenic band at the 9q33 (Nies DE, Hemesath TJ, Kim JH, Gulcher JR, Stefansson K (March 1991). "The complete cDNA sequence of human hexabrachion (Tenascin). A multidomain protein containing unique epidermal growth factor repeats". J Biol Chem. 266 (5): 2818-23. PMID 1704365 [16], Siri A, Carnemolla B, Saginati M, Leprini A, Casari G, Baralle F, Zardi L (May 1991). "Human tenascin: primary structure, pre-mRNA splicing patterns and localization of the epitopes recognized by two monoclonal antibodies". Nucleic Acids Res. 19 (3): 525-31. doi:10.1093/nar/19.3.525. PMC 333643. PMID 1707164 [17]).Tenascin-C is known to be expressed in a context dependent manner and has effects in tissue homeostasis and diseases (Midwood et al., 2016, JCS [10]). The amino acid sequence of Tenacin-C may be a sequence having at least 80%, for example at least 90% of homology with sequence : MGAMTQLLAGVFLAFLALATEGGVLKKVIRHKRQSGVNATLPEENQPVVFN HVYNIKLPVGSQCSVDLESASGEKDLAPPSEPSESFQEHTVDGENQIVFTH RINIPRRACGCAAAPDVKELLSRLEELENLVSSLREQCTAGAGCCLQPATG RLDTRPFCSGRGNFSTEGCGCVCEPGWKGPNCSEPECPGNCHLRGRCID GQCICDDGFTGEDCSQLACPSDCNDQGKCVNGVCICFEGYAGADCSREIC PVPCSEEHGTCVDGLCVCHDGFAGDDCNKPLCLNNCYNRGRCVENECVC DEGFTGEDCSELICPNDCFDRGRCINGTCYCEEGFTGEDCGKPTCPHACH TQGRCEEGQCVCDEGFAGVDCSEKRCPADCHNRGRCVDGRCECDDGFT GADCGELKCPNGCSGHGRCVNGQCVCDEGYTGEDCSQLRCPNDCHSRG RCVEGKCVCEQGFKGYDCSDMSCPNDCHQHGRCVNGMCVCDDGYTGE DCRDRQCPRDCSNRGLCVDGQCVCEDGFTGPDCAELSCPNDCHGQGRC VNGQCVCHEGFMGKDCKEQRCPSDCHGQGRCVDGQCICHEGFTGLDCG QHSCPSDCNNLGQCVSGRCICNEGYSGEDCSEVSPPKDLVVTEVTEETVN LAWDNEMRVTEYLVVYTPTHEGGLEMQFRVPGDQTSTIIQELEPGVEYFIR VFAILENKKSIPVSARVATYLPAPEGLKFKSIKETSVEVEWDPLDIAFETWEII FRNMNKEDEGEITKSLRRPETSYRQTGLAPGQEYEISLHIVKNNTRGPGLK RVTTTRLDAPSQIEVKDVTDTTALITWFKPLAEIDGIELTYGIKDVPGDRTTID LTEDENQYSIGNLKPDTEYEVSLISRRGDMSSNPAKETFTTGLDAPRNLRRV SQTDNSITLEWRNGKAAIDSYRIKYAPISGGDHAEVDVPKSQQATTKTTLTG LRPGTEYGIGVSAVKEDKESNPATINAATELDTPKDLQVSETAETSLTLLWK TPLAKFDRYRLNYSLPTGQWVGVQLPRNTTSYVLRGLEPGQEYNVLLTAEK GRHKSKPARVKASTEQAPELENLTVTEVGWDGLRLNWTAADQAYEHFIIQV QEANKVEAARNLTVPGSLRAVDIPGLKAATPYTVSIYGVIQGYRTPVLSAEA STGETPNLGEVVVAEVGWDALKLNWTAPEGAYEYFFIQVQEADTVEAAQN LTVPGGLRSTDLPGLKAATHYTITIRGVTQDFSTTPLSVEVLTEEVPDMGNL TVTEVSWDALRLNWTTPDGTYDQFTIQVQEADQVEEAHNLTVPGSLRSMEI PGLRAGTPYTVTLHGEVRGHSTRPLAVEVVTEDLPQLGDLAVSEVGWDGL RLNWTAADNAYEHFVIQVQEVNKVEAAQNLTLPGSLRAVDIPGLEAATPYR VSIYGVIRGYRTPVLSAEASTAKEPEIGNLNVSDITPESFNLSWMATDGIFET FTIEIIDSNRLLETVEYNISGAERTAHISGLPPSTDFIVYLSGLAPSIRTKTISAT ATTEALPLLENLTISDINPYGFTVSWMASENAFDSFLVTVVDSGKLLDPQEF TLSGTQRKLELRGLITGIGYEVMVSGFTQGHQTKPLRAEIVTEAEPEVDNLL VSDATPDGFRLSWTADEGVFDNFVLKIRDTKKQSEPLEITLLAPERTRDITGL REATEYEIELYGISKGRRSQTVSAIATTAMGSPKEVIFSDITENSATVSWRAP TAQVESFRITYVPITGGTPSMVTVDGTKTQTRLVKLIPGVEYLVSIIAMKGFE ESEPVSGSFTTALDGPSGLVTANITDSEALARWQPAIATVDSYVISYTGEKV PEITRTVSGNTVEYALTDLEPATEYTLRIFAEKGPQKSSTITAKFTTDLDSPR DLTATEVQSETALLTWRPPRASVTGYLLVYESVDGTVKEVIVGPDTTSYSLA DLSPSTHYTAKIQALNGPLRSNMIQTIFTTIGLLYPFPKDCSQAMLNGDTTSG LYTIYLNGDKAEALEVFCDMTSDGGGWIVFLRRKNGRENFYQNWKAYAAG FGDRREEFWLGLDNLNKITAQGQYELRVDLRDHGETAFAVYDKFSVGDAK TRYKLKVEGYSGTAGDSMAYHNGRSFSTFDKDTDSAITNCALSYKGAFWY RNCHRVNLMGRYGDNNHSQGVNWFHWKGHEHSIQFAEMKLRPSNFRNL EGRRKRA (SEQ ID NO 112). Preferably, the amino acid sequence of Tenacin-C is of sequence SEQ ID NO 112.

In the present, Matrix Binding Motif (M-motif) on Tenascin-C means a binding site on Tenascin-C comprise a sequence of 4 and 8 amino acids (AA) that were separated by at least 40 AA, for example 41 or 44 amino acid. The Matrix Binding Motif (M-motif) on Tenascin-C preferably forms a niche in a 3D representation of the Matrix Binding Motif (M-motif) on Tenascin-C.

In other words the quaternary structure of The Matrix Binding Motif (M-motif) on Tenascin-C preferably forms a niche. The amino acid sequence of Matrix Binding Motif (M-motif) on Tenascin-C may be for example selected in the group comprising TDTTALITWFKPLAEIDGIELTYGIKDVPGDRTTIDLTEDENQYSIGNLKPDTE Y (SEQ ID NO 1), TDNSITLEWRNGKAAIDSYRIKYAPISGGDHAEVDVPKSQQATTKTTLTGLR PGTEY (SEQ ID NO 2) and AETSLTLLWKTPLAKFDRYRLNYSLPTGQWVGVQLPRNTTSYVLRGLEPGQ EY (SEQ ID NO 3), preferably the amino acid sequence of Matrix Binding Motif (M-motif) on Tenascin-C is

AETSLTLLWKTPLAKFDRYRLNYSLPTGQWVGVQLPRNTTSYVLRGLEPGQ
EY (SEQ ID NO 3).

In the present compound binding Matrix Binding Motif (M-motif) on Tenascin-C (TNC) may be any compound known from one skilled in the art adapted to bind to the Matrix Binding Motif (M-motif) on Tenascin-C (TNC). It may be for example compound selected from the group comprising aptamers, antibodies, nanobodies or single-domain antibodies, peptides peptidomimetics and chemical compounds.Compound binding Matrix Binding Motif (M-motif) on Tenascin-C (TNC) may be preferably selected from the group comprising a peptide and single-domain antibody ('nanobodie"). More preferably compound binding Matrix Binding Motif (M-motif) on Tenascin-C (TNC) may be a peptide.

In the present an aptamer refers to a nucleic acid molecule having a binding affinity for a particular target molecule. The aptamer can also inhibit the activity of a particular target molecule by binding to the particular target molecule. In the present, the aptamer according to the invention has a binding affinity to the Matrix Binding Motif (M-motif) on Tenascin-C, preferably to the amino acid sequence of Matrix Binding Motif (M-motif) as disclosed above.

The aptamer of the present invention can be an RNA, a DNA, a modified nucleic acid or a mixture thereof. The aptamer of the present invention may also be in a linear, circular form or mixture thereof.

The aptamer according to the present invention may arbitrarily comprise a base analog, another artificial base, another modified base, or the like. The base analog, another artificial base, another modified base, or the like may be any base analog, another artificial base, another modified base, or the like know, to one skilled on the art.

The aptamer may comprise any adapted chemical and/or structural modification known to one skilled in the art. It may be for example at least one sugar residue been modified, for example a nucleotide comprising a hydroxyl group at the 2' position of ribose, a hydroxyl group at the 2' position of ribose substituted by any adapted chemical group known to one skilled in the art, at least one modified pyrimidine nucleotide, at least one modified and/or substituted phosphate group contained in the aptamer. The chemical and/or structural modification may be carried out by any method know to one skilled in the art.

A method for producing the aptamer according to the present invention is not particularly limited. Any method known in the art by the skilled person may be employed. For example, the aptamer according to the present invention can be chemically synthesized based in accordance with a known solid-phase synthesis method. Regarding a method of chemical synthesis of nucleic acids, see, for example, Current Protocols in Nucleic Acid Chemistry, Volume 1, Section 3. Many life science manufacturers (e.g., Takara Bio Inc. and Sigma-Aldrich Corporation). An aptamer may be prepared by synthesizing several fragments based on the aptamer sequence and then ligating the fragments via, for example, intramolecular annealing or ligation by a ligase.

The term "antibody" is used herein in the broadest sense. According to the present invention, "antibody" refers to any polypeptide which at least comprises (i) a Fc region and (ii) a binding polypeptide domain derived from a variable domain of an immunoglobulin. The said binding polypeptide domain is able to bind specifically one given target antigen or a group of target antigens. In the present, the antibody according to the invention has a binding affinity to the Matrix Binding Motif (M-motif) on Tenascin-C, preferably to the amino acid sequence of Matrix Binding Motif (M-motif) as disclosed above. A binding polypeptide domain which derives from a variable region of an immunoglobulin comprises at least one or more CDRs. Herein, antibodies include, but are not limited to, full-length immunoglobulins, monoclonal antibodies, a VHH domain (also named single heavy chain domain or Nanobody®), multi-specific antibodies, Fc-fusion protein comprising at least one variable region, synthetic antibodies (sometimes referred to herein as "antibody mimetics"), chimeric antibodies. Antibodies also encompass antibody-fusion proteins, antibody conjugates and fragments of each respectively. Accordingly, a variant antibody of the invention comprises, in its Fc region, at least one amino acid modification that increase its binding affinity for Matrix Binding Motif (M-motif) on Tenascin-C (TNC) as compared to its parent antibody. Of particular interest are antibody variants which have increased in vivo half-lives as compared to parent polypeptides.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Harlow et al.: "Antibodies: A Laboratory Manual", Cold Spring Harbor Laboratory Press, 2nd ed. 1988 [19]; Hammerling, et al.: "Monoclonal Antibodies and T-Cell Hybridomas", Elsevier, N. Y., 1981, pp. 563-681 [20]).

The term "monoclonal antibody" as used herein is not limited to antibodies produced through hybridoma technology, and refers to an antibody that may be derived from a single B cell, a single clone, including any eukaryotic, prokaryotic, or phage clone, and not the method by which it is produced.

Methods for producing and screening for specific antibodies using hybridoma technology are routine and well known in the art. In a non-limiting example, mice can be immunized with an antigen of interest or a cell expressing such an antigen.

In one embodiment, a variant antibody of the invention may be selected from the group consisting of variants of parent full-length antibodies. By "full length antibody" herein is meant the structure that constitutes the natural biological form of an antibody, including variable and constant regions. The parent polypeptide of a full-length antibody variant of the present invention can be a wild-type antibody, a mutant of a wild-type antibody (e.g. comprising pre-existing modifications), an engineered version of a wild-type antibody, this list not being limitative. The structure of a full-length antibody is generally a tetramer. Each tetramer is typically composed of two identical pairs of polypeptide chains, each pair having one "light" (typically having a molecular weight of about 25 kDa) and one "heavy" chain (typically having a molecular weight of about 50-70 kDa).

In another embodiment, the antibody is a minibody. Minibodies are minimized antibody-like proteins comprising a scFv joined to a CH3 domain. In some cases, the scFv can be joined to a full-length Fc region, and may also include the hinge region or fragment thereof. In one embodiment, the antibodies of the invention are selected from the group of multispecific antibodies, and notably from the group of bispecific antibodies which are sometimes referred to as "diabodies". These antibodies bind to two (or more) different antigens. Diabodies can be manufactured in a variety of ways known in the art, e.g., chemically prepared or derived from hybridomas.

Covalent modifications of antibodies are also included within the scope of this invention, and are generally, but not always, done post-translationally. Such modifications include, but are not limited to, glycosylates, labelling and conjugation. The term "labeling group" means any detectable label, which is a compound and/or element that can be detected due to its specific functional properties, and/or chemical characteristics, the use of which allows the antibody to which it is attached to be detected, and/or further quantified if desired. In some embodiments, the labeling group is coupled to the antibody via spacer arms of various lengths to reduce potential steric hindrance. Various methods for labeling proteins are known in the art and may be used in performing the present invention. In general, labels fall into a variety of classes, depending on the assay or on the diagnostic procedure in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

In the present, single-domain antibody or nanobody comprises a variable region primarily responsible for antigen recognition and binding and a framework region. The "variable region," also called the "complementarity determining region" (CDR), comprises loops which differ extensively in size and sequence based on antigen recognition. CDRs are generally responsible for the binding specificity of the single-domain antibody or nanobody. Distinct from the CDRs is the framework region. The framework region is relatively conserved and assists in overall protein structure. The framework region may comprise a large solvent-exposed surface consisting of a β-sheet and loop structure. Preferably, single-domain antibodies or nanobodies comprise the VHH domain of the antibody disclosed above.

In the present, nanobody or single-domain antibody may naturally specifically bind a target protein, preferably Matrix Binding Motif (M-motif) on Tenascin-C (TNC), or the nanobody or single-domain antibody may be modified to specifically bind a target protein, preferably Matrix Binding Motif (M-motif) on Tenascin-C (TNC). A nanobody or single-domain antibody that naturally specifically binds a target protein may be obtained by immunizing a subject capable of producing a nanobody or single-domain antibody with a target protein and isolating a nanobody or single-domain antibody from the serum of the subject. Alternatively, CDRs known to specifically bind a target protein may be grafted onto a nanobody or single-domain antibody framework region. The assignment of amino acid sequences to each CDR may be carried out as disclosed in Kabat "Sequences of Proteins of Immunological Interest" National Institutes of Health, Bethesda, Md., 1987 and 1991 [21]; Chothia, et al, J. Mol. Bio. (1987) 196:901-917 [22]; Chothia, et al., Nature (1989) 342:878-883 [23]. Screening Methods for identifing a nanobody or single-domain antibody that specifically binds to a target protein are well known in the art. It a may be for example HTS screening method. Still further, *in-vitro* evolution methods may be used to generate a nanobody or single-domain antibody that specifically binds a target protein. The phrase "in vitro evolution" generally means any method of selecting for a nanobody or single-domain antibody that binds to a target protein. In vitro evolution is also known as "in vitro selection", "SELEX," or "systematic evolution of ligands by exponential enrichment." For example, *in-vitro* evolution may involves screening a pool of random nanobodies or single-domain antibodies for a particular nanobody or single-domain antibody that binds to a target protein or has a particular activity that is selectable. Accordingly, in vitro evolution is used to generate nanobodies or single-domain antibody that specifically bind to distinct epitopes of any given target protein.

According to the invention, the targeted protein or the target molecule is Tenascin-C (TNC), preferably the Matrix Binding Motif (M-motif) on Tenascin-C (TNC) and more preferably the amino acid sequence of Matrix Binding Motif (M-motif) on Tenascin-C.

Another object of the invention relates to a nucleic acid encoding an antibody of the invention, a nanobody or single-domain antibody of the invention.

Another object of the invention relates to an expression vector comprising a nucleic acid encoding an antibody of the invention, a nanobody or single-domain antibody of the invention.

Another object of the invention relates to a stable cell line producing an antibody of the invention, a nanobody or single-domain antibody of the invention comprising the expression vector of the invention.

The stable cell line producing the antibody, a nanobody or single-domain antibody of the invention, and comprising the expression vector as defined above, may be any adapted stable cell line known from one skilled in the art. It may be a stable cell line selected from the group comprising CHO cells, HEK cells.

Nucleic acid encoding antibody of the invention, a nanobody or single-domain antibody of the invention, or the Fc-fusion protein of the invention may be obtained by standard molecular biology or biochemistry techniques, such as DNA chemical synthesis, PCR amplification or cDNA cloning and can be inserted into expression vectors such that the genes are operatively linked to transcriptional and translational control sequences.

In the present, the term "peptide" refers to a linear molecule which is formed by the manner in which the amino acid residues bind together by a peptide bond.

In the present, the compound binding Matrix Binding Motif (M-motif) on Tenascin-C (TNC) may be for example a peptide of general formula A₁-B₁-C₁ (I) wherein
A₁ is an amino acids chain of sequence TXaXbT (SEQ ID NO 4) or STXaXbT (SEQ ID NO 5) or XgXjXkXh (SEQ ID NO 6) or SXgXjXkXh (SEQ ID NO 7)
B₁ is a linker having an amino acid sequence comprising 1 to 50 amino acid,
C₁ is an amino acids chain of sequence XcLQPXdXeXfYS (SEQ ID NO 8), XcLQPXdXeXfY (SEQ ID NO 9), XcLXiPXmXnXoY (SEQ ID NO 10) or XcLXiPXmXnXoYS (SEQ ID NO 11)
   Xa is D or E or T or A,
   Xb is S or T or N,
   Xc is N or G or E or D,
   Xd is G or A,
   Xe is S or T or Q or V or M,
   Xf is E or D,
   Xg is T or A or D or S or R or G,
   Xh is T or S or Q or K,
   Xi is Q or E or R or H or V or L or P,
   Xj is D or E or T or A or L or S or N or P,
   Xk is S or T or N or D or Y or H or R,
   Xm is G or A or E or D or N or W or Y or F,
   Xn is S or T or Q or V or E or M or R or K or C
   Xo is E or D or S or L or K

According to the invention, amino acids chain A₁ may be of sequence XpXaXbXq (SEQ ID NO 12) or SXpXaXbXq (SEQ ID NO 13) wherein Xa is D or E or T or A, Xb is S or T or N, Xp is T or A or D or S or R, Xq is T or S.

According to the invention, amino acids chain A₁ may be of sequence selected from the group comprising TDST (SEQ ID NO 14), STDST (SEQ ID NO 15), TETT (SEQ ID NO 16) or STETT (SEQ ID NO 17), TETS (SEQ ID NO 18), AETS (SEQ ID NO 19), TDNS (SEQ ID NO 20), TENS (SEQ ID NO 21), TENT (SEQ ID NO 22), TATS (SEQ ID NO 23), DENT (SEQ ID NO 24), STST (SEQ ID NO 25), TTDS (SEQ ID NO 26), RATS (SEQ ID NO 27), and TDTT (SEQ ID NO 28), SLTS (SEQ ID NO 29), SSYS (SEQ ID NO 30), ANTS (SEQ ID NO 31), AAHQ (SEQ ID NO 32), GSRK (SEQ ID NO 33). Preferably, amino acids chain C₁ of peptide of general formula A₁-B₁-C₁ (I) may be selected from the group comprising TDST (SEQ ID NO 14), STDST (SEQ ID NO 15), TETT (SEQ ID NO 16) or STETT (SEQ ID NO 17).

According to the invention, B₁ may be any linker adapted known from one skilled in the art. It may be for example an amino acids chain, for example comprising from 1 to 50 amino acids, for example from 1 to 45 amino acids. It may be for example an amino acids chain selected from the group comprising VLVRWTPPRAQITGYRLTVGLTRRGQPRQYNVGPSVSKYPLR (SEQ ID NO 34), ITISWRTKTETITGFQVDAVPANGQTPIQRTIKPDVRSYTIT (SEQ ID NO 35), ITITWRTKTETITGFQIDAIPAASGQNPIQRTISPDVRTYTIT (SEQ ID NO 36) or SAPAS (SEQ ID NO 37). It may be preferably an amino acids chain comprising at least 3 amino acids, for example comprising 5 amino acids, for example B may be of sequence SAPAS (SEQ ID NO 37).

According to the invention, amino acids chain C₁ may also be of sequence XcLXiPXdXeXfY (SEQ ID NO 38) or XcLXiPXdXeXfYS (SEQ ID NO 39) wherein Xc is N or G or E or D, Xd is G or A, Xe is S or T or Q or V or M, Xf is E or D and Xi is Q or E or R or H or V or I or P.

According to the invention, amino acids chain C₁ may be of sequence selected from the group comprising NLQPASEY (SEQ ID NO 40), NLQPASEYS (SEQ ID NO 41), GLQPGTDY (SEQ ID NO 42), GLQPGTDYS (SEQ ID NO 43), GLEPGQEY (SEQ ID NO 44), GLRPGTEY (SEQ ID NO 45), GLHPGTEY (SEQ ID NO 46), GLRPGVEY (SEQ ID NO 47), GLKPGEEY (SEQ ID NO 48), DLVPGTEY (SEQ ID NO 49), ELVPETEY (SEQ ID NO 50), NLQPDTSY (SEQ ID NO 51), GLLPNTEY (SEQ ID NO 52), GLEPGTEY (SEQ ID NO 53), DLIPWMEY (SEQ ID NO 54), ELNPWVEY (SEQ ID NO 55), ELVPGRLY (SEQ ID NO 56), DLTPGKKY (SEQ ID NO 57) GLVPGRKY (SEQ ID NO 58) and NLKPYTKY (SEQ ID NO 77). Preferably, amino acids chain C₁ of peptide of general formula A₁-B₁-C₁ (I) may be of sequence selected from the group comprising NLQPASEY (SEQ ID NO 40), NLQPASEYS (SEQ ID NO 41), GLQPGTDY (SEQ ID NO 42), GLQPGTDYS (SEQ ID NO 43),

According to the invention, the compound binding Matrix Binding Motif (M-motif) on Tenascin-C (TNC) may be a peptide of sequence selected from the group comprising TDSTSAPASNLQPASEY (SEQ ID NO 59) STDSTSAPASNLQPASEYS (SEQ ID NO 60), TETTSAPASGLQPGTDY (SEQ ID NO 61) and STETTSAPASGLQPGTDYS (SEQ ID NO 62).

According to the invention, the peptide binding Matrix Binding Motif (M-motif) on Tenascin-C (TNC) according to the invention may comprise modification at its N-terminus and/or C-terminus. It may be any modification known to one skilled in the art adapted to be on the N-terminus and/or C-terminus end of a peptide. It may be for example adding to an end a polyethyleneglycol, amino acid, peptide, inverted dT, nucleic acid, nucleosides, Myristoyl, Lithocolic-oleyl, Docosanyl, Lauroyl, Stearoyl, Palmitoyl, Oleoyl, Linoleoyl, other lipids, steroids, cholesterol, caffeine, vitamins, pigments, fluorescent substances, anticancer agent, toxin, enzymes, radioactive substance, biotin.

According to the invention the peptide binding Matrix Binding Motif (M-motif) on Tenascin-C (TNC) according to the invention may comprise at its N-terminus and/or C-terminus covalently linked nanoparticles, It may be for example any adapted nanoparticle known from one skilled in the art, it may be for example nanoparticles selected from the group comprising tobacco mosaic virus coat protein derived nanoparticles, nanoparticles composed of poly(lactic-co-glycolic acid) (PLGA), Poly(beta-amino) esters (PBAEs). It may be for example ultrasmall nanoparticles, for example nanoparticle having a size less than 5 nm. It may be for example gadolinium-based ultrasmall nanoparticles having a size less than 5 nm. It may be for example nanoparticles as disclosed in Alexandre Detappe et al. Anti-MUC1-C Conjugated Nanoparticles Potentiate the Efficacy of Fractionated Radiation Therapy, Int J Radiat Oncol Biol Phys 2020 Dec 1 ;108(5):1380-1389 [60] and/or in Rachel J. Fields a, Christopher J. Cheng a, Elias Quijano a, Caroline Weller a, Nina Kristofik a, Nha Duong a, Christopher Hoimes a, Marie E. Egan b, W. Mark Saltzman, Surface modified poly(β amino ester)-containing nanoparticles for plasmid DNA delivery. Journal of Controlled Release 164 (2012) 41-48 [61] and /or in Gamper C, et al. 2019, Functionalized Tobacco Mosaic Virus Coat Protein Monomers and Oligomers as Nanocarriers for Anti-Cancer Peptides, Cancers 11, 1609 [62].

According to the invention, the peptide binding Matrix Binding Motif (M-motif) on Tenascin-C (TNC) disclosed herein can comprise synthetic amino acids in place of one or more naturally-occurring amino acids. It may be any synthetic amino acids known to one skilled in the art, it may be for example aminocyclohexane carboxylic acid, norleucine, α-amino n-decanoic acid, homoserine, S-acetylaminomethyl-cysteine, trans-3- and trans-4-hydroxyproline, 4- aminophenylalanine, 4-nitrophenylalanine, 4-chlorophenylalanine, 4-carboxyphenylalanine, β- phenylserine β-hydroxyphenylalanine, phenylglycine, a-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'- dibenzyl-lysine, 6-hydroxylysine, ornithine, a-aminocyclopentane carboxylic acid, a- aminocyclohexane carboxylic acid, a-aminocycloheptane carboxylic acid, a-(2-amino-2- norbornane)-carboxylic acid, α,γ-diaminobutyric acid, α,β-diaminopropionic acid, homophenylalanine, and a-tert-butylglycine.

In the present the peptide binding Matrix Binding Motif (M-motif) on Tenascin-C (TNC) disclosed herein may comprise any post-translational modifications known to one skilled in the art. It may comprise, for example one or more amino acids with post-translational modification. The post-translational modification of one or more amino acids may be for example phosphorylation, acylation including acetylation and formylation, glycosylation (including N-linked and O-linked), amidation, hydroxylation, alkylation including methylation and ethylation, ubiquitylation, addition of pyrrolidone carboxylic acid, formation of disulfide bridges, sulfation, myristoylation, palmitoylation, isoprenylation, farnesylation, geranylation, glypiation, lipoylation and iodination.

According to the invention, the peptide binding Matrix Binding Motif (M-motif) on Tenascin-C (TNC) disclosed herein can comprises one or more non-naturally occurring amino acids, for example, one or more D-amino acids and/or chemically modified amino acids.

The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, be chemically modified amino acids and for example may comprise alkyl, aryl, or alkylaryl groups not found in natural amino acids. Some examples of non-natural alkyl amino acids include α-aminobutyric acid, β-aminobutyric acid, γ-aminobutyric acid, 8-aminovaleric acid, and ε-aminocaproic acid. Some examples of non-natural aryl amino acids include ortho-, meta-, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid. For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C1-C4 alkyl, such as methyl, ethyl, n-propyl, isopropyl, butyl, isobutyl, or t-butyl, C1-C4 alkyloxy (i.e., alkoxy), amino, C1-C4 alkylamino and C1-C4 dialkylamino (e.g., methylamino, dimethylamino), nitro, hydroxyl, halo (i.e., fluoro, chloro, bromo, or iodo). Some specific examples of non-naturally occurring derivatives of naturally occurring amino acids include norvaline (Nva) and norleucine (Nle).

A method for producing the peptide according to the present invention is not particularly limited. Any method known in the art by the skilled person may be employed. For example, the peptide according to the present invention can be chemically synthesized based in accordance with a known solid-phase synthesis method. Regarding a method of chemical synthesis of peptide, it may be for example a chemical synthesis according to Boc strategy and/or Fmoc strategy.

Another object of the invention relates to a polynucleotide coding for a peptide or a protein described above. In particular, another object of the invention relates to a polynucleotide coding for a peptide binding Matrix Binding Motif (M-motif) on tenascin-C (TNC) according to the invention.

In particular, the polynucleotide may be selected amongst the polynucleotides containing a sequence having at least 80% identity with the polynucleotide coding for a peptide or a protein described above.

The percentage of sequence identity or homology may be determined by any method known to one skilled in the art. It may be determined for example by use of BLASTP and BLASTN, for example using default parameters.

Another object of the present invention is a recombinant vector comprising a nucleic acid encoding a peptide according to the invention.

According to the invention, the vector may be any vector known from one skilled in the art adapted to the expression of a nucleic acid. It may be for example any vector selected from the vectors listed in the catalogue http://www.promega.com/vectors/mammalian_express_vectors.htm [24] or http://www.qiagen.com/pendantview/qiagenes.aspx?gaw=PROTQIAgenes08 07&gkw=mammalian+expression [25], or else http://www.scbt.com/chap _exp_vectors.php?type=pCruzTM%20Expression%20Vectors [26]. It may, for example, be the expression vector described in document WO 83/004261 [13]. The vector may be, for example Adeno-associated Virus (AAV) vectors, a plasmid, a Yeast Artificial Chromosomes (YAC) or a Bacterial Artificial Chromosome (BAC).

The vector may be any adapted adeno-associated virus (AAV) vector known to one skilled in the art and/or commercially available adapted. It may be, for example any adapted AAV disclosed in Naso et al 2017, "Adeno-Associated Virus (AAV) as a Vector for Gene Therapy", BioDrugs. 2017 Aug;31(4):317-334. doi: 10.1007/s40259-017-0234-5 [27] and/or in Zincarelli 2008 "Analysis of AAV Serotypes 1-9 Mediated Gene Expression and Tropism in Mice After Systemic Injection" Mol Ther. 2008 Jun ; 16(6):1073-80. doi: 10.1038/mt.2008.76. Epub 2008 Apr 15 [28].

The vector may be any adapted plasmid known to one skilled in the art and/or commercially available. It may be for example a plasmid selected from the group comprising pMX, pUC19, pHP45-CmR, pcDNA3.1(+), pcDNA3.3-TOPO, pcDNA3.4-TOPO, pFastBac1, pET100/D-TOPO, pET151/D-TOPO, pRSET A, pYes2.1V5-His TOPO, pDONR221, pGEX-3X.

The vector may be any adapted Yeast Artificial Chromosome known to one skilled in the art. It may be for example a Yeast Artificial Chromosomes selected from the group comprising pYAC-RC, pYAC3

The vector may be any adapted Bacterial Artificial Chromosome known to one skilled in the art. It may be for example a Bacterial Artificial Chromosome selected from the group comprising pUvBBAC, pCC1BAC, pBAC 108L

The vector may comprise a polynucleotide sequence, for example an expression cassette, comprising the following in 5' to 3'order:
a promoter sequence;
a nucleic acid sequence encoding a peptide of the invention; and
a polyadenylation (polyA) sequence.

The promoter may be any promoter know to one skilled in the art. It may be for example a constitutive promoter, a promoter of viral gene, tissue specific promoter.

It may be for example promoters of viral genes selected from the group comprising CMV promoter.

The vector may be selected according to the selected host cell. One skilled in the art, taking into consideration its technical knowledge, will adapt the vector in light of the host cell.

The host cell may be any host suitable for the expression of the nucleic acids or the vectors of the invention. It may, for example, be mammalian cells, *E. coli, Pischia pastoris, Saccharomyces cerevisiae,* or insect cells, for example an insect cell-baculovirus system, for example SF9 insect cells used in a baculovirus expression system.

According to the invention, the compound binding Matrix Binding Motif (M-motif) on Tenascin-C (TNC) may be for example a "peptidomimetic" or PM. It may be for example a peptidomimetic of the peptide according to the invention, for example it may be a peptidomimetic comprising D Amino Acid, chemically modified Amino Acid. It may be for example β-peptides.

Peptidomimetic as used herein means a non-peptide chemical moiety. A peptidomimetic chemical moiety includes non-amino acid chemical moieties. A peptidomimetic chemical moiety may also include one or more amino acid that are separated by one or more non-amino acid chemical units. A peptidomimetic chemical moiety does not contain in any portion of its chemical structure two or more adjacent amino acids that are linked by peptide bonds.

One skilled in the art taking into consideration its technical knowledge will know how to obtain a peptidomimetic of the peptide according to the invention.

According to the invention, the compound binding Matrix Binding Motif (M-motif) on Tenascin-C (TNC) may be for example a chemical compound. It may be for example any chemical compound known from one skilled in the art that could bind the Matrix Binding Motif (M-motif) on Tenascin-C (TNC).

According to the invention, the binding of compound according to the invention to the Matrix Binding Motif (M-motif) on Tenascin-C (TNC) may be determined by any adapted method known from one skilled in the art. It may be for example any method adapted to the detection and/or determination of ligand - receptor and/or ligand-ligand and/or molecule-molecule and/or peptide-protein binding known from one skilled in the art. It may be for example a method selected from the group comprising fluorescent ligand binding assays, Radioligand binding assays Surface plasmon resonance (SPR) Plasmon-waveguide resonance (PWR) Nanofluidic Fluorescence Microscopy (NFM) Whispering gallery microresonator (WGM) Resonant waveguide grating (RWG) Biolayer Interferometry Biosensor (BIB) Nuclear magnetic resonance (NMR) X-ray crystallography.

The inventor has also surprisingly demonstrated and is the first to demonstrate that a compound binding Matrix Binding Motif (M-motif) on Tenascin-C (TNC) of the invention allow to specifically modify the binding capacities of Tenascin-C (TNC) to chemokines and/or to fibronectin.

The inventors have demonstrated that the compound binding Matrix Binding Motif (M-motif) on Tenascin-C (TNC) according to the invention allows advantageously to regulate the immune response and/or tissue homeostasis. In particular, the inventors have demonstrated that the compound binding Matrix Binding Motif (M-motif) on Tenascin-C (TNC) according to the invention allow to modulate the immune response, for example to restore or inhibit the immune response for example by blocking the immune-suppressive microenvironment, for example immune-suppressive microenvironment within a tumor or to reduce the pro-inflammatory microenvironment in rheumatoid arthritis.

The inventors have also surprisingly demonstrated that the compound binding Matrix Binding Motif (M-motif) on Tenascin-C (TNC) according to the invention allows advantageously to activate TRAIL-induced cell death, in particular in tumor cells of a mesenchymal phenotype that usually are resistant to TRAIL-induced cell death.

The inventors have also demonstrated that the compound binding Matrix Binding Motif (M-motif) on Tenascin-C (TNC) according to the invention allows advantageously to regulate the immune response and/or tissue homeostasis, in particular by downregulating CCR7 or CD86, in particular on dendritic cells for example in chronically inflamed tissues, for example such as Chronic Kidney Disease, Rheumatoid Arthritic, liver fibrosis, lung fibrosis.

Accordingly an object of the present invention is a compound binding Matrix Binding Motif (M-motif) on Tenascin-C (TNC) for its use as medicament.

The compound binding Matrix Binding Motif (M-motif) on Tenascin-C (TNC) is as defined above.

The inventors have also demonstrated that in addition the binding of TNC to chemokines/cytokines thereby inhibiting the function of the chemokines TNC can also bind to cell surface receptors, for example TLR4, integrin alpha 9 beta 1, alpha v beta 3, EGFR, Annexin V and thereby inducing signal transduction that regulates gene expression; for example activate or inhibit the gene expression. The inventors have demonstrate that compound according to the invention inhibit CCR7 and CD86 expression in dendritic cells TNC signaling induced expression.

An object of the invention is also a compound binding Matrix Binding Motif (M-motif) on Tenascin-C (TNC) for use in the treatment of disease, in particular by regulating the immune response and/or tissue homeostasis.

In the present "regulating the immune response" refers to modify the immune response, for example by enhancing the immune response and/or suppress immune-suppressive microenvironment in any organ and/or any biological tissue, for example by inhibiting binding of TNC to chemokines/cytokines such as TGFβ, CCL21 and CXCL12 thus releasing the chemokine/cytokine to restore the immune response in organ and/or biological tissue. On the contrary, through binding of CCR7 and CD86 on dendritic cells, which is blocked, for example by compound according to the invention, for example the M-motif containing peptide, thus suppressing the pro-inflammatory condition in organs with chronic inflammation for example chronic inflammation in liver, kidney, heart, eye etc and cancer in the mammary gland, tongue, head and neck, pancreas, brain, lung.

In the present "regulating the tissue homeostasis" refers to decreasing and/or ablating inflammation in any organ and/or any biological tissue, for example by blocking TNC binding to its cell surface receptor such as TLR4, integrin alpha 9 beta 1, alpha v beta 3 thus inhibiting arthritis, myocarditis, fibrosis of the kidney, lung and other organs.

An object of the invention is a compound binding Matrix Binding Motif (M-motif) on Tenascin-C (TNC) for use in the treatment disease also refers to any disease in which Tenascin-C may be involved.

In the present "disease in which Tenascin-C is involved" may be a disease and/or disorder in which Tenascin-C may be involved. It may be for example a disease selected from the group comprising cancer, infections, tumor, inflammatory disease, angiogenesis, fibrotic disease, immunological disorders, disease involving fibronectin, tenascin-R, tenascin-W, tenascin-X, collagen 12, collagen 14, contactin 1, contact 3, integrin alpha 1 beta 4, PTPRB, PTPRC, PTPRD or PTPRF. Preferably, disease in which Tenascin-C is involved may be disease selected from the group comprising cancer, infections, tumor, inflammatory disease, angiogenesis, fibrosis disease, immunological disorders, disease involving fibronectin.

In the present disease also refers to disease involving at least one chemokine and/or soluble factor such as TGFβ and /or heparin binding soluble factor. It may be for example any disease involving a dysregulation of at least one chemokine/soluble factor and /or heparin binding soluble or insoluble factor.

In the present, "dysregulation" refers to an increase or a decrease of the level of at least one chemokine/soluble factor and /or heparin binding soluble factor in light of reference level and/or level in subject without any disease.

In the present, chemokine refers to any chemokine known from one skilled in the art involved for example in inflammation and/or angiogenesis and/or fibrosis and/or immune response.

In the present, soluble factor refers to any factor known from one skilled in the art involved for example in inflammation and/or angiogenesis and/or fibrosis and/or immune response. It may be for example VEGFA, soluble factor -4.

In the present "chemokine involved in inflammation" refers to any chemokine known from one skilled in the art to have a role/biological effect in inflammation. It may be for example a chemokine disclosed in Proost, P., Wuyts, A. & Van Damme, J. The role of chemokines in inflammation. Int J Clin Lab Res 26, 211-223 (1996). https://doi.org/10.1007/BF02602952 [18]. It may be for example a chemokine selected from the group comprising CXCL-8, CCL2, CCL3, CCL4, CCL5, CCL11, CXCL10, preferably CCL2.

In the present "soluble factor involved in inflammation" refers to any soluble factor known from one skilled in the art to have a role/biological effect in inflammation. It may be for example a soluble factor disclosed in De Laporte et al., 2013, [3].

In the present "chemokine involved in angiogenesis" refers to any chemokine known from one skilled in the art to have a role/biological effect in angiogenesis. It may be for example a chemokine disclosed in Borna Mehrad et al. "Chemokines as mediators of angiogenesis" Thromb Haemost. 2007 May; 97(5): 755-762 [18]. I may be for example a chemokine selected from the group comprising CXCL1, CXCL2, CXCL3, CXCL5, CXCL6, CXCL7, CXCL8, CCL2, CXCL4, CXCL9, CXCL10, CXCL11, CXCL12, CXCL14.

In the present "chemokine involved in fibrosis" refers to any chemokine known from one skilled in the art to have a role/biological effect in fibrosis. It may be for example a chemokine disclosed in Hacer Sahin Hermann E.Wasmuth Chemokines in tissue fibrosis, Biochimica et Biophysica Acta (BBA) - Molecular Basis of Disease Volume 1832, Issue 7, July 2013, Pages 1041-1048 [34]. It may be for example a chemokine selected from the group comprising CCL1, CCL2, CCL3, CCL5, CCL7, CCL11, CCL17, CCL22, CCL27, CXCL1, CXCL2, CXCL4, CXCL5, CXCL8, CXCL10, CXCL12 and CXCL16.

In the present "chemokine involved in immune response" refers to any chemokine known from one skilled in the art to have a role/biological effect in immune response. It may be for example a chemokine disclosed in Caroline L. Sokol and Andrew D. Luster Cold Spring, The Chemokine System in Innate Immunity, Harb Perspect Biol. 2015 May; 7(5): a016303 [35]. It may be for example a chemokine selected from the group comprising CXCL1, CXCL2, CXCL3, CXCL4, CXCL5, CXCL6, CXCL7, CXCL8, CXCL9, CXCL10, CXCL11, CXCL12, CXCL13 ,CXCL14, Cxcl15, CXCL16, CXCL17, CCL1, CCL2, CCL3, CCL4, CCL5, Ccl6, CCL7, CCL8, Ccl9, CCL10, CCL11, Ccl12, CCL13, CCL14, CCL15, CCL16, CCL17, CCL18, CCL19, CCL20, CCL21, CCL22, CCL23, CCL24, CCL25, CCL26, CCL27, CCL28, XCL1 and XCL2.

Preferably, in the present the chemokine involved in inflammation and/or angiogenesis and/or fibrosis and/or immune response may be selected from the group comprising CXCL12, TRAIL, CCL21.

Preferably, in the present the soluble factor involved in inflammation and/or angiogenesis and/or fibrosis and/or immune response is TGFβ.

As mentioned above, the compound binding Matrix Binding Motif (M-motif) on Tenascin-C (TNC) of the invention may be for use in the treatment of inflammatory disease.

In the present inflammatory disease may be any inflammatory disease known to one skilled in the art. It may be for example inflammatory disease of any organ or tissue of a human or of an animal. It may be for example an inflammatory disease of an organ selected from the group comprising lung, liver, eye, heart, lung, breast, bone, bone marrow, brain, head & neck, esophageal, tracheal, stomach, colon, pancreas, cervical, uterine, bladder, prostate, testicular, skin, rectal, and lymphomas. It may be an inflammatory disease selected from the group comprising rheumatoid arthritis, lupus, ulcerative colitis, Crohn's disease and/ or any disease in which the immune system turns against the bodies' organs. It may be an inflammatory disease selected from the group comprising chronic liver inflammation, chronic kidney inflammation, chronic lung inflammation, chronic heart inflammation, heart inflammation, liver inflammation, kidney inflammation, lung fibrosis, rheumatoid arthritis, osteoarthritis, lupus, skin inflammation.

In the present inflammatory disease may be for example an inflammatory disease involving at least one chemokine. It may be for example inflammatory disease involving any chemokine involved in inflammation, for example as disclosed in Proost, P., Wuyts, A. & Van Damme, J. The role of chemokines in inflammation. Int J Clin Lab Res 26, 211-223 (1996). https://doi.org/10.1007/BF02602952 [18]. It may be for example inflammatory disease involving chemokine selected from the group comprising CXCL-8, CCL2, CCL3, CCL4, CCL5, CCL11, CXCL10, preferably CCL2.

It may be for example an inflammatory disease selected from the group comprising respiratory inflammation, pulmonary inflammation, heart inflammation, rheumatoid arthritis, Kawasaki Disease (KD), Takayasu Arteritis (TA), Pancreatitis (PANC).

As mentioned above, the compound binding Matrix Binding Motif (M-motif) on Tenascin-C (TNC) of the invention may be for use in the treatment of angiogenesis and/or disease involving angiogenesis.

It may be for example any disease involving angiogenesis, preferably angiogenesis involving a dysregulation of at least one chemokine and /or heparin binding soluble factor. It may be disease involving angiogenesis implying any chemokine involved in angiogenesis known from one skilled in the art, for example chemokine as disclosed in Borna Mehrad et al. "Chemokines as mediators of angiogenesis" Thromb Haemost. 2007 May; 97(5): 755-762 [18]. I may be for example any disease comprising angiogenesis involving a chemokine selected from the group comprising CXCL1, CXCL2, CXCL3, CXCL5, CXCL6, CXCL7, CXCL8, CCL2, CXCL4, CXCL9, CXCL10, CXCL11, CXCL12 and CXCL14 and/or soluble factor from the group comprising VEGF, TGFβ, endothelin etc. It may be for example a disease selected from the group comprising chronic inflammatory and fibro proliferative disorders. It may be for example disease selected from the group comprising Idiopathic pulmonary fibrosis (IPF), Bronchiolitis obliterans syndrome (BOS), Acute respiratory distress syndrome (ARDS).

As mentioned above, the compound binding Matrix Binding Motif (M-motif) on Tenascin-C (TNC) of the invention may be for use in the treatment of immune disorder and/or disease involving an immune disorder.

It may be for example a disease involving an immune disorder, for example an immune disorder involving a dysregulation of at least one chemokine and /or heparin binding soluble factor. It may be for example an immune disorder comprising autoimmune disease, immuno-suppression disease, allergy and atopy, a disease comprising imbalance between T helper 1, T helper 17 and T helper 2 immune response, imbalance of T reg, type 2 hypersensitivity. It may be for example a disease selected from the group comprising lupus sjogren's syndrome, scleroderma, Age Related Macular Degeneration (AMD, ARMD), for example Dry and Wet forms of Age Related Macular Degeneration, Antiphospholipid (Catastrophic - Asherson's) Syndrome, Behçet's Disease, Central serous chorioretinopathy (CSC), Dry Eye Syndrome (DYS), Scleroderma (SCLE), Uveitis (UVE), Microscopic Polyangiitis.

As mentioned above, the compound binding Matrix Binding Motif (M-motif) on Tenascin-C (TNC) of the invention may be for use in the treatment of cancer.

It may be for example any cancer of any organ known from one skilled in the art. It may be for example any disease involving abnormal cell growth with the potential to invade or spread to other parts of the body. It may be for example any cancer involving that could involve or not inflammation of tissue. It may be for example cancer of any organ or tissue of a human or of an animal. It may be for example a cancer selected from the group comprising lung, liver, eye, heart, lung, breast, bone, bone marrow, brain, head & neck, esophagus, trachea, stomach, colon, pancreas, cervical, uterine, bladder, prostate, testicules, skin, rectum, and lymphomas. It may be for example a cancer involving a dysregulation of at least one chemokine and /or heparin binding soluble factor. It may be for example any cancer involving a dysregulation of at least one CXCL12, TRAIL, CCL21 and TGFβ. It may be for example any cancer involving an immune disorder and/or neoplasm and/or angiogenesis. It may be for example a cancer involving immuno-suppressive tumor microenvironment (TME). It may be for example a cancer selected from the group comprising breast cancer (all forms), pancreatic cancer, renal cell carcinoma, prostate cancer, esophageal cancer, Primary myelofibrosis, Fibrosarcoma, Dermatofibrosarcoma protuberans, Desmoid tumor, Desmoplastic/nodular medulloblastoma, Neurofibromatosis type 2, Glioblastoma, Sarcoma (all forms), Uveal melanoma, Gastro-intestinal / Colorectal / Gastric cancer, Liver cancer, Lung cancer (all forms), Head and Neck Cancer (all forms), Lymphoma, Leukemya, Radiation-induced fibrosis (RIF).

As mentioned above, the compound binding Matrix Binding Motif (M-motif) on Tenascin-C (TNC) of the invention may be use in the treatment of tumor disease. It may be for example any tumor of any organ known from one skilled in the art. It may be for example any tumor involving abnormal cell growth with the potential to invade or spread to other parts of the body. It may be for example tumor of any organ or tissue of a human or of an animal. It may be for example a tumor of an organ selected from the group comprising lung, liver, eye, heart, lung, breast, mammary gland, bone, bone marrow, brain, head and neck, esophagus, trachea, stomach, colon, pancreas, cervix, uterine, bladder, prostate, testicles, skin, rectum, and lymphomas.

As mentioned above, the compound binding Matrix Binding Motif (M-motif) on Tenascin-C (TNC) of the invention may be used in the treatment of infection. It may be for example any infection known from one skilled in the art. It may be for example a bacterial and/or virus infection.

As mentioned above, the compound binding Matrix Binding Motif (M-motif) on Tenascin-C (TNC) of the invention may be for use in the treatment of fibrosis disorder and/or disease involving a fibrosis disorder. It may be for example any fibrosis disorders, for example any fibrosis disorder of any organ known from one skilled in the art. It may be for example fibrosis disorder of an organ selected from the group comprising lung, liver, eye, heart, lung, breast, mammary gland, bone, bone marrow, brain, head and neck, esophagus, trachea, stomach, colon, pancreas, cervix, uterine, bladder, prostate, testicles, skin, rectum . It may be for example any disease involving a fibrosis disorder known from one skilled in the art. It may be for example respiratory and pulmonary fibrosis, heart fibrosis and ischemia, rheumatoid arthritis, lupus, sjogren's syndrome, scleroderma, cystic fibrosis, Age Related Macular Degeneration (AMD, ARMD, Dry and Wet forms), Antiphospholipid (Catastrophic - Asherson's) Syndrome, Behçet's Disease, Central serous chorioretinopathy (CSC), Dry Eye Syndrome (DYS), Idiopathic Pulmonary Fibrosis (IPF), Kawasaki Disease (KD), Microscopic Polyangiitis (MP), Oral submucous fibrosis (OSMF), Ovarian fibrosis or polycystic ovarian syndrome (OVF), Pancreatitis (PANC), Proliferative vitreoretinopathy (PVR), Retroperitoneal Fibrosis -Ormond's Disease-(RPF), Scleroderma (SCLE), Stargardt's disease (STGD) , Takayasu Arteritis (TA), Uveitis (UVE).

As mentioned above, the compound binding Matrix Binding Motif (M-motif) on Tenascin-C (TNC) of the invention may be for use in the treatment of immunological disorders. It may be for example any immunological disorders wherein Tenascin-C may be involved known from one skilled in the art.

The inventors have demonstrated that the compound binding Matrix Binding Motif (M-motif) on Tenascin-C (TNC) according to the invention allow advantageously to inhibit and/or block the binding and/or interaction between TNC and the fibronectin.

An object of the invention is a compound binding Matrix Binding Motif (M-motif) on Tenascin-C (TNC) for use in the treatment disease also refers to any disease in which fibronectin may be involved, for example any disease in which Tenascin-C and fibronectin interaction may be involved.

An object of the invention is a compound binding Matrix Binding Motif (M-motif) on Tenascin-C (TNC) for use in the treatment disease also refers to any disease in which Tenascin-C and fibronectin interaction may be involved.

In the present, disease in which Tenascin-C and fibronectin interaction may be involved may be for example a disease comprising fibrosis of the joints, kidney, liver, lung, cancer, for example cancer of the brain, cancer of mammary gland. It may be for example a disease selected from the group comprising brain cancer, mammary gland cancer.

An object of the invention is a compound binding Matrix Binding Motif (M-motif) on Tenascin-C (TNC) according to the invention for use in the treatment of inflammatory and fibrotic disease selected from the group comprising respiratory and pulmonary inflammation as well as fibrosis, heart inflammation and ischemia, rheumatoid arthritis, lupus, sjogren's syndrome, scleroderma, cystic fibrosis, Age Related Macular Degeneration (AMD, ARMD, Dry and Wet forms), Antiphospholipid (Catastrophic - Asherson's) Syndrome, Behçet's Disease, Central serous chorioretinopathy (CSC), Dry Eye Syndrome (DYS), Idiopathic Pulmonary Fibrosis (IPF), Kawasaki Disease (KD), Microscopic Polyangiitis (MP), Oral submucous fibrosis (OSMF), Ovarian fibrosis or polycystic ovarian syndrome (OVF), Pancreatitis (PANC), Proliferative vitreoretinopathy (PVR), Retroperitoneal Fibrosis -Ormond's Disease-(RPF), Scleroderma (SCLE), Stargardt's disease (STGD) , Takayasu Arteritis (TA), Uveitis (UVE).

An object of the invention is also a compound binding Matrix Binding Motif (M-motif) on Tenascin-C (TNC) according to the invention for use in the treatment of dysbiosis related disorders selected from the group comprising inflammatory bowel diseases (ulcerative colitis, crohn's disease), Intestinal Fibrosis (IF), colitis, metabolic disorders, obesity induced disorders, microvascular diabetic complications, hepatic failures and fibrosis (NASH, NAFLD), kidney failures and fibrosis.

An object of the invention is also a compound binding Matrix Binding Motif (M-motif) on Tenascin-C (TNC) according to the invention for use in the treatment of neoplasms, for example comprising tumors and related complications characterized by immunosuppressive inflammation and/or angiogenesis and/or fibrosis, for example breast cancer (all forms), pancreatic cancer, renal cell carcinoma, Prostate cancer, Esophageal cancer, Primary myelofibrosis, Fibrosarcoma, Dermatofibrosarcoma protuberans, Desmoid tumor, Desmoplastic/nodular medulloblastoma, Neurofibromatosis type 2, Glioblastoma, Sarcoma (all forms), Uveal melanoma, Gastro-intestinal / Colorectal / Gastric cancer, Liver cancer, Lung cancer (all forms), Head and Neck Cancer (all forms), Lymphoma, Leukemya, Radiation-induced fibrosis (RIF).

In the present, the compound of the invention may be used in a combination therapy, for example with a therapeutic agent.

"Combination therapy" (or "co-therapy") includes the administration of a compound binding Matrix Binding Motif (M-motif) on Tenascin-C (TNC) and/or pharmaceutical composition of the invention, and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected).

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

Sequential or substantially simultaneous administration of each therapeutic agent can be effected by any appropriate route including, but not limited to, topical routes, oral routes, intravenous routes, intramuscular routes, and direct absorption through mucous membrane tissues. The therapeutic agents can be administered by the same route or by different routes. For example, a first therapeutic agent of the combination selected may be administered by injection while the other therapeutic agents of the combination may be administered topically.

A "therapeutic agent" is any compound known in the art that is used in the detection, diagnosis, or treatment of a condition or disease. Such compounds may be naturally-occurring, modified, or synthetic. Non-limiting examples of therapeutic agents may include drugs, therapeutic compounds, genetic materials, metals (such as radioactive isotopes), proteins, peptides, carbohydrates, lipids, steroids, nucleic acid based materials, or derivatives, analogues, or combinations thereof in their native form or derivatized with hydrophobic or charged moieties to enhance incorporation or adsorption into a cell. Non-limiting examples of therapeutic agents may include immune-related agents, thyroid agents, respiratory products, antineoplastic agents, anti-helmintics, anti-malarials, mitotic inhibitors, hormones, anti-protozoans, antituberculars, cardiovascular products, blood products, biological response modifiers, anti-fungal agents, vitamins, peptides, anti-allergic agents, anticoagulation agents, circulatory drugs, metabolic potentiators, anti-virals, anti-anginals, antibiotics, anti-inflammatories, anti-rheumatics, narcotics, cardiac glycosides, neuromuscular blockers, sedatives, local anesthetics, general anesthetics, or radioactive atoms or ions. A therapeutic agent may be a toxin, a small therapeutic molecule, a therapeutic nucleic acid, or a chemotherapeutic agent. A chemotherapeutic agent refers to a chemical compound that is useful in the treatment of cancer. The compound may be a cytotoxic agent that affects rapidly dividing cells in general, or it may be a targeted therapeutic agent that affects the deregulated proteins of cancer cells.

In the present, the therapeutic agent may be preferably selected from group comprising anticancer drug, anti-inflammatory drug, anti-fibrotic drug, anti-angiogenic drug, anti-hypertensive drug, anti-oxidative drug.

In the present, the therapeutic agent may be anticancer drug, preferably a cytotoxic drug. As used herein, the term "cytotoxic drug" refers to a molecule that when entering in contact with a cell, optionally upon internalization into the cell, alters a cell function (e.g. cell growth and/or proliferation and/or differentiation and/or metabolism such as protein and/or DNA synthesis) in a detrimental way or leads to cell death. As used herein, the term "cytotoxic drug" encompasses toxins, in particular cytotoxins. It may be, for example, a compound selected from the group comprising calicheamycin, dolastin 10, dolastin 15, auristatin E, auristatin EB (AEB), auristatin EFP (AEFP), monomethyl auristatin F (MMAF), monomethylauristatin-D (MMAD), monomethyl auristatin E (MMAE), and 5-benzoylvaleric acid-AE ester (AEVB) and duocarmycin; nitrogen mustard analogues for example cyclophosphamide, melphalan, ifosfamide or trofosfamide; ethylenimines such as thiotepa; nitrosoureas for example carmustine; alkylating agents for example temozolomide or dacarbazine; folate-like metabolic antagonists such as methotrexate or raltitrexed; purine analogues for example thioguanine, cladribine or fludarabine; pyrimidine analogues for example fluorouracil, tegafur or gemcitabine; vinca alkaloids for example vinblastine, vincristine or vinorelbine and analogues thereof; podophyllotoxin derivatives for example etoposide, taxans, docetaxel or paclitaxel; anthracyclines for example doxorubicin, epirubicin, idarubicin and mitoxantrone, and analogues thereof; other cytotoxic antibiotics for example bleomycin and mitomycin; platinum compounds for example cisplatin, carboplatin and oxaliplatin; pentostatin, miltefosine, estramustine, topotecan, irinotecan and bicalutamide, and toxins for example ricin toxin, liatoxin and Vero toxin. It may also, for example be radioisotopes, for example those disclosed in Peter J Hoskin, Radiotherapy in Practice - Radioisotope Therapy, 2007 [23], small molecules blocking the cell microtubules, such as 6-mercaptopurine, siRNAs, for example siRNA delivery system to silence PHB1 expression in prostate cancer (Xu X et al 2017 [24]), toxins, for example saporin, gelonin, ricin, shiga toxin, etc. (for review see Allahyari H et al 2017 [25]), tissue factors, for example membrane-bound full-length tissue factor or soluble alternatively spliced tissue factor (Eisenreich A et al. [26]), peptides, for example _{D}(KLAKLAK)₂ (SEQ ID NO 73) disclosed in Arap et al [27]; Leuschner and Hansel 2005 [28].

In the present, the therapeutic agent may be an anti-inflammatory drug. It may be for example any an anti-inflammatory drug known from one skilled in the art. It may be for example Nonsteroidal anti-inflammatory drugs, It may be for example anti-inflammatory drug selected from the group comprising aspirin, ibuprofen, naproxen ibuprofen, diclofenac, celecoxib, mefenamic acid, etoricoxib, indomethacin.

In the present, the therapeutic agent may be an anti-fibrotic drug. It may be for example any anti-fibrotic drug known from one skilled in the art. It may be for example an anti-fibrotic drug selected from the group comprising pirfenidone, Nintedanib, prednisolone, bethametasone and dexamethasone.

In the present, the therapeutic agent may be an anti-angiogenic drug. It may be for example any anti-angiogenic drug known from one skilled in the art. It may be for example an anti-angiogenic drug selected from the group comprising bevacizumab, itraconazole, carboxyamidotriazole, TNP-470, IFN-α, IL-12, suramin, SU5416, thrombospondin, angiostatin, endostatin, 2-methoxyestradiol, tecogalan, tetrathiomolybdate, thalidomide, thrombospondin, prolactin, linomide, ramucirumab, tasquinimod, ranibizumab, sorafenib, sunitinib ,pazopanib and everolimus.

In the present, the therapeutic agent may be an anti-hypertensive drug. It may be for example any anti-hypertensive drug known from one skilled in the art. It may be for example antihypertensive drug selected from the group comprising acebutolol, atenolol, bisoprolol, betaxolol, carteolol, carvedilol, labetalol, metoprolol, nadolol, nebivolol, oxprenolol, penbutolol, pindolol, propranolol, timolol, doxazosin, phentolamine, indoramin, phenoxybenzamine, prazosin, terazosin, tolazoline, bucindolol, carvedilol, labetalol, eplerenone, spironolactone, clonidine, guanabenz, guanfacine, methyldopa and moxonidine.

In the present, the therapeutic agent may be an anti-oxydative drug. It may be for example any anti-oxydative drug known from one skilled in the art. It may be for example an anti-oxydative drug selected from the group comprising ascorbic acid, vitamin a, vitamin e, lipoic acid, masoprocol, pramipexole, nitric oxide, allopurinol, pentoxifylline, melatonin, dimethyl sulfoxide, probucol, 3,4-dihydroxycinnamic acid, resveratrol, 3-hydroxyanthranilic acid, dihydrolipoic acid, p-coumaric acid, quercetin, aeol 10150, transcrocetinate, acetylcysteine, nicaraven, lodoxamide, ferulic acid, uric acid, idebenone, chromic chloride, thiosulfuric acid.

In the present, the therapeutic agent may be an immune checkpoint inhibitor.

In the present, the administration of the medicament may be carried out by any way known to one skilled in the art. It may, for example, be carried out directly, i.e. pure or substantially pure, or after mixing of compound binding Matrix Binding Motif (M-motif) on Tenascin-C thereof with a pharmaceutically acceptable carrier and/or medium. According to the present invention, the medicament may be an injectable solution, a medicament for oral administration, for example selected from the group comprising a liquid formulation, a multiparticle system, an orodispersible dosage form. According to the present invention, the medicament may be a medicament for oral administration selected from the group comprising a liquid formulation, an oral effervescent dosage form, an oral powder, a multiparticle system, an orodispersible dosage form.

The medicament may be in any form that can be administered to a human or an animal. It may for example be a pharmaceutical composition as defined below.

According to the invention, a compound binding Matrix Binding Motif (M-motif) on Tenascin-C (TNC) according to the invention may be used as a medicament, for example, in a pharmaceutical composition.

Another object of the present invention is a pharmaceutical composition comprising at least one a compound binding Matrix Binding Motif (M-motif) on Tenascin-C (TNC) according to the invention.

Another object of the present invention is a pharmaceutical composition comprising at least one a compound binding Matrix Binding Motif (M-motif) on Tenascin-C (TNC) for use in the prevention and/or treatment of disease.

The compound binding Matrix Binding Motif (M-motif) on Tenascin-C (TNC) is as defined above.

According to the present invention, the pharmaceutical composition may comprise any pharmaceutically acceptable and/or therapeutically effective amount of a compound binding Matrix Binding Motif (M-motif) on Tenascin-C (TNC).

The pharmaceutical composition may be in any form that can be administered to a human or an animal. The person skilled in the art clearly understands that the term "form" as used herein refers to the pharmaceutical formulation of the medicament for its practical use. For example, the medicament may be in a form selected from the group comprising an injectable form, an oral suspension, a pellet, a powder, granules or topical form (e.g. cream, lotion, collyrium).

The pharmaceutical composition may comprise a pharmaceutically acceptable carrier.

The pharmaceutically acceptable carrier may be any known pharmaceutical support used for the administration of an aptamer to a human or animal, depending on the subject to be treated. It may be for example a pharmaceutically acceptable carrier selected from the group comprising, excipients such as sucrose, starch, mannitol, sorbitol, lactose, glucose, cellulose, talc, calcium phosphate, and calcium carbonate; binders such as cellulose, methylcellulose, hydroxylpropylcellulose, polypropylpyrrolidone, gelatin, gum arabic, polyethylene glycol, sucrose, and starch; disintegrants such as starch, carboxymethylcellulose, hydroxylpropylstarch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizin-ammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like.

According to the invention, the pharmaceutical composition may be administrated by any adapted route of administration known to one skilled in the art. For example, the pharmaceutical composition may be administrated by oral administration and/or parenteral administration.

For example, for parenteral administration aqueous and non-aqueous isotonic sterile injectable liquids may be used, which may comprise an antioxidant, a buffer solution, a bacteriostatic agent, an isotonizing agent and the like. Aqueous and non-aqueous sterile suspensions can also be mentioned, which may comprise a suspending agent, a solubilizer, a thickener, a stabilizer, an antiseptic and the like. The preparation may be included in a container such as an ampoule or a vial in a unit dosage volume or in several divided doses.

According to the invention, the pharmaceutical composition may be a sustained-release preparations. For example the pharmaceutical composition may comprise sustained release from carriers or containers embedded in the body, such as artificial bones, biodegradable bases or non-biodegradable sponges, bags and the like. For example, the pharmaceutical composition may comprise biodegradable compounds and/or pharmaceutical support, for example liposome, cationic liposome, Poly(lactic-co-glycolic) acid (PLGA), atherocollagen, gelatin, hydroxyapatite, polysaccharide sizofiran. For example the pharmaceutical composition may be delivered with devices for continuous or intermittent, systemic or topical delivery from outside the body, such as drug pumps and osmotic pressure pumps.

According to the invention, the pharmaceutical composition may be an inhalants, for example suitable for transpulmonary administration, ointments suitable for percutaneous administration. For example, the pharmaceutical composition may comprise surfactant, oil and/or cyclodextrin.

According to the invention, the pharmaceutical composition may be coated by any compound and/or any process known to one skilled in the art. For example the pharmaceutical composition may be coated by a coating agents selected from the group comprising hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, pigments, for example red iron oxide, titanium dioxide.

Advantageously, the coating and/or coating agent may be useful to improve the compliance of the pharmaceutical composition, for example the taste masking, its enteric dissolution and/or its sustained release.

The pharmaceutical form or method of administering a pharmaceutical composition may be selected with regard to the human or animal subject to be treated. For example, for a child, for example from 1 to 17 years old, or a baby, for example under 1 year old, a syrup or an injection may be preferred. Administration may for example be carried out with a weight graduated pipette, a syringe. For example, for an adult over 17 years old, an injection may be preferred. Administration may be carried out with an intravenous weight graduated syringe.

According to the present invention, the pharmaceutical composition may comprise any pharmaceutically acceptable and/or therapeutically effective amount of a compound binding Matrix Binding Motif (M-motif) on Tenascin-C (TNC).

The compound binding Matrix Binding Motif (M-motif) on Tenascin-C (TNC) is as defined above.

For example, in the case of cancer, the therapeutically effective amount of compound binding Matrix Binding Motif (M-motif) on Tenascin-C (TNC) according to the invention may reduce the number of cancer cells; reduce the tumor size; inhibit (i.e. slow to some extent and preferably stop) cancer cell infiltration into peripheral organs; inhibit (i.e. slow to some extent and preferably stop) tumor metastasis; inhibit, to some extent, tumor growth; and/or relieve to some extent one or more of the symptoms associated with the cancer.

For example, in the case of inflammatory disease, the therapeutically effective amount of compound binding Matrix Binding Motif (M-motif) on Tenascin-C (TNC) according to the invention may reduce scarring, fibrosis, angiogenesis, immune cell activation, immune cell inhibition, and promote tumor cell death and/or relieve to some extent one or more of the symptoms associated with the inflammatory disease.

For example, in the case disease involving angiogenesis, the therapeutically effective amount of compound binding Matrix Binding Motif (M-motif) on Tenascin-C (TNC) according to the invention may reduce formation of new blood vessels, vessel leakiness, stem cell niches associated with blood vessels and/or relieve to some extent one or more of the symptoms associated with angiogenesis.

For example, in the case disease involving immune disorder, the therapeutically effective amount of compound binding Matrix Binding Motif (M-motif) on Tenascin-C (TNC) according to the invention may promote TRAIL actions on immune and tumor cells, activate release of immune cell subtypes such as macrophages, dendritic cells and T cells from the extracellular matrix, inhibit dendritic cell activation and/or relieve to some extent one or more of the symptoms associated with the immune disorder.

For example, in the case disease involving fibrosis, the therapeutically effective amount of compound binding Matrix Binding Motif (M-motif) on Tenascin-C (TNC) according to the invention may prevent expression of extracellular matrix molecules such as collagen and fibronectin, prevent matrix assembly of Collagen, prevent activation of TGFβ signaling causing matrix expression and/or relieve to some extent one or more of the symptoms associated with fibrosis.

For example, in the case of tissue repair such as wound healing, aortic aneurism, the therapeutically effective amount of compound binding Matrix Binding Motif (M-motif) on Tenascin-C (TNC) according to the invention may cause release, for example, of TGFb, CXCL12, VEGF, from Tenascin-C (TNC) and allow to attract cells, for example fibroblasts, macrophages, endothelial cells, into the injured tissue to promote wound healing and tissue repair.

Other advantages may still be apparent to those skilled in the art by reading the examples below, illustrated by the accompanying figures, given by way of illustration.

### Brief description of the drawing

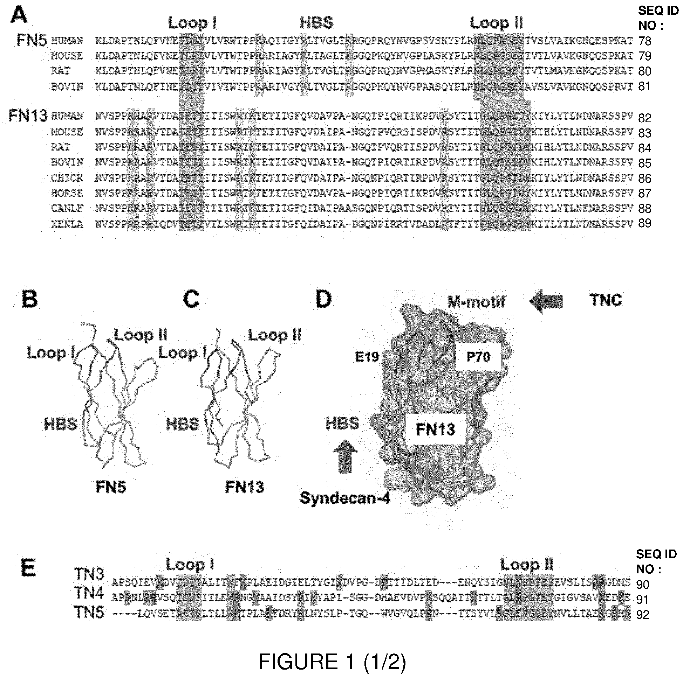

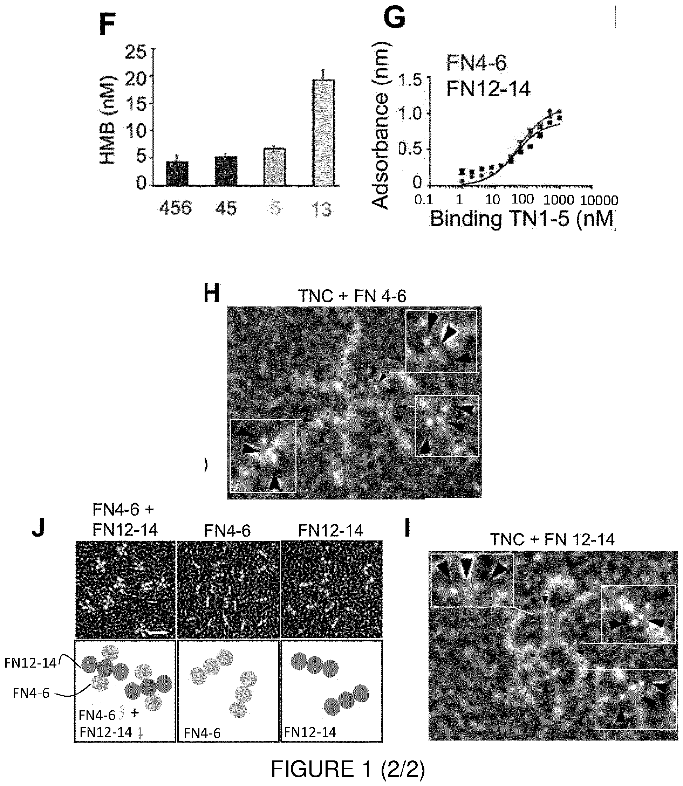

- Figure 1 represents: figure 1A alignment of FN13 and FN5 sequences from different species wherein the M-motif Loop I (Loop I), M-motif Loop II (Loop II) and the heparin binding site (HBS) are marked. Figure 1B -D show schematic 3D representations of structural models of FN5 and FN13 highlighting Loop I, Loop II, glutamate in position 19 (E19) and prolin at position 70 (P70) and the HBS. Figure 1D is a structural model of FN13, revealing that the cationic cradle (syndecan-4 binding site) and the M-motif binding TNC are on opposite sites within FN13. The Kd of TNC to FN13 was determined as 128 nM and that of syndecan-4 to FN13 as 63 nM (Huang et al., 2001 [5]). Figure 1E represents the alignment of TN3, TN4 and TN5 sequences wherein the M-motif Loop I (Loop I), M-motif Loop II (Loop II) are identified. Figure 1F and G show ELISA experiments. In Figure 1F FN5 (5), FN4/5 (45), FN4-6 (456) and FN13 (13) are adsorbed to the dish and TNC binding is measured as half maximal binding (HMB, nM).Figure 1G represent a diagram representing the binding curves of FN4-6 and FN12-14 to TNC (TN1-5). Figure 1H-I show results from negative EM imaging. Figure 1H and I are images show binding (arrows) of FN4-6 and FN12-14, respectively to TNC. Figure 1J are negative EM imaging of a mix of FN4-6 and FN12-14 (FN4-6 + FN12-14), FN4-6 alone and FN12-14 alone and shows binding of FN4-6 to FN12-14 whereas FN4-6 and FN12-14 do not form homotypic binding.

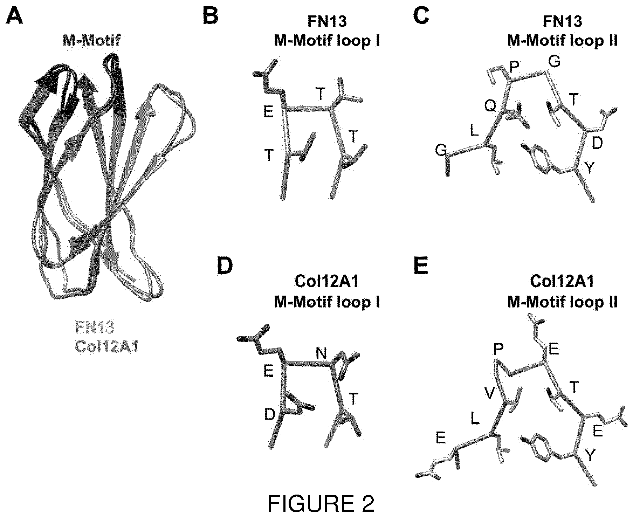

- Figure 2 is a schematic representation of structural modeling of FN13 structure (grey) with a FNIII repeat (FN1) in Col 12A1 (black). Figure 2B is a schematic representation of FN13 M-Motif loop I, Figure 2C is a schematic representation of FN13 M-Motif loop II, Figure 2D is a schematic representation of Col 12A1 M-Motif loop I and Figure 2E is a schematic representation of Col 12A1 M-Motif loop II.

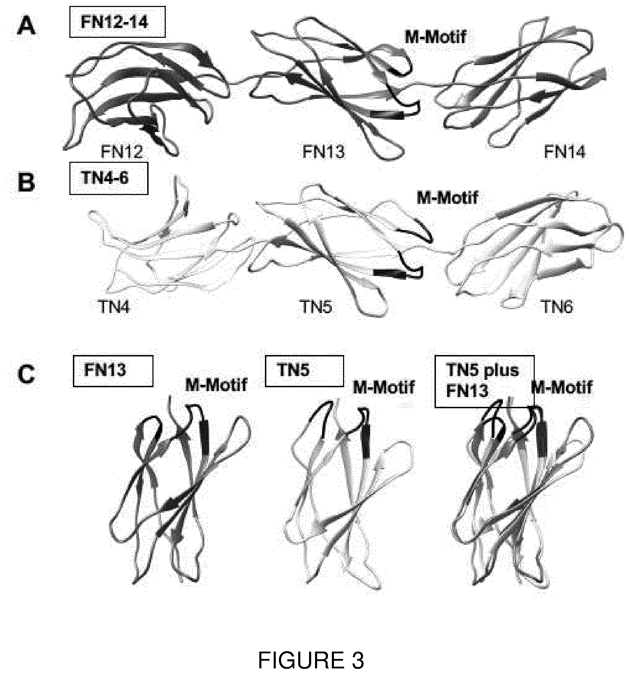

- Figure 3 is schematic representations of structural models of FN12-14 and TNC4-6 (figure 3A, B) and of overlay of the structural models of TN5 with FN13 (figure 3 C).

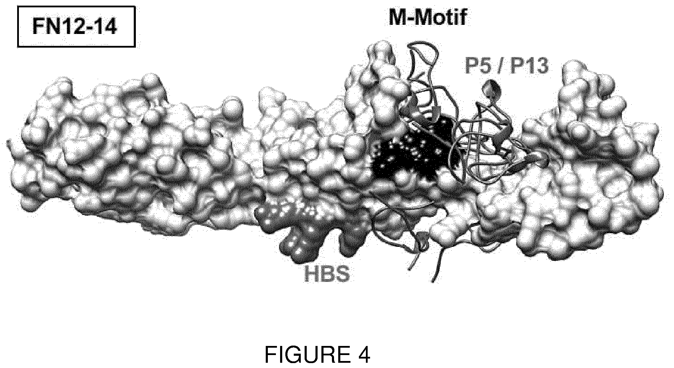

- Figure 4 is a schematic representations of a model of FN12-14 where binding of P5 and P13, respectively was modelled. Note more than 50 different interaction possibilities of P5 and P13 in the M-motif of FN13 in contrast to no binding in the HBS.

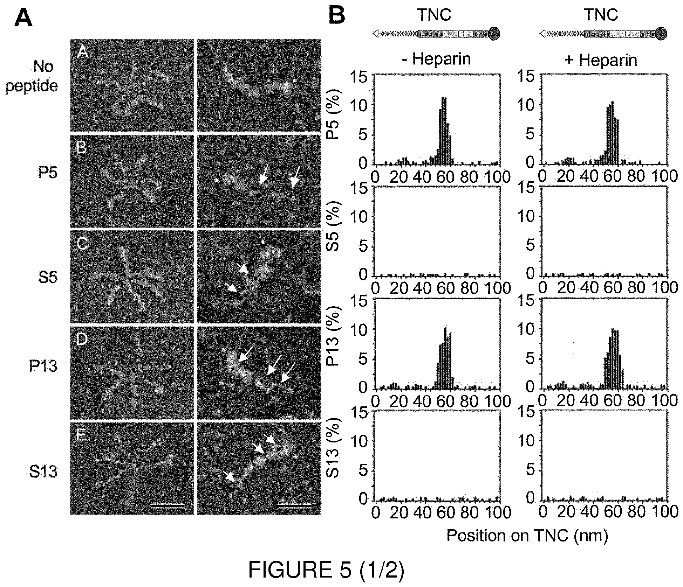

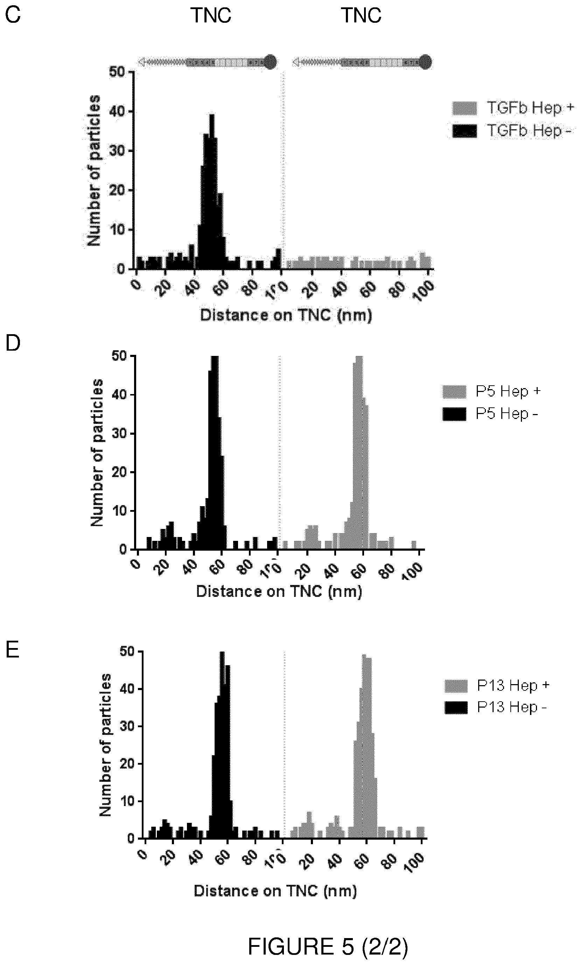

- Figure 5 are results from negative EM imaging of gold labeled peptides P5 and P13 (and control peptide S5 and S13) to full length TNC (figure 5 A), the arrows points a bound gold beads. Figure 5B represent diagrams illustrating and quantifying of the binding of peptides P5, S5, P13 and S13, respectively to TNC in the presence (+Heparin) or absence (-Heparin) of heparin (figures 5B). Ordinate represents binding of gold particles along the length of the TNC molecule (nm), coordinate represents relative binding of the peptides or TGFβ (%) Figure 5C to E represent diagrams from negative EM imaging illustrating and quantifying binding of TGFβ to TNC in comparison to binding of P5 and P13 to TNC in the absence (- Heparin/Hep -) or presence (+Heparin/Hep+) of heparin. Abcissa represents binding of gold particles along the length of the TNC molecule (nm), ordinate represents number of binding of particle. Note that P5 and P13 bind in the middle of TNC (TN5) which is not inhibited by heparin. In contrast S5 nor S13 bind to TNC. P5 and P13 bind at the same site as TGFβ indicating that TN5 is the binding site for P5 and P13 respectively.

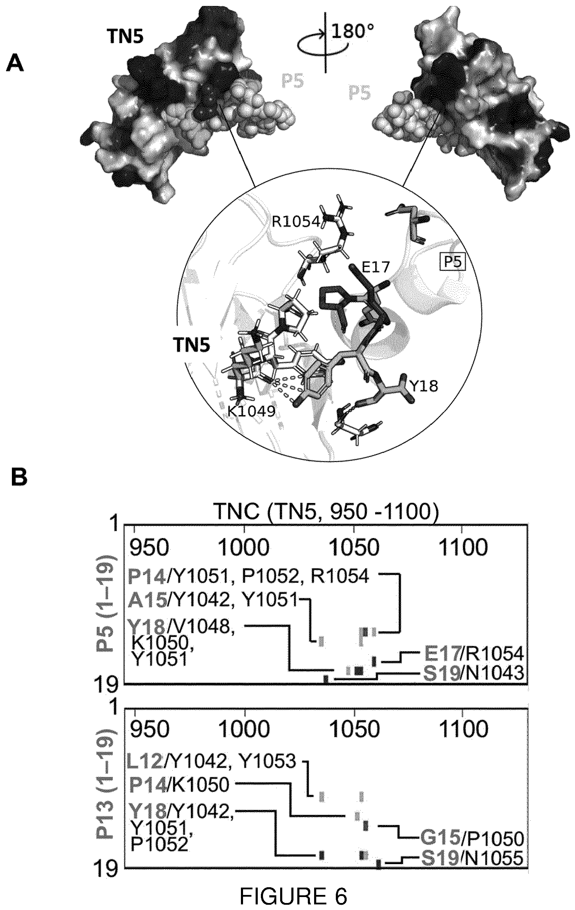

- Figure 6 is a schematic representing the modeling of the interaction of TNC (TN5) with peptide P5 (SEQ ID NO 59)/ peptide P13 (SEQ ID NO 62 ). Figure 6A shows the vicinity of the interacting amino acids of P5 with that in TN5 (Figure 6A). Figure 6B shows the interaction map of P5 and P13, respectively with the respective amino acids in TN5. P5 and P13 are marked in grey. Note multiple interaction sites for several amino acids in P5 and P13, respectively with TN5.

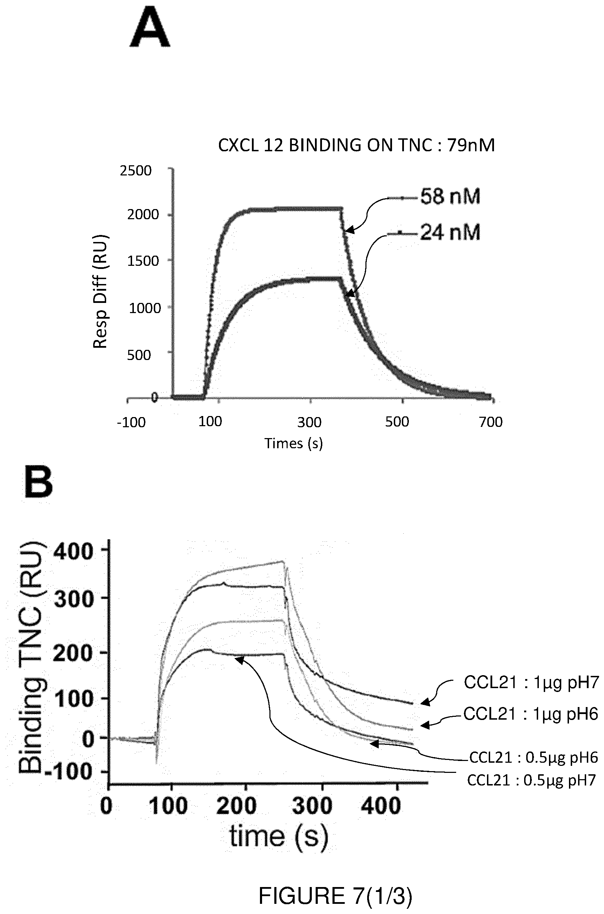

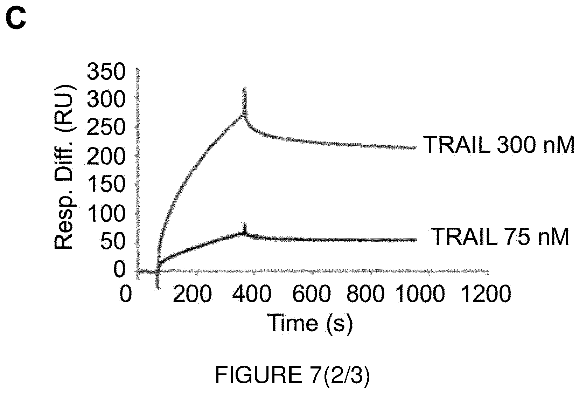

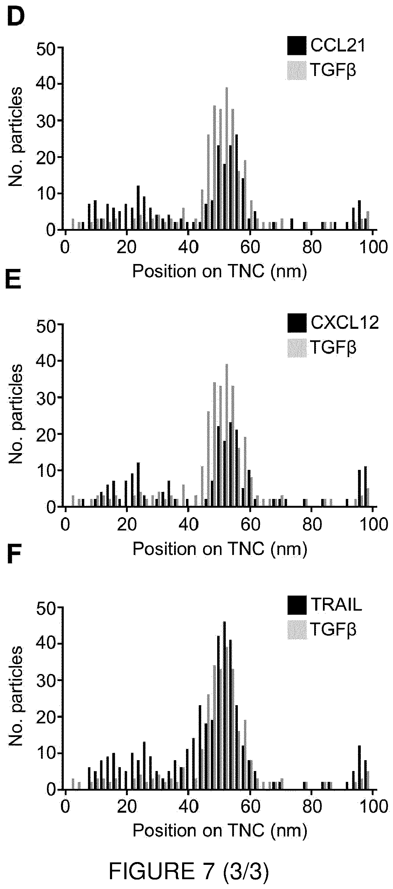

- Figure 7 displays diagrams representing results from Surface Plasmon Resonance (SPR) measurement of binding of CXCL12 on TNC (Figure 7A), of binding of CCL21 on TNC (Figure 7B), and of binding of TRAIL on TNC (figure 7 C). The abscissa represents the time in seconds and the ordinate the Resonance Unit (RU). Figure 7D, represents quantification of negative EM images of binding of TGFβ (grey) to TNC in comparison to CCL21 (black, figure 7D), CXCL12 (black, figure 7E) and TRAIL (black, figure 7F). Note an overlap of binding of CCL21, CXCL12 and TRAIL with TGFβ around the position 50nm. As TGFβ was shown to bind to TN5 (DeLaporte et al.), TN5 is a major bing site for CCL21, CXCL12 and TRAIL. On figures 7 D - F ordinate represent the number of bound particles and abscissa the distance on TNC (in nm).

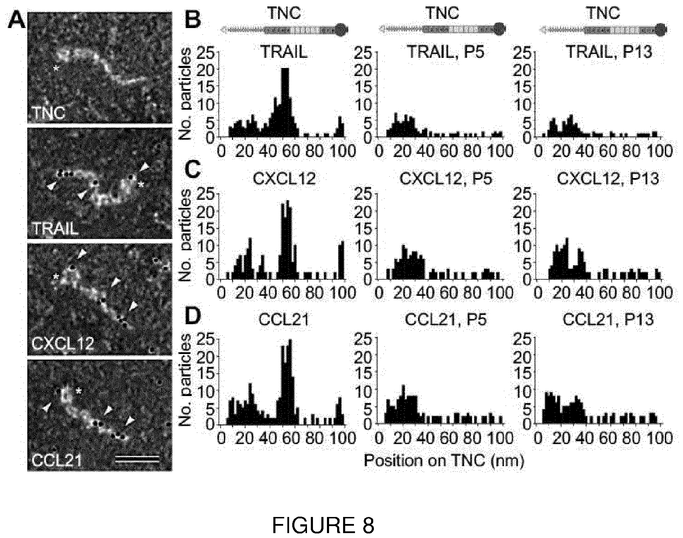

- Figure 8 displays images of negative EM imaging and diagrams, figure 8A are images of negative EM imaging of gold labeled TRAIL, CXCL12, CCL21 in absence of peptide P5 or P13, arrows indicates binding of TRAIL, CXCL12 and CCL21 on TNC. Figures 8 B-C are diagrams representing the quantification of binding of TRAIL (figure 8B), CXCL12 (figure 8C), CCL21 (figure 8D) in presence or absence of peptide P5 or P13 along TNC (coordinate represents the number of bound particles and ordinate the distance on TNC (in nm)).

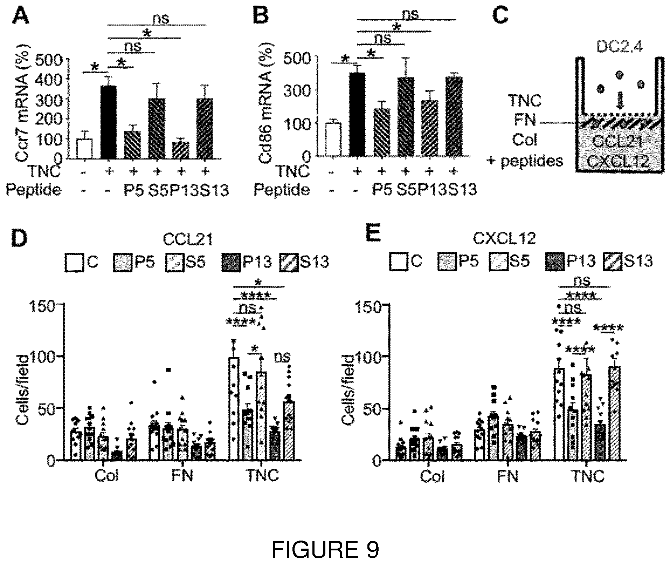

- Figure 9 displays diagrams representing *Ccr7* and *Cd86* mRNA levels in DC2.4 cells upon stimulation with TNC in the presence (+) or absence (-) of peptide P5, P13, S5 and S13, respectively (figures 9 A-B) (*p<0.05; ns = not statistically significant) Figure 9C is a schematic representation of the experimental setup used for measuring the chemoretention of dendritic DC2.4 cells on TNC in the presence of CCL21, CXCL12, peptide P5, S5, P13 or S13 present in the lower chamber. Figure 9D is a diagram showing chemoretention of dendritic DC2.4 cells on TNC or collagen (Col) or fibronectin (FN) in the presence of CCL21 in presence of peptide P5, S5, P13, S13 or no peptide (control, C), (*p<0.05; ****p<0.0001 ; ns = not statistically significant) and Figure 9E is a diagram showing chemoretention of dendritic DC2.4 cells on TNC or collagen (Col) or fibronectin (FN) in the presence of CXCL12 in presence of peptide P5, S5, P13, S13 or no peptide (control, C) The ordinate represents the chosen conditions of surface coating with Col, FN or TNC and the coordinate represents the number of cells that were counted on the coated surface after fixation with methanol and DAPI staining after 5 hours of migration (****p<0.0001; ns = not statistically significant).

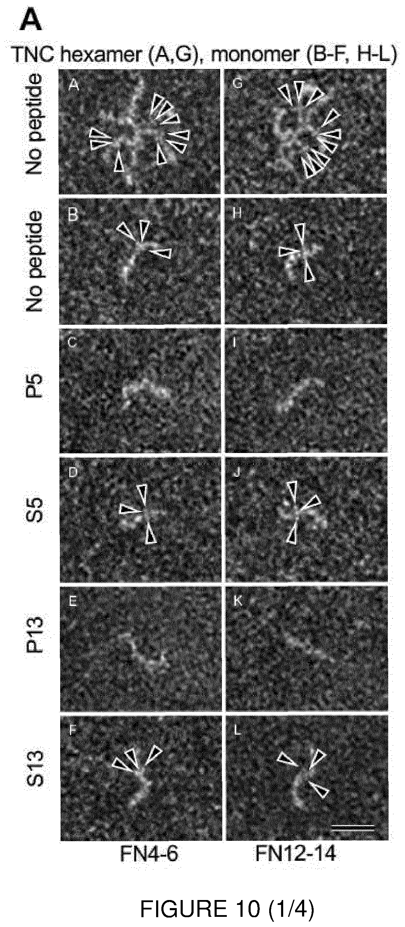

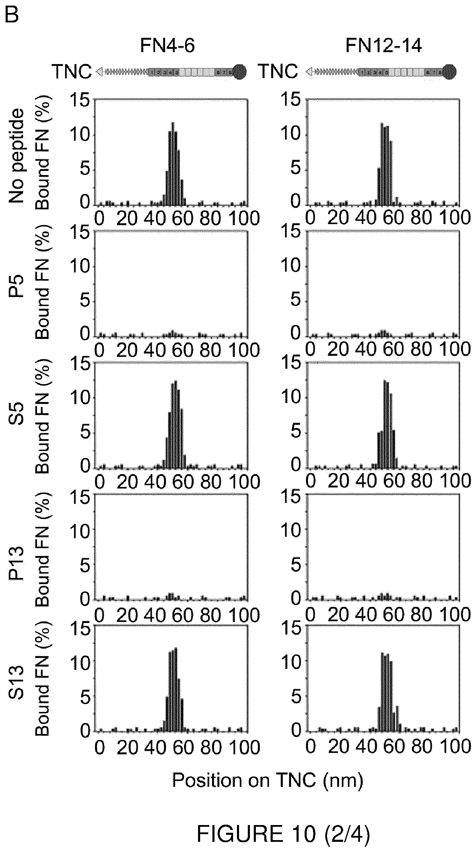

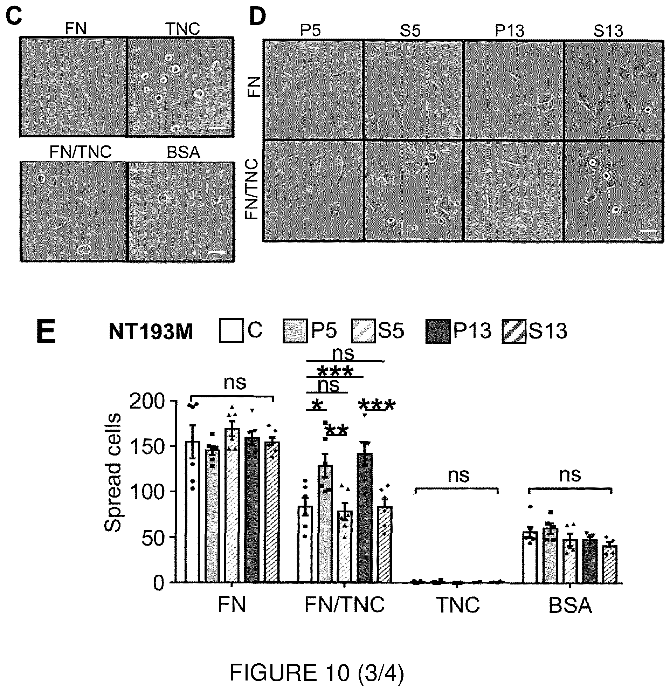

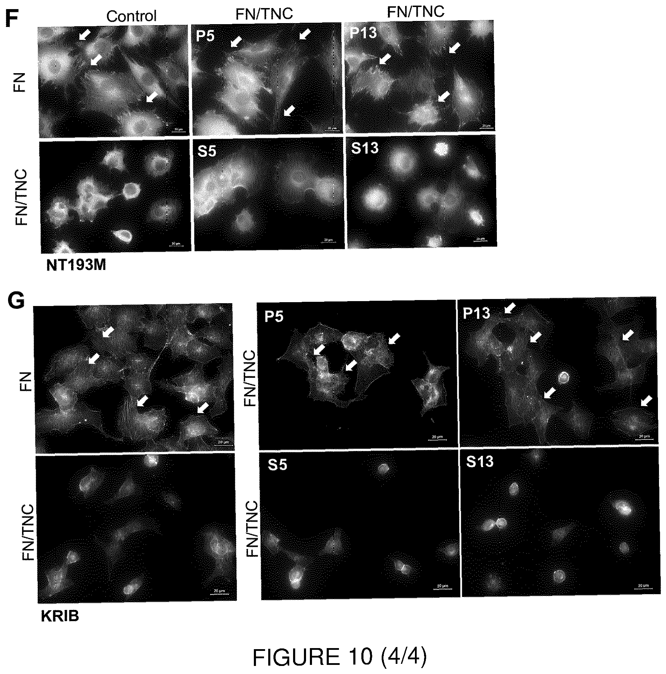

- Figures 10A, B represent negative EM images of binding of FN4-6 and FN12-14, respectively to TNC in the presence or absence of P5, S5, P13 or S13. TNC as hexamer or monomer is shown. Arrows point at the trimers of FN4-6 or FN12-14 bound to TNC. Figure 10B represents diagrams illustrating and quantifying binding of FN4-6 and FN12-14, respectively to TNC. Ordinate represents binding of FN4-6 and FN12-14 along the length of the TNC molecule (nm), abscissa represents relative binding of FN4-6 and FN12-14 (%) Note that FN molecule binding is blocked by P5 and P13, yet not by S5 nor S13. Figure 10 C-D shows phase-contrast images of cell adhesion measurement upon plating of NT193M cells for one hour on FN, FN/TNC, TNC and BSA in serum-free medium (control C) or in presence of peptides P5, P13, S5 and S13 that were added during the coating at 25µm (TNC, 1.25nM). Figure 10E is a diagram quantifying the spread cells 3h after plating in serum-free medium (control C) or in presence of peptides P5, P13, S5 or S13 (ordinate represents the coated surface and the coordinate the number of spread cells, (*p<0.05; **p<0.01; ***p<0.001; ns = not statistically significant). Figure 10F and 10G represent images of fixed and stained NT193M cells (Figure F) and KRIB cells 3h after plating (Figure 10G). Arrows point at vinculin positive focal adhesions (Figure 10F) and actin stress fibers (Figure 10G) that were seen in cells that adhered on FN but not on FN/TNC. Addition of P5 or P13, respectively (yet not S5 or S13) restored cell spreading, focal adhesions and actin stress fibers in cells plated on FN/TNC.

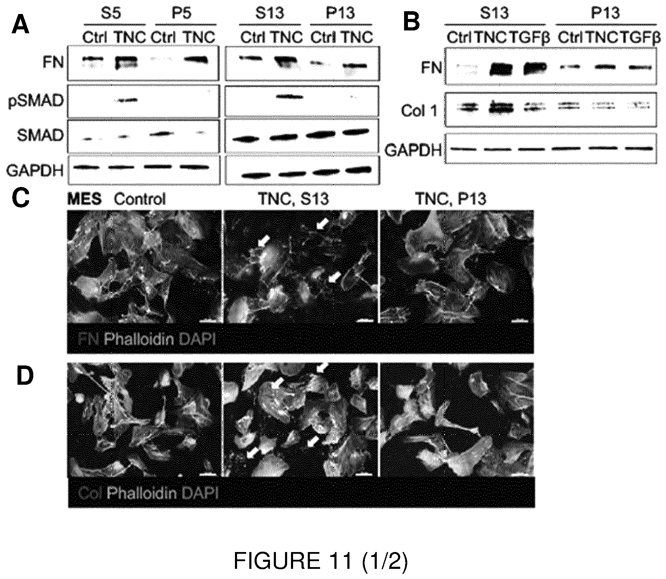

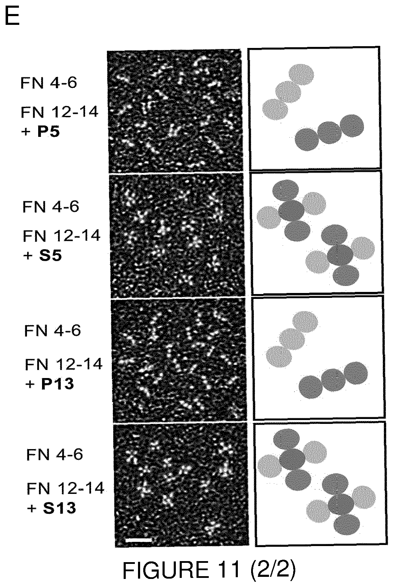

- Figure 1 1A, B are images of western blot results from mesangial cells (MES) incubated for 4 hours with TNC (figure 11A, B) or TGFβ (figure 11B) and the peptides S5, P5, S13 and P13 as indicated. Expression of fibronectin (FN), Smad2 and phospho-Smad2 (figure 11A) and of fibronectin (FN) and collagen 1 (Col 1) is shown (figure 11B) with Glyceraldehyde 3-phosphate dehydrogenase (GAPDH) as control. Figure 11 C, D are images of fibronectin (FN) and Collagen 1 (Col) matrix assembly on top of cultured mesangial cells (MES) for 4 hours after fixation (without permeabilization) and staining for FN or Col.. Arrows point at fibronectin (FN) and collagen 1 (Col) matrix networks outside the cells that form upon addition of TNC in the presence of S13 but not in the presence of P13. Figure 11E are images of negative EM imaging showing binding of FN4-6 to FN12-14 in the FN5 and FN13 domain, respectively (Figure 11E) and inhibition of binding by P5 and P13 (yet not S5 and S13) (Figure 11E). Note in presence of S5 or S13 the cross shaped structures form.

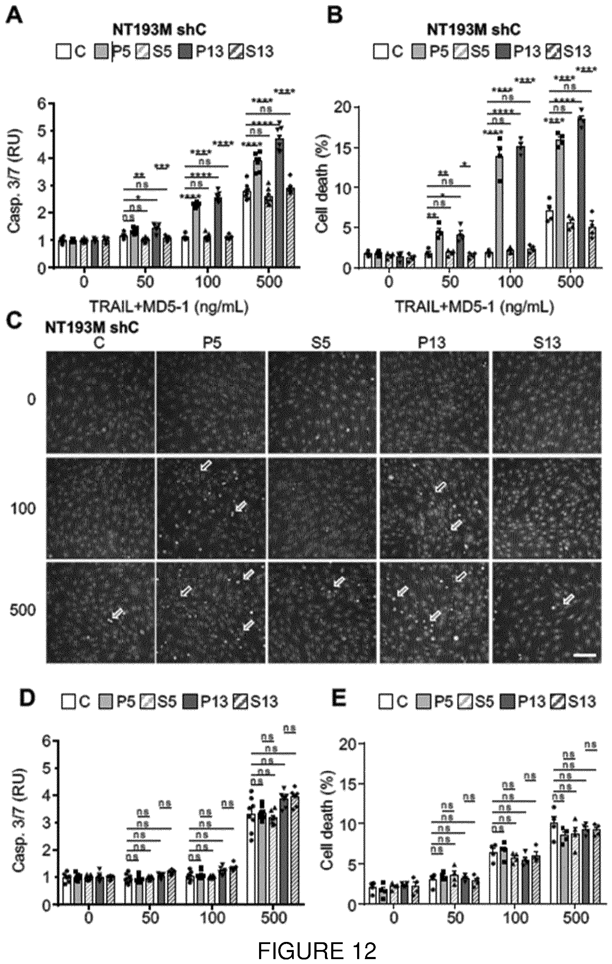

- Figure 12 shows effects of peptide P5 (P5), peptide P13 (P13), peptide S5 (S5), peptide S13 (S13) on TRAIL-induced cell death in NT193M tumor cells. Figure 12 A is a diagram representing the capsase 3/7 activity (ordinate, concentration of TRAIL and MD5-1 (ng/ml), coordinate is relative units (RU) of caspase 3/7 activity). Figure 12 B is a diagram representing cell death (ordinate, concentration of TRAIL and MD5-1 (ng/ml), coordinate is % of dead cells, (*p<0.05; **p<0.01;; ****p<0.0001; ns = not statistically significant)).Figure 12C is a representative image of cells stained with acridin orange and ethidium bromide. Arrows point at dead cells. On the left the amount of TRAIL and MD5-1 (ng/ml) is indicated: 0, 100 or 500 ng/ml. Figure 12C shows caspase 3/7 activity and number of dead cells in NT193shTNC cells in the presence or absence of peptides P5, S5, P13 or S13. Whereas S5 and S13 nor TRAIL together with MD5-1 did induce caspase 3/7 activity (as readout for apoptosis) nor cell death (as measured by incorporation of ethidium bromide), P5 and P13 largely increased caspase 3/7 activity and cell death in some 15% of cells (comparison to 1-5% of control cells) (Figure 12A-C). Figure 12 D is a diagram representing the caspase 3/7 activity (ordinate, concentration of TRAIL and MD5-1 (ng/ml), coordinate is relative units (RU) of caspase 3/7 activity of TRAIL-induced cell death in NT193M tumor cells with a TNC knockdown. Figure 12 E is a diagram representing cell death of TRAIL-induced cell death in NT193M tumor cells (abscissa is the concentration of TRAIL and MD5-1 (ng/ml), ordinate is % of dead cells, ns means non specific).Tenascin-C (TNC, expressed by the NT193M tumor cell), is instrumental in this response as neither P5 nor P13 induced cell death in NT193M cells with a TNC knockdown (Figure 12D, E).

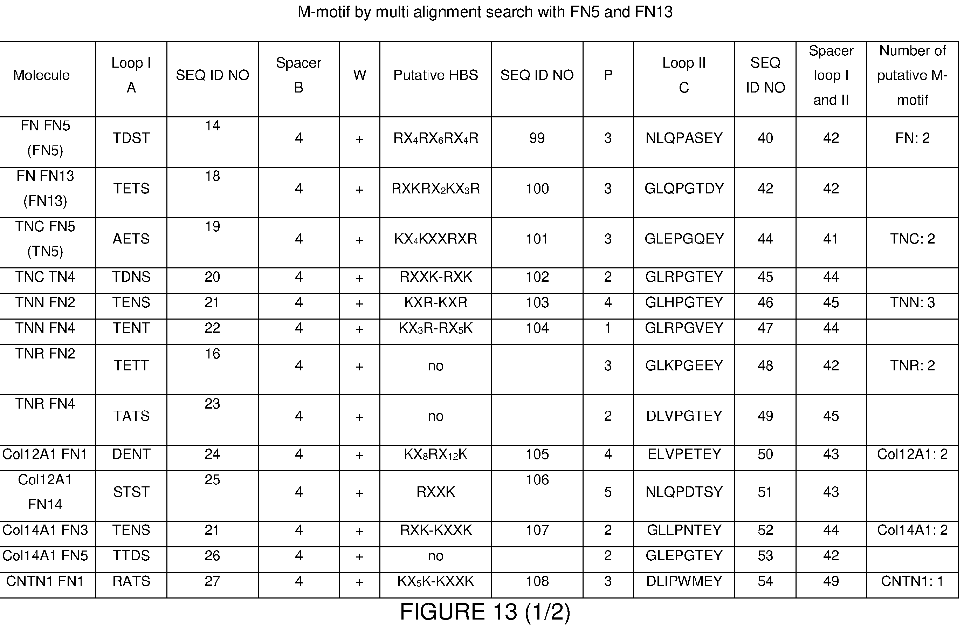

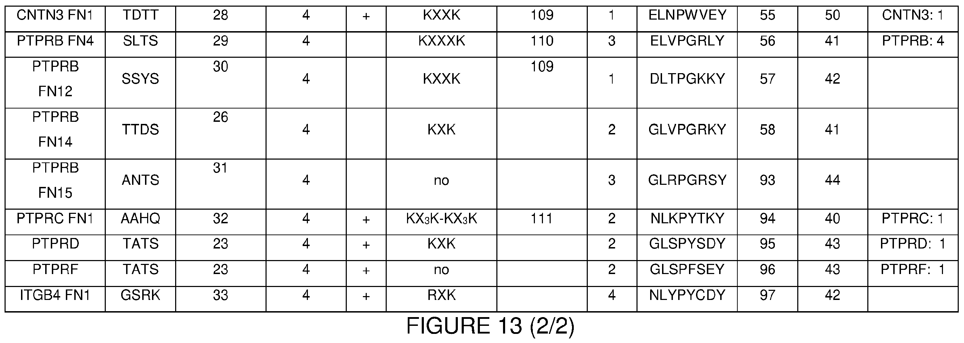

- Figure 13 is a table indicating molecules with putative M-motif and corresponding sequences of Loop I and Loop II consensus. This table also mentions the landmark Tryptophan (W) that is placed between Loop I and Loop II, 4 amino acid apart from Loop I, positively charged amino acids Arginin (R) and Lysin (K) generating proven (FN13, Bloom et al. [1],) and putative Heparin Binding Sites (HBS), the length of the spacer between Loop I and Loop II, the number of prolines (P) within the spacer sequence between Loop I and Loop II, Sequence identification number (SEQ ID NO) for Loop I and Loop II and the number of M-motifs in the respective molecule.

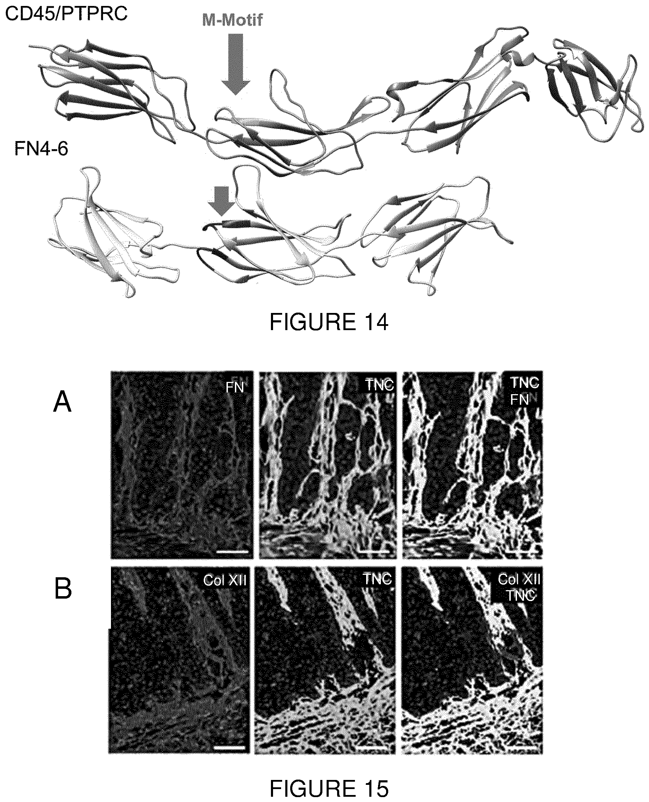

- Figure 14 represents a schematic model of CD45/PTPRC in comparison to a model of FN4-6. The M-motif in both molecules is marked with an arrow.
- Figure 15 are images of tumor tissue from a 4NQO-induced tongue OSCC stained with antibodies against Tenascin-C (TNC) and fibronectin (FN) (Figure 15A) and Collagen 12 (CoIXII) and Tenascin-C (TNC) indicating overlapping networks (Figure 15B).

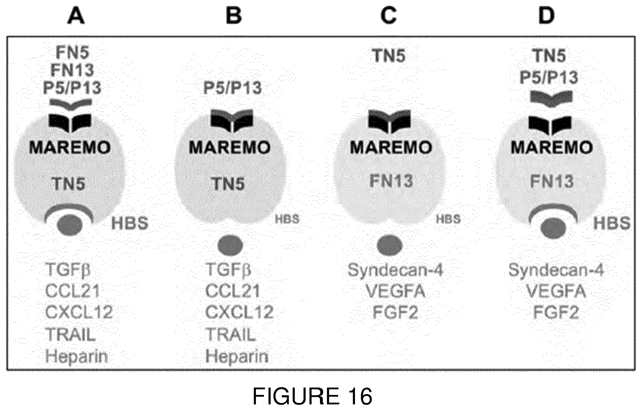

- Figure 16 A-D represents a cartoon of interactions of TNC (TN5) with FN and soluble factors. On these figures the Matrix Binding Motif (M-motif) is mentioned as MAREMO. Figure 16A and B represent TGFβ binding in TNC (TN5) which is competed by heparin. In addition to TGFβ also CXCL12, CCL21 and TRAIL bind in TNC (TN5) probably in the HBS as binding is competed by heparin. Figure 16C shows that P5 and P13, mimicking the M-motif in FN, also bind to TNC (TN5). Binding of P5 and P13 is not competed by heparin suggesting that these peptides do not bind in the HBS but in the M-motif. Binding of P5 and P13 to TNC inhibits binding of TGFβ and of the chemokines to TNC presumably by an allosteric conformational change thereby masking the HBS. Figure 16D represents the identification of binding sites for TNC and syndecan-4 in FN13 at opposite sites within the FN13 molecule. As VEGFA and FGF2 also bind to FN13 in a heparin competitive manner, it is likely that they share the binding site with syndecan-4 in the HBS. TNC binds in FN13 within the M-motif that is opposite to the HBS. This interaction may cause an allosteric conformational change that abrogates binding of syndecan-4 (and potentially VEGFA and others) to the HBS in FN (FN13).

### EXAMPLES

### Example 1 : Identification of Matrix Binding Motif (M-motif) on Tenascin-C (TNC) and compounds binding the Matrix Binding Motif (M-motif) on Tenascin-C

### I. Materials and Methods

### Cell culture

The NT193 murine breast cancer cell line has been established from a primary MMTV-NeuNT breast tumor (Arpel et al., 2014 [40]). *Tnc* gene knockdown in NT193M shC and shTNC has been realized using lentiviral particles, as previously described (lentiviral particles: shC-SHC202V; shTNC-TRCN0000312138 from Sigma, (Sun et al., 2019 [13]). NT193M cells were cultured in Dulbecco's modified Eagle's medium (DMEM Dutscher L0104-500) containing 4.5 g/L glucose, L-glutamine, sodium pyruvate supplemented with 10% of inactivated fetal bovine serum (FBS, Dutscher S1510-500), penicillin (10000 U/mL), streptomycin (10 mg/mL) (Dutscher P06-07100). Selection pressure was maintained over time using puromycin (10 µg/mL, Thermo Fisher A11138-03). KRIB cells (v-Ki-ras transformed human osteosarcoma cells (Berlin et al., 1993)) was maintained in DMEM containing 4.5 g/L glucose, L-glutamine, sodium pyruvate, supplemented with 10% of inactivated FBS, penicillin (10000 U/mL), streptomycin (10 mg/mL). DC2.4 murine dendritic cells (Shen et al., 1997 [41]) were cultured in Roswell Park Memorial Institute 1640 (RPMI-1640, Dutscher L0500-500) medium containing 4.5 g/L glucose, L-glutamine, sodium pyruvate, with HEPES (20mM, Merck PHG0001) supplemented with 10% of inactivated Foetal Bovine Serum (FBS), penicillin (10 000 U/mL), streptomycin (10 mg/mL). Human lymphatic endothelial cells, LECs (ATCC, HDMVECn, PCS-110-010, 2018) were cultured in Endothelial Cell Growth Medium (ECGM Promocell C22110) with penicillin (10 000 U/mL), streptomycin (10 mg/mL) and a supplemental growth factor cocktail according to Promocell. Mesangial cells (MES, Sarrab et al., 2011 [42]) were cultured in RPMI-1640 (Dutscher L0500-500containing 4.5 g/L glucose, L-glutamine, sodium pyruvate, supplemented with 10% of inactivated FBS, 1% Insulin-transferrin-selenite (Sigma 11884), penicillin (10000 U/mL) and streptomycin (10 mg/mL). Cells were kept at 33°C to proliferate in a humidified atmosphere of 5% CO₂. When indicated, cells were kept at 37°C to differentiate upon degradation of the SV40 T-antigen. New vials of cells were thawed each 10-15 trypsinizations (Trypsin-EDTA Ca²⁺, Mg²⁺ free, Dutscher P10-022100 + P10-15100) and cell culture was realized at 37°C in a humidified atmosphere of 5% CO₂.

### TNC cloning and purification

Recombinant human his-tagged TNC (hTNC) and murine strep-tagged TNC (mTNC) was used. hTNC and mTNC were purified as previously described (Giblin et al., 2018 [43]; Huang et al., 2001 [44], Spenlé et al., 2020 [45]).

### Mass spectrometry analysis

Following mTNC purification, 1 M NaCl eluted proteins (as mentioned above) were subjected to acetone precipitation to reduce the volume and salt concentration. With ice-cold acetone (four times the sample volume) the proteins were precipitated at -20°C overnight, followed by centrifugation at 15,000 g for 10 min at 4°C. After discarding the supernatant, the pellet was air-dried at room temperature, i.e. 20°C, for 10 min. Then the protein pellet was resuspended in 8 M urea (Merck 33247), 50 mM triethylammoniumbicarbonate buffer (Merck T7408). The samples were then incubated with DTT (5 mM) (Merck 43819) for 60 min followed by 2-chloroacetamide (40 mM) (Merck 22790) for 30 min in the dark at room temperature, i.e. 20°C, for reduction and alkylation of disulfide bonds. Next, the samples were digested by lysyl endopeptidase (Promega V1671) for 4h. The sample urea concentration was then reduced to 2M urea by adding triethylammoniumbicarbonate buffer and trypsin (Promega V5280) digestion was performed overnight. Finally, the digestion was stopped by 1% formic acid and peptides were purified with SDB-RP stage tips as previously described (Rappsilber et al., 2007 [54]). Peptides were analyzed using the quadrupole-Orbitrap-based Q Exactive Plus mass spectrometer (Thermo Scientific) at the Cologne Cluster of Excellence proteomics core facility. The relative quantification of the proteins was performed in R program with the artMS plugin (Jimenez-Morales et al., 2020 [55]).

### Peptides and ECM proteins / fragments

P5 (STDSTSAPASNLQPASEYS (SEQ ID NO 59), P13 (STETTSAPASGLQPGTDYS SEQ ID NO 62)), S5 (STTDSSAPASYESAPGLNS (SEQ ID NO 63)) and S13 (STTETSAPASYDTGPQLGS (SEQ ID NO 64)) (Thermo Fisher PEP95UNMOD) were diluted in PBS at a stock concentration of 1 mg/mL and stored at -20°C. Recombinant human fibronectin (FN) was expressed and purified as previously described (Huang et al., 2001 [44]). FN plasmids encoding FN4-6 (e.g. HBP/III4-6), FN4-5 (e.g. HBP/III4,5), fibronectin 5 (FN5) (e.g. HBP/III5) derived from Angeles Garcia-Pardo (Moyano et al., 1997 [45]) and, fibronectin 13 (FN13n glutathione (GST) and histidine (His) tagged) from Laird Bloom (Bloom et al., 1999 [47]) were purified as described (Huang et al., 2001 [44]). The human TN1-5, FN4-6 and FN12-14 sequences were cloned into sleeping beauty transposon vectors with a N-terminal double Strep-tag and a bone morphogenic 40 (BM40) signal peptide. Finally, all molecules were dialyzed against Tris buffered saline (TBS) (50mM Tris, 150 mM NaCl, pH 7.4) (TBS Fisher Bioreagents BP2472-1).

The fibronectin type III domains 1-5 and domains 4-5 of human tenascin-C (UniProtKB/Swiss-Prot: P24821|aa: 625-1075 and aa: 895-1075) and fibronectin type III domains 4-6 and 12-14 of human fibronectin (UniProtKB/Swiss-Prot: P02751|aa: 909-1175 and aa: 1635-1908) were cloned into sleeping beauty transposon vectors (https://www.sciencedirect.com/science/article/pii/S0022283600940476) modified with a N-terminal double Strep-tag and a BM40 signal peptide. Adherent HEK293 cells were transfected with Fugene HD (Promega E2312) and selected with 3 µg/ml puromycin (Serva 33835). Protein expression was induced in Triple flasks (Thermo Fisher Scientific 734-2001) with 1 µg/ml doxycycline (Sigma D9891) containing DMEM/F12 medium/2% FCS and the medium was collected and replaced every 2-3 day. The Strep-tagged proteins were then purified with Streptactin XT Sepharose (IBA 2-5010-025) according to the manufacturer protocol and the proteins were then dialyzed 3 times against Tris buffered saline (TBS) (50mM Tris, 150 mM NaCl, pH 7.4) and stored at 4° C.

### Binding assay (by ELISA)

Protein biotinylation was performed as previously described (Pankhurst et al., 1998 [48]). 2mg of FN4-6 and FN12-14 were dialyzed 3 times against 50 mM bicarbonate buffer/ 500 mM NaCl, pH 8.3 and biotinylated with 20-fold molar excess Biotin-Ahx-NHS (Iris Biotech RL-2020.0500). For the TN1-5 / biotinylated FN4-6 or TN1-5 / biotinylated FN12-14 binding assay, TN1-5 was diluted in TBS pH 7.4, and 10 µg/ml (0.5µg/well) were coated onto 96-well plates (Nunc Maxisorb 735-0083) at 4° C overnight. After washing with TBS, unspecific binding sites were blocked at room temperature, i.e. 20°C, with 100 µL Pierce Protein-free blocking solution (Thermo Fisher 37570) for 1 hour. Biotinylated ligands were then serial diluted in protein-free blocking solution to concentrations from 1 µM to 1 nM and 50 µL were used per well. After 90 minutes incubation at room temperature the ELISA plates were carefully washed 3 times with TBS buffer and a HRP conjugated anti Biotin antibody 1:10000 (Aviva Systems Biology OAIA00064) in protein-free blocking solution was applied for 1 hour at room temperature and the plates were washed 3 times. Horseradish peroxidase activity was detected with 50 µL 1-Step Ultra TMB ELISA substrate solution (Thermo Fisher Scientific 34029). Then the reaction was stopped with 50 µL 10% H2SO4 and the absorbance was measured at 450 nm. The binding curves were plotted and analyzed with GraphPad Prism 5.

### Surface Plasmon Resonance (SPR) analysis

SPR binding experiments were performed with a Biacore 2000 instrument (Biacore) at 25°C. TNC was immobilized at high surface density (around 7000 resonance units) on an activated CM5 ship (GE Healthcare BR-1000-12) using a standard amine-coupling procedure according to the manufacturer's instruction. Soluble molecules were added at a concentration of 10 µg/mL in 10 mM sodium acetate, pH 5.0, at a flow rate of 5 µL/min for 20 minutes before addition of 1 M ethanolamine. CCL21 (R&D Systems 457-6C), CXCL12 (R&D Systems 460-SD-050) and TRAIL (Enzo Lifesciences ALX-201-130-C020) were added to the chip at a flow rate of 10 µL/min in a 10 mM HEPES, 150 mM sodium chloride, 0.005% (v/v) surfactant P20. A blank CM5 chip was used for background correction. The dissociation constant (Kd) was determined using the 1:1 Langmuir association model as described by the manufacturer.

### Negative Electron Microscopy (EM) imaging

The interaction of TNC with chemokines and peptides was visualized by negative staining and transmission electron microscopy as described previously (Bober et al., 2010 [29]). Briefly, TNC samples (20 nM) were incubated with a 3 molar excess of chemokines or peptides for 1 hour at 37°C in TBS, pH 7.4. For visualization, the proteins were conjugated with 5 nm colloidal gold (Baschong and Wrigley, 1990). For inhibition experiments, TNC samples were pre-incubated with a 10 molar excess of non-labeled peptides, or a 10 molar excess of heparin (10 molar, Merck 1304016), for 1 hour at 37°C. Specimens were examined in a Philips/FEI CM 100 TWIN transmission electron microscope operated at 60 kV accelerating voltage. Images were recorded with a side-mounted Olympus Veleta camera with a resolution of 2048 x 2048 pixels (2k x 2K) and the ITEM acquisitions software.

Binding of molecules to TNC was determined by counting the number of gold particles along the length of the TNC monomer. Numbers from 500 randomly picked distinct TNC molecules were determined as % of all bound molecules. As positive control, TGFβ (R&D Systems 240-B-010) was used that was shown to bind in the TNfnIII5. Previously, negative controls Epidermal Growth Factor (EGF) and Bovine Serum Albumin (BSA) (shown not to bind to TNC) were used, respectively (De Laporte et al., 2013 [3]; Spenlé et al., 2020 [45]). For peptide competition, peptides (10 molar excess) were incubated with CXCL12, CCL21 or TRAIL (each 20 nM) for 1 hour in TBS before mixing with TNC for another hour before adsorbing of the mixture on the EM grid, followed by washing steps and standard negative staining procedures.

### Sequence and bioinformatic tools

UniProt protein ID sequences are listed in the Table 1 below.

**Table 1: uniprot ID sequence number**

| **Protein** | **UniProt ID** |
|---|---|
| Fibronectin *Homo sapiens (Human)* | P02751 |
| Fibronectin *Mus musculus (Mouse)* | P11276 |
| Fibronectin *Rattus norvegicus (Rat)* | P04937 |
| Fibronectin *Bos taurus (Bovine)* | P07589 |
| Fibronectin *Canis lupus familiaris (Dog)* | Q28275 |
| Fibronectin *Equus caballus (Horse)* | Q28377 |
| Fibronectin *Gallus gallus (Chicken)* | P11722 |
| Fibronectin *Xenopus Iaevis (African clawed frog)* | Q91740 |
| Tenascin-C *Homo sapiens (Human)* | P24821 |
| Tenascin-X *Homo sapiens (Human)* | P22105 |
| Tenascin-N *Homo sapiens (Human)* | Q9UQP3 |
| Tenascin-R *Homo sapiens (Human)* | Q92752 |
| Collagen XII A1 *Homo sapiens (Human)* | Q99715 |
| Collagen XIV A1 *Homo sapiens (Human)* | Q05707 |
| Receptor-type tyrosine-protein phosphatase F (PTPRF) *Homo sapiens (Human)* | P10586 |
| Receptor-type tyrosine-protein phosphatase D (PTPRD) *Homo sapiens (Human)* | P23468 |
| Integrin beta-4 (ITGB4) *Homo sapiens (Human)* | P16144 |

Sequence multiple alignments were performed using Clustal Omega (Madeira et al., 2019 [31]). The 3D models are obtained from RCSB Protein Data Bank and listed in Table2 below

**Table 2 : PDB ID sequence numbers of 3D models**

| **Model** | **PDB ID** |
|---|---|
| FNfnIII 4-7 | 6MFA |
| FNfnIII 12-14 | 3R8Q |
| TNfnIII 4-6 | 1TDQ |
| COL12A1fnIII-13 | 3T1W |
| CD45fn III 1-2 | 5FMV |

The Evolutionary Trace Annotation (ETA) Server has been used to detect the Loops I and II in 3D models of fibronectin (FN) molecules (Matthew Ward et al., 2009 [49]). Peptide interacting models with fibronectin type-III domains were predicted using PEP-FOLD 3.5 (Lamiable et al., 2016 [50]) and 3D models were visualized with UCSF Chimera program (Pettersen et al., 2004 [51]). Residues interaction contact maps were designed using COCOMAP (bioCOmplexes COntact MAPS, (Vangone et al., 2012 [52]) as previously described (Ksouri et al., 2018 [53]).

### Cell adhesion assay

In 96-well plates, coating with purified FN and TNC was realized in 0.01% Tween-20 PBS at 1µg/cm² before saturation with heat inactivated BSA as previously described (Huang et al., 2001). The peptides were added to TNC in 0.01% Tween-20 at a concentration of 50 µg/mL 10 minutes before coating (TNC, 1.25 nM, peptides, 25 µM). NT193 cells were then seeded at a concentration of 5 000 cells/well in culture medium without FBS and allowed to adhere at 37°C. Phase contrast pictures were taken after 1 and 3 hours of adhesion and images were analyzed for cell counting with the Zen Lite 2012 edition software.

### Immunofluorescence staining

Following the protocol of cell adhesion, NT193M, KRIB and MES cells were fixed with 4% paraformaldehyde (PFA, Santa Cruz SC-281692) in PBS for 10 minutes, then permeabilized with 0.25% Triton (EUROMEDEX 2000-A) in PBS for 15 minutes (except MES with no permeabilization). After washing, blocking solution (5% normal goat serum in PBS, Southern Biotech 0060-01) was applied for 30 minutes before primary antibody anti-vinculin (clone VLN01, Invitrogen) incubation of 1 hour at room temperature, i.e. 20°C. Corresponding secondary antibodies and rhodamine coupled phalloidin (Thermo Fisher P1951) were incubated 45 minutes after washing. The slides were then washed, stained with DAPI (Sigma D9542) for 10 minutes at room temperature, i.e. 20°C and finally sealed with Fluoromount-GTM (Thermo Fisher 00-4958-02). Fluorescence pictures were acquired with a Zeiss Axio Imager Z2 microscope and analyzed with Zen Lite 2012 edition software. Antibodies are listed in Table 3 below.

### Tissue Immunofluorescence staining (IF)

Tissue sections (8 µm thick) from murine 4-Nitroquinoline 1-oxide (4NQO) tongue tumors (Spenlé et al., 2020 [45]) were incubated for 1 hour with a blocking solution (5% normal goat serum) at room temperature, i.e. 20°C, before overnight incubation with primary antibodies at 4°C. After washing, the slides were incubated with corresponding secondary antibodies for 1 hour at room temperature. Nuclei were stained with DAPI (Sigma D9542) and slides were sealed. Fluorescence pictures were acquired with a Zeiss Axio Imager Z2 microscope and analyzed with Zen Lite 2012 edition software. The image acquisition setting (microscope, magnification, light intensity, exposure time) was kept constant per experiment conditions. Antibodies are listed in Table 3 below.

**Table 3: Antibodies**

| **Target** | **Antibody type / Application** | **References** |
|---|---|---|
| GAPDH | Primary / WB | Cell Signalling 2118S |
| FN | Primary / WB, IF | Sigma F3648 |
| TNC | Primary / WB, IF | Spenle et al., 2015 [63] |
| Smad 2/3 | Primary / WB | Cell Signalling 3102S |
| p-Smad 2 | Primary / WB | Cell Signalling 3108S |
| Col I | Primary / WB | Abcam Ab34710 |
| Col XII | Primary / IF | Spenlé et el, 2020 [45] |
| Vinculin | Primary / IF | Sigma SAB4200729 |
| Anti-rabbit | Secondary / WB | Cell Signalling 7074S |
| Anti-rat | Secondary / WB | Cell Signalling 7077S |
| Anti-mouse | Secondary / WB | Cell Signalling 7076S |
| Anti-rabbit | Secondary / IF | Jackson Lab 111-165-003 |
| Anti-rat | Secondary / IF | Jackson Lab A11006 |
| Anti-mouse | Secondary / IF | Jackson Lab 115-165-003 |

In the above table 3, primary means primary antibody, secondary means secondary antibody, WB means Western Blot and IF means ImmunoFluorescence

### Chemoretention assay

DC2.4 cells chemoretention assay was performed in 5 µm-pore size transwell chambers (Corning 3421). The lower surface of the transwell inserts was coated with FN, TNC or collagen I (Col1) as described in the cell adhesion assay. The peptides were incubated overnight at 4°C on the coating at a concentration of 50 µg/mL. The insert surfaces were washed before cell plating. In the upper chamber, 20 000 DC2.4 cells were seeded in 1% FBS medium. The same medium containing CCL21 (200 µg/mL) or CXCL12 (100 ng/mL) was used in the lower chamber as a chemoattractant. After 5 hours of migration at 37°C, cells were fixed with ice cold methanol. Cells remaining in the upper chamber were removed with a cotton dub and the cells on the lower side were stained with DAPI. Fluorescence pictures were acquired with a Zeiss Axio Imager Z2 microscope and analyzed for cell counting with Zen Lite 2012 edition software.

### Gene expression analysis by qRTPCR

DC2.4 and LEC cells were seeded at 500 000 cells in 6-well plates (Falcon 353046) in corresponding media prior to overnight starving in serum-free medium. Cells were then treated with the peptides at 50 µg/mL, in serum depleted medium, for 24 hours. Cell lysates were prepared after cell scraping for total RNA extraction with TriReagent (Life Technologies AM9738). RNA was treated with DNAse I (Roche 04716728001) and reverse transcribed (MultiScribe reverse transcriptase, Applied Biosystems 10117254) before determination of the concentration and the purity with NanoDrop 2000 (Thermo Fisher). qPCR was done on cDNA (diluted 1:5 in water) with a QuantStudio™ 5 Real-Time PCR System (Applied Biosystems) using TaqMan™ Fast Advanced Master Mix (Applied Diosystems 4444557) or Master Mix PCR Power SYBR™ Green (Applied Biosystems™ 4309155). *Gapdh* was used as housekeeping gene in the comparative cycle threshold method (2-ddCt). Primers are listed in Table 4 below. The primers for *Gapdh* were primers commercialized by Thermofisher under the commercial trademark Taqman probe: Mm99999915_g1, the primers for *Cxcl12* were primers commercialized by Thermofisher under the commercial trademark: Taqman probe: Mm00445553_m1.

**Table 4 : primer sequence**

| Gene symbol | Primer name | Sequence (5'-3') | SEQ ID NO |
|---|---|---|---|
| *Ccl21* | mCCL21_F | TCCAAGGGCTGCAAGAGA | 65 |
| | mCCL21_R | TGAAGTTCGTGGGGGATCT | 66 |
| *Ccr7* | mCCR7_F | CTCCTTGTCATTTTCCAGGTG | 67 |
| | mCCR7_R | TGGTATTCTCGCCGATGTAGT | 68 |
| *Cd80* | mCD80_F | CCATGTCCAAGGCTCATTCT | 69 |
| | mCD80_R | GGCAAGGCAGCAATACCTTA | 70 |
| *Cd86* | mCD86_F | CATGGGCTTGGCAATCCTTA | 71 |
| | mCD86_R | AAATGGGCACGGGAGATATG | 72 |

### Protein expression assessment by immunoblotting and ELISA

Human mesangial cells (MES) (Am J Physiol Renal Physiol. 2011 Nov; 301 (5): F1131-F1138 [59]) were seeded at 1,6 x 10⁵ cells in a 6 well plate (Dutscher, 2515082) for 3 days at 33°C and then 5 days at 37°C in Roswell Park Memorial Institute 1640 (RPMI-1640) medium (Dutscher, L0500-500) supplemented with 10% of Fetal Bovine Serum (FBS) (Dutscher, S1810-500), penicillin (10 000 U/mL), streptomycin (10 mg/mL) (Dutscher, P06-07100), 1% Insulin/transferrin/selenium (Sigma, I1884).The cells were starved overnight and then treated with TNC 10 µg/ml and TGFβ 10 ng/ml (Miltenyi, 130-095-066) with or without P5 or P13 at 50 µg/mL in serum free RPMI medium for 4 hours. Dendritic DC2.4 cells (ATCC) were seeded at 500 000 cells in 6-well plates in RPMI medium prior to overnight starvation in serum-free medium. Cells were then treated with the peptides (P5, S5, P13, S13) at 50 µg/mL, in serum free medium, for 24 hours. Cell lysates were prepared using Radioimmunoprecipitation assay (RIPA) buffer (150mM Tris-HCI pH8, 150mM NaCl, 1% NP-40, 1% sodium deoxycholate, 0.1% SDS) supplemented with protease and phosphatase inhibitors (Pierce EDTA-Free mini tablets, Thermo Fisher A32961) and protein concentration was assessed with a Bradford assay (BioRad 500-0006). For immunoblotting, proteins (20 µg/well) were loaded with Laemmli buffer (BioRad 161-0747) containing 10% β-mercaptoethanol (Merck 1.120006) into precasted 4-20% gradient gels (BioRad 4561094). Electrophoresis and protein transfer on nitrocellulose membranes were realized with BioRad equipment and consumables (Mini-PROTEAN Tetra electrophoresis and Trans-Blot Turbo (registered trademark) systems). The blots were soaked in blocking solution (5% non-fat milk diluted in 0.01% Tween-20 PBS) during 1 hour at room temperature, i.e. 20°C before incubation with primary antibodies overnight at 4°C. Secondary antibodies were incubated 1.5 hour at room temperature. Amersham ECL Western Blotting detection reagent (GE Healthcare RPN2106) or SuperSignal (registered trademark) West Femto Maximum Sensitivity Substrate (Thermo Fisher 34095) were used to detect protein bands in a ChemiDoc (registered trademark) Imager (BioRad). Antibodies are listed in Table 3 above. For ELISA, CCL21 expression was determined by using the 6-Ckine ELISA kit (Thermo Fisher EMCCL21A) according to the manufacturer's instructions. The absorbance of each sample and standard was measured with a plate reader Varioskan (registered trademark) LUX (Thermo Fisher).

### TRAIL killing activity in NT193M cells

Mouse recombinant SUPERKILLERTRAIL (registered trademark) (Enzo Lifesciences ALX-201-130-C020) was used in combination with the MD5-1 antibody (Functional grade, eBioscience™ 16-5883-82) to induce cell death. Briefly, NT193 cells were seeded in their corresponding media in 96-well plates, at 10 000 cells/well, for 24 hours before treatment. TRAIL, MD5-1 or TRAIL+MD5-1 treatments were applied for 16 hours in DMEM (Dutscher L0104-500, final volume 100 µL). Caspases 3 and 7 activity were assessed using Caspase-Glo (registered trademark) 3/7 Assay (registered trademark) according to the manufacturer's instructions (Promega G811A). Cells were incubated with the Caspase-Glo (registered trademark) 3/7 reagent for 1.5 hour before luciferase luminescence measurement with a Varioskan™ LUX (registered trademark) (Thermo Fischer). Cell death and viability was assessed by acridine orange / ethidium bromide (AO/EB) incorporation measurement. Briefly, cells were seeded and treated with TRAIL as previously explained. AO/EB solution (5 µL/well, stock solution at 100 µg/mL in PBS) was mixed to the cells 5 minutes before plate centrifugation (300 x g, 5 minutes). Fluorescence pictures of the centers of each well were acquired using a Motic™ AE31E trinocular microscope (registered trademark) and cell counting was done with Zen Lite 2012 edition software. MD5-1 concentration was maintained at 50 ng/mL
Peptides P5, P13, S5 and S13 were synthetized according to know process of one skilled in the art and were purchase from Thermo Fisher PEP95UNMOD.

### Statistical analysis

Gaussian distribution was tested by the d'Agostino-Pearson normality test. Unpaired t-test (with Welch's correction in case of unequal variance) or ANOVA two-way test were applied when data followed a normal distribution. Otherwise, Mann Whitney test or a non-parametric ANOVA followed by Dunns post-test were used. Analysis and graphical representation were done using GraphPad Prism (version 6). P values < 0.05 were considered statistically significant. Data are representative of at least two individual experiments, expressed as the mean ± SEM. (*p<0.05; **p<0.01; ***p<0.001; ****p<0.0001; ns = not statistically significant).

### II. Results

### 1. Sequence and method of production examples of compounds binding the M-Motif on TNC, in particular peptides of the invention and/or use in the present invention

### a) Identification of sequence conservation in FN13 and FN5

Sequences with an important function are often conserved amongst specie ( Lichtarge et al.,1996 [58]).). The inventors consider that this may also apply to TNC binding sequences within FN. Therefore, the inventors searched for potential inter-species sequence conservation within the FN13 and FN5 domains, respectively. Indeed, the alignment revealed that the sequence of the FN5 and FN13 domain are highly conserved amongst species in particular in two regions that we called Loop I and Loop II (Figure 1 A). Also the FN5 sequence displayed high homology between species (Figure 1A).

Comparing FNIII PDB models in the evolutionary trace annotation server (ETA), the inventors have demonstrated residues with conserved physical and chemical characteristics clustered in FN5 and FN13, in the Loop I and Loop II with the following (human) sequence identity for FN5, TDST (SEQ ID NO 14) (Loop I) and NLQPASEY (SEQ ID NO 40) (Loop II) and for FN13, TETT (SEQ ID NO 16) (Loop I) and GLQPGTDY (SEQ ID NO 42) (Loop II). Loop I in FN5 and FN13 is 4 amino acids (aa) apart from a conserved W. The spacer sequence between Loop I and Loop II is 42 aa in both domains (Figure 1 A).

By using bioinformatics tools and based on the previously characterized three-dimensional structure of FN13 (Sharma et al., 1999 [12]), the inventors investigated M-motif loop I and loop II organization within the FN13 domain in more detail. They demonstrated that both loops are directly adjacent to each other in 3D despite the presence of a 42/43- AA - spacer (Figure 1B). In addition, in the three-dimensional structures of FN12-14 and FN4-7 (Table 2), Loop I and Loop II in FN5 and FN13 are brought into vicinity generating putative niches that resembled each other and which correspond to a Matrix Binding Motif (M-Motif) (Figure 1 B-D)

The localisation of the binding functions was also search by investigating binding of FN4-6, FN4/5, FN5, FN13 or FN12-145, full length TNC. First by determining the half maximal binding (HMB, nM) FN5 was shown to bind to TNC similarly as FN4/5 or FN4-6, a molecule that previously was shown to bind to TNC (Huang et al., 2001) (Figure 1F). By offering TN1-5 as coating, the results obtained by ELISA demonstrate that both FN molecules (FN4-6 and FN12-14) bound to TN1-5 with a similar Kd (44 nM and 52 nM, respectively) (Figure 1G). The negative electron microcopy (EM) imaging results demonstrated that FN4-6 and FN12-14 bound to TNC in the center of each TNC monomer (TN5), and in the center of each trimer (FN5 and FN13, respectively) as indicated by the cross shaped structures (Figure 1H, I). This results support that the central FN5 and FN12 domains interacted with TNC. By incubating FN4-6 and FN12-14 in a homotypic or heterotypic manner it has been shown that each triplet molecule did not bind to each other homotypically. Yet, when mixed, FN4-6 and FN12-14 formed cross-shaped structures, supporting a major interaction domain in FN5 and FN13 which may be involved in FN network formation in vivo (Figure 1J).

### b) Identification of a matrix regulating motif MAREMO, (M-motif) in TNC and other ECM molecules

Expression of TNC and Col12 completely overlapped in tumor tissue suggesting a potential interaction *in vivo.* In addition, whereas Col12 expression was high in 4NQO-induced tongue tumors, its expression was very low in TNCKO tongue tumors suggesting an unknown interdependence (Spenle et al, 2020 [45]).

The M-motif consensus sequence was searched and found in FN5 and FN13 (Loop I, Loop II and spacing by some 40 amino acids) in Col12A1. Indeed, they found one FN13-like M-motif consensus in Col12A1 where Loop I and Loop II are separated by 44 amino acids. In addition there were at least 4 additional FN5-like TBM consensus sequences present in Col 12A1 with lower sequence homology. The predicted 3D structure of Col12A1 overlaps well with that of FN13 in the Loop I and Loop II regions (Figure 2).

Analysis of the TNC sequence for homologies with the M-motif in FN5 and FN13 revealed also a M-motif in TN5 where both motifs showed 100% sequence overlap. Structural modelling revealed strong similarities between TN5 and FN13 (Figure 3A, C).

### c) Design of M-motif mimicking peptides P5 and P13

Next short peptides were designed : peptides of 19 aa length by placing Loop I and Loop II of FN5 and FN13, respectively in vicinity to each other. Both loop sequences were connected with an artificial linker sequence of 5 aa (SAPAS (SEQ ID NO 37)) instead of the 42 aa that are present in the native FN sequence. The spacer comprised a proline in the middle to bring Loop I and Loop II close to each other in order to mimic the spatial organization of the M-motif in FN5 and FN13 respectively. To enhance solubility, a serine was added at each end giving rise to peptide P5 (STDSTSAPASNLQPASEYS (SEQ ID NO 60) and peptide P13 (STETTSAPASGLQPGTDYS (SEQ ID NO 62)). Control peptides were also generated, in which Serine were kept at each end of the molecule and conserved the spacer between Loop I and Loop II (SAPAS (SEQ ID NO 37)) but scrambled the sequence within each loop resulting in peptide S5 (STTDSSAPASYESAPGLNS (SEQ ID NO 63)) and peptide S13 (STTETSAPASYDTGPQLGS (SEQ ID NO 64)).

### d) Binding of peptides P5 and P13 to TNC (TN5)

The inventors reasoned that if the M-motif is important for the interaction of FN with TNC they may see binding of the peptides to TNC, respectively. In that purpose, the interaction probabilities between the amino acids constituting the peptides and FN5 was determined. This modelling predicted a major interaction site for P5 and P13, respectively in Loop II of FN5 (Figure 2D, Table 5)

**Table 5: Modeling of minimal distances between TNC (fibronectin FN5 domain) and the peptides P5 / P13**

| **Peptide P5** | | | | **FN5 Domain** | | | | **Interaction** | |
|---|---|---|---|---|---|---|---|---|---|
| AA | N°AA | Atom | Chain | AA | N°AA | Atom | Chain | Distance (Å) | Interaction type |
| ALA | 15 | CB | B | TYR | 1042 | CD2 | A | 2.76 | Phob-PhIL |
| GLU | 17 | OE1 | B | ARG | 1054 | CD | A | 2.88 | Phil-Phil |
| PRO | 14 | CB | B | PRO | 1052 | O | A | 2.87 | Phob-Phob |
| SER | 19 | OG | B | ASN | 1043 | O | A | 2.59 | Phil - Phil |
| PRO | 14 | CG | B | ARG | 1054 | N | A | 2.29 | Phob - Phil |
| TYR | 18 | OH | B | LYS | 1050 | O | A | 2.67 | Phil - Phil |
| TYR | 18 | OH | B | VAL | 1048 | CG2 | A | 1.86 | Phil - Phob |
| ALA | 15 | CA | B | TYR | 1051 | OH | A | 2.45 | Phob - Phil |
| TYR | 18 | CD2 | B | TYR | 1051 | CE1 | A | 1.99 | Phil - Phil |
| PRO | 14 | O | B | TYR | 1051 | OH | A | 2.67 | Phob - Phil |

| **Peptide P13** | | | | **FN5 Domain** | | | | **Interaction** | |
|---|---|---|---|---|---|---|---|---|---|
| AA | N°AA | Atom | Chain | AA | N°AA | Atom | Chain | Distance (Å) | Interaction type |
| G LY | 15 | N | B | PRO | 1052 | CD | A | 2.69 | Phob - Phob |
| TYR | 18 | CE1 | B | PRO | 1052 | O | A | 1.47 | Phil - Phob |
| LEU | 12 | CD1 | B | TYR | 1042 | CD2 | A | 2.12 | Phob - Phil |
| LEU | 12 | CB | B | TYR | 1051 | OH | A | 1.81 | Phob - Phil |
| SER | 19 | CA | B | ASN | 1055 | ND2 | A | 2.15 | Phil - Phil |
| TYR | 18 | CD2 | B | TYR | 1042 | CE2 | A | 2.83 | Phil - Phil |
| PRO | 14 | CB | B | LYS | 1050 | O | A | 2.59 | Phob - Phi |
| TYR | 18 | CE2 | B | TYR | 1051 | CE2 | A | 2.08 | Phil - Phil |

In table 5 Amino acids represented in the contact map. Atoms, interaction distance and interaction type are listed. Distance in ångström (Å) and interaction types: Phob = hydrophobic, Phil =hydrophylic.

The inventors addressed binding of P5 and P13 to TNC by negative EM imaging.

The negative EM imaging also demonstrated that both peptides P5 and P13 (yet not S5 nor S13) bound similarly mostly in the middle of the TNC monomer, predominantly in two FNIII domains that potentially represent TN4 and TN5 (Figures 5A, B). As TNC (TN5) was previously shown as an interaction site for several molecules including TGFβ (De Laporte et al., 2013 [3]), binding of TGFβ to TNC with that of the peptides was compared. This comparison revealed identical binding patterns of TGFβ and P5/P13 to TNC, respectively revealing a major binding site in the middle of the TNC monomer in TN5 (Figure 5 C-E). By using heparin, the binding of TGFβ to TN5 was studied in context of the whole TNC molecule, and if it was dependent on glycosaminoglycans (GAG) as previously suggested for the isolated TN5 domain (De Laporte et al., 2013) and indeed inhibition of TGFβ binding to TNC by heparin was seen (Figure 5C). This confirms that TN5 binding to TGFβ is dependent on its glycosaminoglycans. Moreover, these results suggest that the putative Heparin Binding Site (HBS) in TNC (TN5) is involved in binding to TGFβ. In contrast to TGFβ, binding of P5 and P13 to TNC was not competed by heparin suggesting that TGFβ and P5/P13 bind at different sequences within TN5, for instance TGFβ in the HBS and P5/P13 in the M-motif (Figure 5A-E).

### f) Binding of TN5 to FN13 through the M-motif

Modelling provides an explanation for how P5/P13 would block binding of TN5 to FN13 (Figure 4). the mode of binding of peptide P5 and P13 to TN5 was addressed in more detail by structural modeling (Figure 6, Table 2). This analysis revealed a close fit in the M-motif where P5 interacts through P14 with Y1051, P1052 and R1054 in TN5, through A15 with Y1042, A1051 in TN5, through Y18 with V1048, K1050 and Y1051 in TN5, through E17 with R1054 in TN5 and through S19 with N1043 in TN5. P13 interaction with TN5 is as follows: L12 with Y1042 and Y1053 in TN5, P14 with 1050 in TN5, Y18 with Y1042, Y1051 and P1052 in TN5, G15 with P1050 in TN5 and S19 with N1055 in TN5 (Figures 6A, B). It is interesting to note that several amino acids in P5 and P13, respectively interact with more than one amino acid in TN5 thus likely explaining the close fit in the M-motif.

These examples clearly demonstrate and illustrate compounds, in particular peptides, which bound the Matrix Binding Motif (M-motif) on Tenascin-C (TNC) of the invention.

### 2. Use of compound binding the Matrix Binding Motif (M-motif) on Tenascin-C (TNC) and effects on Tenascin-C (TNC) properties

### a) Peptides P5 and P13 inhibit binding of CCL21, CXCL12 and TRAIL to tenascin-C

CXCL12, TRAIL and CCL21 have been observed to be regulated by TNC in the tongue tumours (OSCC), and breast tumors (MMTV-NeuNT and NT193) . This regulation modulate the immune cell responses towards TNC (Spenlé et al., 2020 [45]). The inventors asked whether these molecules potentially bind to TNC. Indeed by SPR measurement they found that all investigated molecules bind to TNC with different affinities (CCL21 = 5,8 x 10⁻⁸ M, CXCL12 = 7,9 x 10₋₇ M and TRAIL = 2,8 x 10⁻⁹ M, , Figure 7A-C). By negative EM imaging they visualized where these molecules that were coupled to gold beads bind within TNC. Therefore TNC was incubated with the chemokines or TRAIL in solution before addition of the mixture onto a grid followed by EM. This experiment revealed binding of the gold labelled molecules to three domains within TNC, the EGFL repeats, TN5 and the fibrinogen like globe (FBG). There is more binding to TN5 than to the other domains (Figure 7D-F). Whereas binding to the EGFL repeats is unaltered, heparin blocked binding of the chemokines to TN5 and FBG, indicating that these interactions are glycosaminoglycan (GAG)-chain dependent (Figure 5C, Spenlé et al, 2020 [45]. Comparing the binding profiles of TGFβ and P5/P13 with TNC to that of the chemokines revealed similarities, confirming binding of TRAIL, CXCL12 and CCL21 in TN5 which is the major binding site in TNC (Figure 7D-F).

### b) compound binding the Matrix Binding Motif (M-motif) on Tenascin-C (TNC), for example peptides P5 and P13 inhibit binding of the chemokines / soluble factors to TNC

As the soluble factors (CCL21, CXCL12, TRAIL) and P5/P13 bound in the same TNC domain (TN5) the effect of P5/P13 on the binding to TNC was determined by negative EM imaging by preincubating the peptides with the soluble factors for 60 minutes in TBS (pH7.4) solution before adding TNC for another 60 minutes. In particular, TNC was incubated with the soluble factor-adsorbed gold beads in solution in the presence of the peptides before addition of the mixture onto a grid followed by negative EM imaging and quantification. The images and results obtained demonstrated that addition of P5 or P13 blocked binding of TRAIL, CXCL12 and CCL21 to TNC (whereas the control peptides S5 and S13 did not block binding (Figure 8A-D)).

Thus, this example clearly demonstrates that P5 and P13, respectively, competed binding of TRAIL, CXCL12 and CCL21 to TNC (Figure 8A-D).

This example also clearly demonstrates that compound, in particular peptide P5 and P13, binding Matrix Binding Motif (M-motif) on Tenascin-C (TNC) allows to inhibit and block the binding of soluble factors/chemokine, in particular TRAIL, CXCL12 and CCL21.

### c) Compound binding the Matrix Binding Motif (M-motif) on Tenascin-C (TNC), for example peptides P5 and P13, modify the immune functions of Tenascin-C (TNC)

The expression of CCR7 and CD86 in dendritic DC2.4 was studied. TNC induced Cd86 and Ccr7 in DC2.4 cells and expression of Ccr7 and Cd86 was downregulated by P5 and P13 (Figure 9A and B). As binding of CCL21 and CXCL12 by TNC was instrumental in immobilizing dendritic cells and CD8+ T cells respectively (Spenle et al., 2020 [45], the effect of peptide P5 or P13 on chemoretention by TNC was studied. Immobilization of DC2.4 cells on TNC in context of CCL21 and CXCL12 was determined respectively. The obtained results demonstrate that both P5 and P13 induce a release of cells from the TNC substratum (Figure 9 C -E).

These results clearly demonstrate that compound binding the Matrix Binding Motif (M-motif) on Tenascin-C (TNC), in particular peptide P5 and P13, allows to inhibit and block the binding of soluble factors/chemokines, in particular TRAIL, CXCL12 and CCL21, and also has an effect on the immune functions of Tenascin-C, in particular compound binding the Matrix Binding Motif (M-motif) on Tenascin-C (TNC), modify the chemoretention of TNC

### d) Compound binding the Matrix Binding Motif (M-motif) on Tenascin-C (TNC), for example peptides P5 and P13 inhibit fibronectin homophilic interactions and tenascin-C actions in cell adhesion, matrix expression and matrix assembly

The inventors consider that TNC interacted with FN through the M-motif, thus causing cell rounding on a mixed FN/TNC substratum (Huang et al., 2001). By negative EM imaging binding of TNC to FN4-6 and FN12-14, was respectively investigated. Both FN molecules formed cross shaped structures together with TNC binding in the middle of the TNC molecule (TN5). FN4-6 and FN12-14 binding to TN5 is inhibited by compound P5 and P13, respectively yet not by S5 nor S13 (Figure 10A, B). The effect of compound binding the Matrix Binding Motif (M-motif) on interaction of TNC with FN was determined by a cell adhesion assay on a FN/TNC substratum where half of NT193 cells with a mesenchymal phenotype (NT193M) stayed round which was in contrast to cells that spread on FN. The obtained results demonstrate that in presence of P5 and P13 a large majority of cells spread on the FN/TNC substratum, this spreading was not obtained with the control peptides S5 or S13 (Figure 10 C-E). NT193M and KRIB cells plated on FN/TNC were stained to determine the quality of spreading together with the peptides, with an anti-vinculin antibody to assess focal adhesion formation and phalloidin for detection of actin stress fibres. The obtained results demonstrate that peptide P5 and P13 induced focal adhesions and actin stress fibres on FN/TNC without affecting cell adhesion on FN nor TNC alone (Figure 10F, G). These results support that peptide P5 and P13 compete TNC binding to FN5 and FN13, thus rendering the FN13 site available for ligation by syndecan-4 to cooperate with integrin α5β1 in cell spreading on FN (Bloom et al., 1999 [1], Huang et al., 2001, Cancer Res 61, 8586-94[6]) .

As the peptides inhibited the anti-adhesive properties of TNC on NT193M or KRIB cells, the effect of compound binding the Matrix Binding Motif (M-motif) on Tenascin-C (TNC), in particular P5 and P13 on the intracellular signalling activated by TNC resulting in the expression of other matrix proteins such as FN and Col I, as described for fibrosis (Bhattacharyya et al., 2016 [57]) was studied. By treating mesangial cells (MES) with TNC and the peptides, the results show that P5 and P13 inhibit the activation of SMAD2 signalling by TNC (indicated by a decrease of SMAD2 phosphorylation, Sun et al., 2019, Matrix Bio 83, 26-47 [13]) and that P13 also decreased the expression of FN and Col I (Figure 11 A - B). By investigating FN and Col matrix assembly by IF, the results demonstrate that TNC (in presence of S13) increased matrix assembly, whereas P13 abrogated TNC-induced matrix assembly (Figures 11 C, D).

To investigate how the Matrix Binding Motif (M-motif) on Tenascin-C (TNC) would affect matrix assembly, binding of FN4-6 to FN12-14 was determined by negative EM imaging that revealed that FN4-6 bound FN12-14 in the central domains FN5 and FN13, whereas each molecule did not form homotypic interactions (Figure 1J). The Matrix Binding Motif (M-motif) on Tenascin-C (TNC) inhibited FN4-6 binding to FN12-14 as P5 and P13, respectively blocked this interaction (Figure 11E).

Accordingly, this example clearly demonstrates that compound binding the Matrix Binding Motif (M-motif) on Tenascin-C (TNC), for example peptides P5 and P13, can inhibit matrix expression and matrix assembly. Accordingly, this example clearly demonstrates that compound binding the Matrix Binding Motif (M-motif) on Tenascin-C (TNC) can be used in the treatment of disease and /or pathological conditions, for example involving matrix assembly, such as fibrosis or cancer.

### f) Compound binding the Matrix Binding Motif (M-motif) on Tenascin-C (TNC), for example peptides P5 and P13 enhance TRAIL-induced cell death

The effect of compound binding the Matrix Binding Motif (M-motif) on Tenascin-C (TNC), for example peptides P5 and P13, on TRAIL functions was studied. In this example NT193M cells that express TNC were used. Whereas these cells were refractory to TRAIL-induced killing, it was studied if TNC produced by NT193M cells could bind to TRAIL, thus lowering sensitivity towards TRAIL-induced cell death. Surprisingly, although TRAIL killed only about 5% of NT193M cells, P5 and P13 (but not S5 nor S13) significantly increased caspases 3 and 7 activation, and cell death in 15% of cells, which occurred in a TRAIL and MD5-1 concentration dependent manner (Figure 12 A-C). Moreover, P5/P13 did not induce cell death in cells with a TNC Knock Down (Figure 12 D E), supporting a role of TNC in inhibiting TRAIL-induced killing through capturing TRAIL.

Accordingly, this example clearly demonstrates that compound binding the Matrix Binding Motif (M-motif) on Tenascin-C (TNC), for example peptides P5 and P13, can activate/enhance cell death induced by Trail. Accordingly, this example clearly demonstrates that compound binding the Matrix Binding Motif (M-motif) on Tenascin-C (TNC) can be used in the treatment of disease and /or pathological condition in which abnormal cell growth is involved, for example in the treatment abnormal angiogenesis, in the treatment of abnormal angiogenesis, dysplasia, hyperplasia, tumours and cancer. P5 and P13 could also be used to potentiate TRAIL induced tumour cell killing thus promoting approaches in which TRAIL activation is used as therapeutic approach such as in cancer.

### g) Identification of sequences similar to the M-motif in other tenascin-C interacting molecules

Many molecules interact with TNC, yet for most interactions it is unknown how (Brellier and Chiquet-Ehrismann, 2012 [65]; Giblin and Midwood, 2014 [43]). To investigate the question how molecules interact with TNC. Those molecules expressed by engineered HEK293:TNC cells were determined by copurification with and adhered to TNC. In particular, during the purification of recombinant TNC, all proteins that were bound to TNC were collected and were eluted upon a high salt wash, and their identity was determined their identity by mass spectrometry. 600 molecules were found in the eluate with more than 60 molecules from the matrisome (see table 6 below) (Hynes and Naba, 2012 [7]). Apart from known interactors such as fibronectin (FN), pariostin (POSTN), aggregan and versican (Midwood and Orend, 2009 [10]), also other molecules such as Tenascin-W/TNN, laminins (LAM A4, A5, B1, B2, C1), nidogens 1 and 2, several collagens (Col 4A2, 4A6, 5A1, 6A3,12A1 and 18A1), TGFβ-Induced 1 (TGFBI), emilin2, agrin, fibulin1, Cyr61 (CCN1) and matrix remodeling enzymes such as ADAMTS1, LOXL2 and, TIMP1 and TIMP2 were identified. Moreover, cell surface associated molecules that were previously identified as TNC receptors such as Protein Tyrosine Phosphatase Receptor Type Z/B (PTPRZ1/PPTPRB) and Annexin-2 were present in the eluate (Chiquet-Ehrismann, 1995 [67]; Jones and Jones, 2000 [66]). In particular, the Annexin family of molecules was represented by several members such as ANX A2, A2P2, A4, A5 and A6.

**Table 6: Matrisomal molecules copurifying with TNC**

| **Uniprot ID** | **Entry name** | **Protein names** |
|---|---|---|
| Q61838 | A2M | Pregnancy zone protein |
| Q6GQT1 | A2M | Alpha-2-macroglobulin-P |
| Q61282 | ACAN | Aggrecan core protein |
| P97857 | ADAMTS1 | A disintegrin and metalloproteinase with thrombospondin motifs 1 |
| P28798 | GRN | Progranulin |
| P07356 | ANXA2 | Annexin A2 |
| P97429 | ANXA4 | Annexin A4 |
| P48036 | ANXA5 | Annexin A5 |
| P14824 | ANXA6 | Annexin A6 |
| P33622 | APOC3 | Apolipoprotein C-III |
| P14211 | CALR | Calreticulin |
| Q06890 | CLU | Clusterin |
| Q62507 | COCH | Cochlin |
| Q60847 | COL12A1 | Collaqen alpha-1 |
| P39061 | COL18A1 | Collaqen alpha-1 |
| P08122 | COL4A2 | Collaqen alpha-2 |
| 088207 | COL5A1 | Collaqen alpha-1 |
| P18406 | CYR61 | CCN family member 1 |
| 035474 | EDIL3 | EGF-like repeat and discoidin I-like domain-containing protein 3 |
| Q8K482 | EMILIN2 | EMILIN-2 |
| Q8BH61 | F13A1 | Coagulation factor XIII A chain |
| Q08879 | FBLN1 | Fibulin-1 |
| P11276 | FN1 | Fibronectin |
| Q9QZF2 | GPC1 | Glypican-1 |
| P51655 | GPC4 | Glypican-4 |
| P51859 | HDGF | Hepatoma-derived growth factor |
| Q9D0E1 | HNRNPM | Heterogeneous nuclear ribonucleoprotein M |
| P08113 | HSP90B1 | Endoplasmin |
| Q05793 | HSPG2 | Basement membrane-specific heparan sulfate proteoglycan core protein |
| Q9R118 | HTRA1 | Serine protease HTRA1 |
| P97927 | LAMA4 | Laminin subunit alpha-4 |
| Q61001 | LAMA5 | Laminin subunit alpha-5 |
| P02469 | LAMB1 | Laminin subunit beta-1 |
| Q61292 | LAMB2 | Laminin subunit beta-2 |
| P02468 | LAMC1 | Laminin subunit gamma-1 |
| Q07797 | LGALS3BP | Galectin-3-binding protein |
| P58022 | LOXL2 | Lysyl oxidase homolog 2 |
| 089029 | MATN4 | Matrilin-4 |
| P12025 | MDK | Midkine |
| P21956 | MFGE8 | Lactadherin |
| Q8C119 | NDNF | Protein NDNF |
| P10493 | NID1 | Nidogen-1 |
| O88322 | NID2 | Nidogen-2 |
| O09118 | NTN1 | Netrin-1 |
| Q3V1G4 | OLFML2B | Olfactomedin-like protein 2B |
| P52480 | PKM | Pyruvate kinase PKM |
| Q62009 | POSTN | Periostin |
| B9EKR1 | PTPRZ1 | Receptor-type tyrosine-protein phosphatase zeta |
| Q3UQ28 | PXDN | Peroxidasin homolog |
| P14206 | RPSA | 40S ribosomal protein SA |
| Q8C4U3 | SFRP1 | Secreted frizzled-related protein 1 |
| Q9CW03 | SMC3 | Structural maintenance of chromosomes protein 3 |
| Q8BLY1 | SMOC1 | SPARC-related modular calcium-binding protein 1 |
| Q8CD91 | SMOC2 | SPARC-related modular calcium-binding protein 2 |
| Q8VCC9 | SPON1 | Spondin-1 |
| Q9R0M3 | SRPX | Sushi-repeat-containing protein SRPX |
| P82198 | TGFBI | Transforming growth factor-beta-induced protein ig-h3 |
| P12032 | TIMP1 | Metalloproteinase inhibitor 1 |
| P39876 | TIMP3 | Metalloproteinase inhibitor 3 |
| Q80YX1 | TNC | Tenascin |
| Q80Z71 | TNN | Tenascin-N |
| Q62059 n | VCAN | Versican core protei |

By sequence comparison, sequences similar to M-motif in the other tenascin family members (TNN, TNR, TNX), some collagens, contactins, PTPR molecules, integrin α4 and other molecules were identify. In contrast to some molecules known to interact with TNC such as CALEB/CSPG5, syndecan-4, POSTN and Annexin-2 that do not have sequences similar to M-motif, other molecules listed in Figure 13 have one or several sequences similar to M-motif. FN13- comprises a similar M-motif sequence with a consensus sequences in Col12A1-FN1 with sequences for Loop I (DENT (SEQ ID NO 24)) and Loop II (ELVPETEY (SEQ ID NO 50)) and in Col12A1-FN14 with Loop I (STST (SEQ ID NO 25) and Loop II (NLQPDTSY (SEQ ID NO 51) that are separated by 43 amino acids (Figure 13). In addition, there were at least 4 additional consensus sequences similar to M-motif (FN5) present in Col12A1 with lower sequence homology (Figure 13). Next, 3D conformation of Col12A1-FN1 and observed similarities to the M-motif in FN13 where Loop I and Loop II regions formed a niche resembling the M-motif (Figure 2A-E, Figure 14). In support of a physical interconnection an overlap of TNC and Col12 expression and TNC and FN expression, respectively in OSCC tumor tissue by IF staining (Figure 15A,B) was observed and found to be reduced Col12 expression in tumors lacking TNC expression (Spenlé et al., 2020). The analysis revealed that whereas all FNIII domains have a similar organization and some sequence similarity, the sequence similar to the M-motif was only present in distinct FNIII domains. A consensus amino acid sequence for similar Loop 1 for fibronectin type (FN-type) is XpXqXrXs (SEQ ID NO 74) wherein Xp is any amino acid, Xq is D or E, Xr is T or N or S and Xs is S or T, 5 aa apart from a conserved W, and observed other Loop I like amino acid sequence such as GSRK (SEQ ID NO 33) (β4 integrin-type) and amino acid sequence AAHQ (SEQ ID NO 32) (PTPRC-type) in β4 integrin and PTPRC, respectively. Concerning similar Loop II, a consensus amino acid sequence, in particular consensus amino acid sequence XtLXuPXvXwX₁Y (SEQ ID NO 75) wherein Xt, Xu, Xv and Xw are independently any amino acid and X1 is E or D, or consensus amino acid sequence X₂LX₃PX₄X₅KY (SEQ ID NO 76) wherein X₂, X₃, X₄ and X₅ are independently any amino acid sequence (K-type) in TNX and PTPRC. As in FN and TNC, similar Loop I and Loop II are separated by a non-conserved spacer of 40 - 50 aa including more than one proline (Figure 13). Since the Loop I and Loop II sequence similarity and spacing is alike to that in FN5 and FN13, the inventors propose that TNN, TNR, TNX, Col12A1, Col14A1, CNTN1/3, PTPRB/C/D/F and integrin β4 have one or several similar M-motifs (Figure 13). In addition, a FNIII domain in the protein tyrosine phosphatase receptor family (PTPR) as family member beta/zeta (PTPRB) has been shown to bind TNC which may occur through the M-motif (Adamsky et al., 2001, Oncogene 20, 609 [64]). In PTPRB four putative similar M-motifs (Loop 2 K-type) were found. Moreover, in family members PTPRD and PTPRF one similar M-motif was present.

A similar M-motif in PTPRC (leukocyte surface molecule CD45) was identified where the spacing of 40 aa is conserved between Loop I of amino acid sequence AAHQ (SEQ ID NO 32) (Loop 1- PTPRCtype) and Loop II of amino acid sequence NLKPYTKY (SEQ ID NO 98) (Loop 2 K-type) (Figure 13). The M-motif of PTPRC/CD45 shows conformational similarity to that in FN5 and FN13 (Figure 14). The M-motif in CD45/PTPRC could explain accumulation of CD45+ leukocytes in TNC-rich stroma (thus contributing to immune suppression) of murine tumors such as Rip1Tag2 insulinoma and 4NQO-induced OSCC, and human tongue tumors (Spenlé et al., 2015 [63], Spenlé, et al., 2020 [45]).

Thus, a MAREMO/M-motif may exist in several FNIII containing molecules potentially shaping molecular interactions amongst matrix molecules and binding of cell surface receptors to matrix.

This example clearly supports that peptides comprising sequences similar to the M-Motif of Tenascin-C, for example comprising similar Loop I and Loop II sequences of the M-Motif in Tenascin-C can bind the specific Matrix Binding Motif (M-motif) on Tenascin-C (TNC)

### Discussion

TNC is a highly interactive molecule, yet the underlying mechanisms are poorly known. It is also known that TNC has an important binding site in TN5 (De Laporte et al., 2013 [3]). Here these interactions are investigated in more detail. In particular, the example demonstrates and studies the interactions of TNC with other matrix molecules and chemokines. The example and study revealed a common sequence in FNIII repeats of several TNC binding molecules that the inventors coined M-motif or MAtrix REgulating MOtif (MAREMO). The inventors demonstrate and found the M-motif in FN, TNN, Col12A1 and in TNC itself amongst other molecules. The example clearly demonstrates that the M-motif regulates binding of TNC to FN as compound binding the M-motif, for example peptides mimicking the M-motif, for example peptide P5 or P13, prevented binding of FN to TNC and relieved the anti-adhesive properties of TNC.

The examples revealed two mechanisms by which molecules can interact with TNC, either in a not yet formally proven HBS (heparin binding sequence) or in the M-motif. The results demonstrate that these interactions appear to be mutually exclusive (Figure 16A-D). Whereas several glycosylated molecules such as TGF *β*, CCL21, CXCL12 and TRAIL (that do not have an M-Motif), interacted with TNC in a heparin-dependent manner, presumably in the HBS of TN5, FN interacts with TNC through the M-motif. Compound binding the M-motif, in particular peptides that mimic the M-motif, in particular peptide P5 and P13 were developed. By using negative EM imaging and competition experiments different modes of binding were determined. The results clearly support that binding of the peptides P5 and P13 induce an allosteric conformational change in TNC that masks the HBS, thus blocking binding of the chemokines to TNC.

The M-motif in FN, TNC and other matrix molecules might be an important sequence to regulate molecular interactions. This may in particular apply to syndecan-4. The results clearly support that binding of TNC to FN13 may induce an allosteric conformational change in the HBS thereby denying access to syndecan-4 (Figure 16C,D).. Binding of TNC may induce an allosteric shift thereby masking the HBS and denying access of syndecan-4 to the HBS in FN13 (Figure 10B-D). This mechanism could explain how TNC blocks syndecan-4 function (Huang et al., 2001 [6], Orend et al., 2003 [11]). Compound binding the M-motif, for example peptides P5 and P13 may compete TNC binding to FN13, thus allowing syndecan-4 to bind to FN and, inducing cell spreading on the FN/TNC substratum (Fig. 16C, D).

The inventors had demonstrated, with the use of TNC knockout tumor models, that TNC is a regulator of an immuno-suppressive TME where TNC affects binding of chemokines, in particular CCL21, CXCL12 and TRAIL (Spenlé et al., 2020 [45]). As demonstrated, compound binding the M-motif, for example the peptide of the invention, for example peptides P5 and P13, inhibited TNC-induced expression of Ccr7 and Cd86 in dendritic cells. This inhibition is important and demonstrates that compound binding the M-motif according to the invention are useful for the treatment of inflammatory diseases, such as rheumatoid arthritis and fibrosis, and could normalize immunity in such diseases. As also demonstrated, compound binding the M-motif, for example the peptide of the invention, for example peptides P5 and P13 inhibit chemoretention by TNC/CCL21 and TNC/CXCL12.

This example clearly demonstrates that compound binding the M-motif, for example the peptide of the invention, for example peptides P5 and P13, allow the release of TNC-bound immune regulatory molecules. This example also clearly demonstrates that since the compound binding the M-motif on Tenascin-C (TNC) according to the invention allow the release TNC-bound immune regulatory molecules, the use of compound binding the M-motif on Tenascin-C (TNC) according to the invention allow to regulate the immunity and/or the immune response. This example also clearly demonstrates that the compound binding the M-motif on Tenascin-C (TNC) according to the invention allow to reduce the immuno-suppressive properties of disease, for example of the tumor microenvironment (TME).

This example also clearly demonstrate that TNC inhibits TRAIL functions in anti-tumor immunity and the compound binding the Matrix Binding Motif (M-motif) on Tenascin-C (TNC) thus enhanced cell death, compound binding Matrix Binding Motif (M-motif) on Tenascin-C (TNC) according to the invention, in particular peptide P5 and P13 have a wide range of applications in diseases where TNC and TRAIL are expressed such as cancer.

The inventors also noticed less dense collagen fibrils in tumors from TNCKO mice. In addition, expression of many matrix (and other matrisomal molecules) was reduced in these tumors. Interestingly, both FN and Col12 formed tight networks with TNC that were lost in the TNCKO tumors (Spenlé, et al., 2020 [45]). Accordingly, it is likely that also network assembly is altered in the absence of TNC. The alteration of network assembly can be illustrated by measurements of tissue stiffness through atomic force microscopy and relaxation of FN, two parameters that were specific for the tumor compared to adjacent normal tissue and changed in the absence of TNC. In support, the inventors have shown that TNC induced matrix expression and assembly in mesangial cells which was inhibited by P5 and P13, respectively. Also TGFβ signalling was reduced by P5/P13, a pathway that is linked to fibrosis. The hypothesis is that TNC is a master regulator of matrix assembly through its particular "stickiness" that is provided by the M-motif and HBS in TNC. As TNC can interact with FN through the M-motif the inventors propose that FN and other matrix molecules, such as Col12 and TNN may use their M-motifs to generate intermolecular interaction networks to form large fibrillar matrix alignments that are seen in the TMT (Spenlé et al., 2015 [15]), TACS (Tomko et al., 2018 [55]) and in other networks in fibrotic tissues (Bhattacharyya et al., 2016 [57]). Interestingly, blocking the M-motif with compound according to the invention, for example peptides P5/P13 or with TNC specific nanobodies or single-domain antibodies, can impact network formation and immune surveillance in tumors, and immunity in inflamed and fibrotic tissues. In addition, the example clearly demonstrates that occupancy of the M-motif, binding the M-Motif with compounds according to the invention, alters the structural conformation, thus abrogating the HBS and precluding binding of molecules including soluble factors, for example TGFβ, syndecan-4 and others to the same FNIII domain.)

This example also clearly provides a method to identify compound binding to TNC, in particular compounds binding the M-motif on Tenascin-C (TNC) and/or novel interactors of TNC due to the presence of a M-motif. Accordingly, this example clearly demonstrates that the compound binding the M-motif on Tenascin-C (TNC) according to the invention, for example peptides and/or nanobodies or single-domain antibodies, and/or antibodies, and/or aptamer according to the invention, could be useful on the treatment and/or for targeting fibrotic and inflamed tissues, in particular to reduce matrix network formation and/or binding of chemokines/cytokines/growth factors by TNC as well as other matrix molecules, and thus advantageously reducing the immuno-suppressive properties of the TME and/or ameliorating the pro-inflammatory conditions of inflamed and fibrotic tissues.

Advantageously, the compound binding the M-motif on Tenascin-C (TNC) according to the invention could also reduce the tissue stiffness which is an independent malignancy marker. Accordingly, this example clearly demonstrates that the compound binding the M-motif on Tenascin-C (TNC) according to the invention, for example peptides and/or nanobodies or single-domain antibodies, and/or antibodies and/or aptamer according to the invention, could be useful on the treatment and/or for targeting tumors and/or cancer.

In addition, as demonstrated in the example, the present invention is also useful to generate engineered biomaterials with chemokines that could serve as substratum in wound healing or as anti-inflammatory tools. Single domain molecules of FN and TNC such as FN5, FN13 and TN5 might be useful due to their HBS that would allow to immobilize the soluble factors and/or chemokines. It is known that wound healing is improved with soluble wound healing factors due to their capacity to bind to wound specific matrix, in particular to TNC and FN (Martino et al., 2014 [57]). By targeting the M-motif/MAREMO and providing compound binding the M-Motif on Tenascin-C (TNC) the present invention could allow to fine tune the binding and release of these molecules.

### List of References

1. Bloom, L., Ingham, K.C., and Hynes, R.O. (1999). Fibronectin regulates assembly of actin filaments and focal contacts in cultured cells via the heparin-binding site in repeat 11113. Mol. Biol. Cell 10, 1521-1536.
2. Chiquet-Ehrismann, R., Kalla, P., Pearson, C.A., Beck, K., and Chiquet, M. (1988). Tenascin interferes with fibronectin action. Cell 53, 383-390.
3. De Laporte, L., Rice, J.J., Tortelli, F., and Hubbell, J.A. (2013). Tenascin C Promiscuously Binds Growth Factors via Its Fifth Fibronectin Type III-Like Domain. PLoS One 8.
4. Deligne, C., Murdamoothoo, D., Gammage, A.N., Gschwandtner, M., Erne, W., Loustau, T., Marzeda, A.M., Carapito, R., Paul, N., Velázquez-Quesada, I., et al. (2020). Matrix-targeting immunotherapy controls tumor growth and spread by switching macrophage phenotype. Cancer Immunol Res.
5. Huang, J.-Y., Cheng, Y.-J., Lin, Y.-P., Lin, H.-C., Su, C.-C., Juliano, R., and Yang, B.-C. (2010). Extracellular Matrix of Glioblastoma Inhibits Polarization and Transmigration of T Cells: The Role of Tenascin-C in Immune Suppression. The Journal of Immunology 185, 1450-1459.
6. Huang, W., Chiquet-Ehrismann, R., Moyano, J.V., Garcia-Pardo, A., and Orend, G. (2001). Interference of tenascin-C with syndecan-4 binding to fibronectin blocks cell adhesion and stimulates tumor cell proliferation. Cancer Res. 61, 8586-8594.
7. Hynes, R.O. (2009). The extracellular matrix: not just pretty fibrils. Science 326, 1216-1219.
8. Ingham, K.C., Brew, S.A., and Erickson, H.P. (2004). Localization of a cryptic binding site for tenascin on fibronectin. J. Biol. Chem. 279, 28132-28135.
9. Jachetti, E., Caputo, S., Mazzoleni, S., Brambillasca, C.S., Parigi, S.M., Grioni, M., Piras, I.S., Restuccia, U., Calcinotto, A., Freschi, M., et al. (2015). Tenascin-C Protects Cancer Stem-like Cells from Immune Surveillance by Arresting T-cell Activation. Cancer Res. 75, 2095-2108.
10. Midwood, K.S., Chiquet, M., Tucker, R.P., and Orend, G. (2016). Tenascin-C at a glance. Journal of Cell Science 129, 4321-4327.
11. Orend, G., Huang, W., Olayioye, M.A., Hynes, N.E., and Chiquet-Ehrismann, R. (2003). Tenascin-C blocks cell-cycle progression of anchorage-dependent fibroblasts on fibronectin through inhibition of syndecan-4. Oncogene 22, 3917-3926.
12. Sharma, A., Askari, J.A., Humphries, M.J., Jones, E.Y., and Stuart, D.I. (1999). Crystal structure of a heparin- and integrin-binding segment of human fibronectin. EMBO J. 18, 1468-1479.
13. Sun, Z., Velázquez-Quesada, I., Murdamoothoo, D., Ahowesso, C., Yilmaz, A., Spenlé, C., Averous, G., Erne, W., Oberndorfer, F., Oszwald, A., et al. (2019). Tenascin-C increases lung metastasis by impacting blood vessel invasions. Matrix Biol 83, 26-47.
14. Van Obberghen-Schilling, E., Tucker, R.P., Saupe, F., Gasser, I., Cseh, B., and Orend, G. (2011). Fibronectin and tenascin-C: accomplices in vascular morphogenesis during development and tumor growth. Int. J. Dev. Biol. 55, 511-525.
15. Spenlé, C., Gasser, I., Saupe, F., Janssen, K.-P., Arnold, C., Klein, A., van der Heyden, M.,Mutterer, J., Neuville-Méchine, A., Chenard, M.-P., et al. (2015). Spatial organization of the tenascin-C microenvironment in experimental and human cancer. Cell Adhesion & Migration 9, 4-13.
16. Nies DE, Hemesath TJ, Kim JH, Gulcher JR, Stefansson K (March 1991). "The complete cDNA sequence of human hexabrachion (Tenascin). A multidomain protein containing unique epidermal growth factor repeats". J Biol Chem. 266 (5): 2818-23. PMID 1704365,
17. Siri A, Carnemolla B, Saginati M, Leprini A, Casari G, Baralle F, Zardi L (May 1991). "Human tenascin: primary structure, pre-mRNA splicing patterns and localization of the epitopes recognized by two monoclonal antibodies". Nucleic Acids Res. 19 (3): 525-31. doi:10.1093/nar/19.3.525. PMC 333643. PMID 1707164
18. Borna Mehrad et al. "Chemokines as mediators of angiogenesis" Thromb Haemost. 2007 May; 97(5): 755-762 [18].
19. Harlow et al., Antibodies: A Laboratory Manual, (Harlow et al.: "Antibodies: A Laboratory Manual", Cold Spring HarborLaboratory Press, 2nd ed. 1988.
20. Hammerling, et al.: "Monoclonal Antibodies and T-Cell Hybridomas", Elsevier, N. Y., 1981, pp. 563-681.
21. Kabat "Sequences of Proteins of Immunological Interest" National Institutes of Health, Bethesda, Md., 1987 and 1991;
22. Chothia, et al, J. Mol. Bio. (1987) 196:901-917;
23. Chothia, et al., Nature (1989) 342:878-883
24. http://www.promega.com/vectors/mammalian_express_vectors.htm [10] or http://www.qiagen.com/pendantview/qiagenes.aspx?gaw=PROTQIAgenes0807& gkw=mammalian+expression,
25. http://www.scbt.com/chap
   _exp_vectors.php?type=pCruzTM%20Expression%20Vectors.
26. WO 83/004261
27. Naso et al 2017, "Adeno-Associated Virus (AAV) as a Vector for Gene Therapy", BioDrugs. 2017 Aug;31 (4):317-334. doi: 10.1007/s40259-017-0234-5
28. Zincarelli 2008 "Analysis of AAV Serotypes 1-9 Mediated Gene Expression and Tropism in Mice After Systemic Injection" Mol Ther. 2008 Jun; 16(6):1073-80. doi: 10.1038/mt.2008.76. Epub 2008 Apr 15
29. Bober, M., Enochsson, C., Collin, M., and Mörgelin, M. (2010). Collagen VI is a subepithelial adhesive target for human respiratory tract pathogens. J Innate Immun 2, 160-166
30. Baschong, W., and Wrigley, N.G. (1990). Small colloidal gold conjugated to fab fragments or to immunoglobulin g as high-resolution labels for electron microscopy: A technical overview. J. Elec. Microsc. Tech. 14, 313-323
31. Madeira, F., Park, Y. mi, Lee, J., Buso, N., Gur, T., Madhusoodanan, N., Basutkar, P., Tivey, A.R.N., Potter, S.C., Finn, R.D., et al. (2019). The EMBL-EBI search and sequence analysis tools APIs in 2019. Nucleic Acids Res 47, W636-W641
32. Lamiable, A., Thévenet, P., Rey, J., Vavrusa, M., Derreumaux, P., and Tufféry, P. (2016). PEP-FOLD3: faster de novo structure prediction for linear peptides in solution and in complex. Nucleic Acids Res 44, W449-W454
33. Pettersen, E.F., Goddard, T.D., Huang, C.C., Couch, G.S., Greenblatt, D.M., Meng, E.C., and Ferrin, T.E. (2004). UCSF Chimera-A visualization system for exploratory research and analysis. Journal of Computational Chemistry 25, 1605-1612
34. Hacer Sahin Hermann E.Wasmuth Chemokines in tissue fibrosis, Biochimica et Biophysica Acta (BBA) - Molecular Basis of Disease Volume 1832, Issue 7, July 2013, Pages 1041-1048.
35. Caroline L. Sokol and Andrew D. Luster Cold Spring, The Chemokine System in Innate Immunity, Harb Perspect Biol. 2015 May; 7(5): a016303.
36. Caroline L. Sokol and Andrew D. Luster Cold Spring, The Chemokine System in Innate Immunity, Harb Perspect Biol. 2015 May; 7(5): a016303.
37. Hauzenberger et al., Tenascin-C inhibits beta1 integrin-dependent T lymphocyte adhesion to fibronectin through the binding of its fnlll 1-5 repeats to fibronectin. Eur J Immunol. 1999 May;29 (5):1435-47. doi: 10.1002/(SICI)1521-4141(199905)29:05<1435::AID-MMU1435>3.0.CO;2-N.
38. Spenle, C., Loustau, T., Murdamoothoo, D., Erne, W., Beghelli-de la Forest Divonne, S., Veber, R., Petti, L., Bourdely, P., Mörgelin, M., Brauchle, E.-M., et al. (2020). Tenascin-C Orchestrates an Immune-Suppressive Tumor Microenvironment in Oral Squamous Cell Carcinoma. Cancer Immunol Res 8, 1122-1138.
39. Turley et al., « Immunological hallmarks of stromal cells in the tumour microenvironment" 2015, Nat Rev Immunol 15, 669-82.
40. Arpel, A., Sawma, P., Spenlé, C., Fritz, J., Meyer, L., Garnier, N., Velázquez-Quesada, I., Hussenet, T., Aci-Sèche, S., Baumlin, N., et al. (2014). Transmembrane Domain Targeting Peptide Antagonizing ErbB2/Neu Inhibits Breast Tumor Growth and Metastasis. Cell Reports 8, 1714-1721
41. Shen, Z., Reznikoff, G., Dranoff, G., and Rock, K.L. (1997). Cloned dendritic cells can present exogenous antigens on both MHC class I and class II molecules. The Journal of Immunology 158, 2723-2730
42. Sarrab, R.M., Lennon, R., Ni, L., Wherlock, M., Welsh, G., and Saleem, M. (2011). Establishment of conditionally immortalized human glomerular mesangial cells in culture, with unique migratory properties. American Journal of Physiology. Renal Physiology..
43. Giblin, S.P., and Midwood, K.S. (2014). Tenascin-C: Form versus function. Cell Adh Migr 9,48-82
44. Huang, W., Chiquet-Ehrismann, R., Moyano, J.V., Garcia-Pardo, A., and Orend, G. (2001). Interference of tenascin-C with syndecan-4 binding to fibronectin blocks cell adhesion and stimulates tumor cell proliferation. Cancer Res. 61, 8586-8594
45. Spenle, C., Loustau, T., Murdamoothoo, D., Erne, W., Beghelli-de la Forest Divonne, S., Veber, R., Petti, L., Bourdely, P., Mörgelin, M., Brauchle, E.-M., et al. (2020). Tenascin-C Orchestrates an Immune-Suppressive Tumor Microenvironment in Oral Squamous Cell Carcinoma. Cancer Immunol Res 8, 1122-1138.Moyano et al., 1997
46. Bloom, L., Ingham, K.C., and Hynes, R.O. (1999). Fibronectin Regulates Assembly of Actin Filaments and Focal Contacts in Cultured Cells via the Heparin-binding Site in Repeat 11113. Mol Biol Cell 10, 1521-1536.)
47. Lamiable, A., Thévenet, P., Rey, J., Vavrusa, M., Derreumaux, P., and Tufféry, P. (2016). PEP-FOLD3: faster de novo structure prediction for linear peptides in solution and in complex. Nucleic Acids Res 44, W449-W454
48. Matthew Ward, R., Venner, E., Daines, B., Murray, S., Erdin, S., Kristensen, D.M., and Lichtarge, O. (2009). Evolutionary Trace Annotation Server: automated enzyme function prediction in protein structures using 3D templates. Bioinformatics 25, 1426-1427
49. Matthew Ward, R., Venner, E., Daines, B., Murray, S., Erdin, S., Kristensen, D.M., and Lichtarge, O. (2009). Evolutionary Trace Annotation Server: automated enzyme function prediction in protein structures using 3D templates. Bioinformatics 25, 1426-1427
50. Lamiable, A., Thévenet, P., Rey, J., Vavrusa, M., Derreumaux, P., and Tufféry, P. (2016).PEP-FOLD3: faster de novo structure prediction for linear peptides in solution and in complex. Nucleic Acids Res 44, W449-W454
51. Pettersen, E.F., Goddard, T.D., Huang, C.C., Couch, G.S., Greenblatt, D.M., Meng, E.C., and Ferrin, T.E. (2004). UCSF Chimera-A visualization system for exploratory research and analysis. Journal of Computational Chemistry 25, 1605-1612
52. Vangone, A., Oliva, R., and Cavallo, L. (2012). CONS-COCOMAPS: a novel tool to measure and visualize the conservation of inter-residue contacts in multiple docking solutions. BMC Bioinformatics 13, S19
53. Ksouri, A., Ghedira, K., Ben Abderrazek, R., Shankar, B.A.G., Benkahla, A., Bishop, O.T., and Bouhaouala-Zahar, B. (2018). Homology modeling and docking of Aahll-Nanobody complexes reveal the epitope binding site on Aahll scorpion toxin. Biochemical and Biophysical Research Communications 496, 1025-1032
54. Rappsilber, J., Mann, M., and Ishihama, Y. (2007). Protocol for micro-purification, enrichment, pre-fractionation and storage of peptides for proteomics using StageTips. Nat Protoc 2, 1896-1906.Jimenez-Morales et al., 2020)
55. Tomko, L.A., Hill, R.C., Barrett, A., Szulczewski, J.M., Conklin, M.W., Eliceiri, K.W., Keely, P.J., Hansen, K.C., and Ponik, S.M. (2018). Targeted matrisome analysis identifies thrombospondin-2 and tenascin-C in aligned collagen stroma from invasive breast carcinoma. Sci Rep 8, 12941
56. Martino, M.M., Briquez, P.S., Güç, E., Tortelli, F., Kilarski, W.W., Metzger, S., Rice, J.J., Kuhn, G.A., Müller, R., Swartz, M.A., et al. (2014). Growth factors engineered for superaffinity to the extracellular matrix enhance tissue healing. Science 343, 885-888.
57. Bhattacharyya, S., Wang, W., Morales-Nebreda, L., Feng, G., Wu, M., Zhou, X., Lafyatis, R., Lee, J., Hinchcliff, M., Feghali-Bostwick, C., et al. (2016). Tenascin-C drives persistence of organ fibrosis. Nature Communications 7, 11703.
58. Lichtarge et al.,1996
59. Am J Physiol Renal Physiol. 2011 Nov; 301(5): F1131-F1138
60. Alexandre Detappe, Clélia Mathieu, Caining Jin, Michael P Agius, Marie-Charlotte Diringer , Vu-Long Tran , Xavier Pivot, Francois Lux, Olivier Tillement, Donald Kufe, Peter P Ghoroghchian, Anti-MUC1-C Antibody-Conjugated Nanoparticles Potentiate the Efficacy of Fractionated Radiation Therapy Int J Radiat Oncol Biol Phys 2020 Dec 1;108(5):1380-1389. doi: 10.1016/j.ijrobp.2020.06.069. Epub 2020 Jul 4.
61. Rachel J. Fields a, Christopher J. Cheng a, Elias Quijano a, Caroline Weller a, Nina Kristofik a, Nha Duong a, Christopher Hoimes a, Marie E. Egan b, W. Mark Saltzman, Surface modified poly(β amino ester)-containing nanoparticles for plasmid DNA delivery. Journal of Controlled Release 164 (2012) 41-48
62. Gamper C, Spenlé C, Boscá S, van der Heyden M, Erhardt M, Orend G, Bagnard D*, Heinlein M*, 2019, Functionalized Tobacco Mosaic Virus Coat Protein Monomers and Oligomers as Nanocarriers for Anti-Cancer Peptides, Cancers 11, 1609.
63. Spenlé C, Gasser I, Saupe F, Janssen KP, Arnold C, Klein A, van der Heyden M, Mutterer J, Neuville-Méchine A, Chenard MP, Guenot D, Esposito I, Slotta-Huspenina J, Amburtsumian N, Simon-Assmann P, Orend G. 2015. Spatial organization of the tenascin-C microenvironment in experimental and human cancer, Cell Adh Migr, 9, 4 - 13.
64. Adamsky, K., Schilling, J., Garwood, J., Faissner, A., and Peles, E. (2001). Glial tumor cell adhesion is mediated by binding of the FNIII domain of receptor protein tyrosine phosphatase β (RPTPβ) to tenascin C. Oncogene 20, 609-618. Brellier and Chiquet-Ehrismann, 2012
65. Jones and Jones, 2000 Jones, F.S., and Jones, P.L. (2000). The tenascin family of ECM glycoproteins: Structure, function, and regulation during embryonic development and tissue remodeling. Developmental Dynamics 218, 235-259.
66. Chiquet-Ehrismann, 1995Chiquet-Ehrismann, R. (1995). Inhibition of cell adhesion by anti-adhesive molecules. Current Opinion in Cell Biology 7, 715-719.

## Claims

1. Compound binding Matrix Binding Motif (M-motif) on Tenascin-C (TNC) for use in the prevention and/or treatment of disease.

2. Compound binding Matrix Binding Motif (M-motif) on Tenascin-C (TNC) for use in the treatment of disease, in particular by regulating the immune response and/or tissue homeostasis.

3. Compound binding Matrix Binding Motif (M-motif) on Tenascin-C (TNC) for use in a treatment of immune response disorder and/or tissue homeostasis disorders.

4. Use according to claim 1 or 2 wherein the amino acid sequence of binding Matrix Binding Motif (M-motif) on Tenascin-C (TNC) is selected in the group comprising

TDTTALITWFKPLAEIDGIELTYGIKDVPGDRTTIDLTEDENQYSIGNLK
PDTEY (SEQ ID NO 1),

TDNSITLEWRNGKAAIDSYRIKYAPISGGDHAEVDVPKSQQATTKTTL
TGLRPGTEY (SEQ ID NO 2) and

AETSLTLLWKTPLAKFDRYRLNYSLPTGQWVGVQLPRNTTSYVLRG
LEPGQEY (SEQ ID NO 3)

5. Use according to any preceding claim wherein the compound is selected from the group comprising peptides, antibodies, nanobodies or single-domain antibodies, aptamers, peptidomimetics

6. Use according to any one preceding claim wherein the compound is a peptide of general formula A₁-B₁-C₁ (I) wherein
A₁ is an amino acids chain of sequence TXaXbT (SEQ ID NO 4) or STXaXbT (SEQ ID NO 5) or XgXjXkXh (SEQ ID NO 6) or SXgXjXkXh (SEQ ID NO 7),
B₁ is a linker having an amino acid sequence comprising 1 to 50 amino acid,
C₁ is an amino acids chain of sequence XcLQPXdXeXfYS (SEQ ID NO 8), XcLQPXdXeXfY (SEQ ID NO 9), XcLXiPXmXnXoY (SEQ ID NO 10) or XcLXiPXmXnXoYS (SEQ ID NO 11)
Xa is D or E or T or A,
Xb is S or T or N,
Xc is N or G or E or D,
Xd is G or A,
Xe is S or T or Q or V or M,
Xf is E or D,
Xg is T or A or D or S or R or G,
Xh is T or S or Q or K,
Xi is Q or E or R or H or V or L or P,
Xj is D or E or T or A or L or S or N or P,
Xk is S or T or N or D or Y or H or R,
Xm is G or A or E or D or N or W or Y or F,
Xn is S or T or Q or V or E or M or R or K or C
Xo is E or D or S or L or K

7. Use according to claim 5 wherein A₁ is of sequence selected from the group comprising TDST (SEQ ID NO 14), STDST (SEQ ID NO 15), TETT (SEQ ID NO 16) or STETT (SEQ ID NO 17), TETS (SEQ ID NO 18), AETS (SEQ ID NO 19), TDNS (SEQ ID NO 20), TENS (SEQ ID NO 21), TENT (SEQ ID NO 22), TATS (SEQ ID NO 23), DENT (SEQ ID NO 24), STST (SEQ ID NO 25), TTDS (SEQ ID NO 26), RATS (SEQ ID NO 27), and TDTT (SEQ ID NO 28), SLTS (SEQ ID NO 29), SSYS (SEQ ID NO 30), ANTS (SEQ ID NO 31), AAHQ (SEQ ID NO 32), GSRK (SEQ ID NO 33).
B is of sequence SAPAS (SEQ ID NO 10)
C is of sequence selected from the group comprising NLQPASEY (SEQ ID NO 40), NLQPASEYS (SEQ ID NO 41), GLQPGTDY (SEQ ID NO 42), GLQPGTDYS (SEQ ID NO 43), GLEPGQEY (SEQ ID NO 44), GLRPGTEY (SEQ ID NO 45), GLHPGTEY (SEQ ID NO 46), GLRPGVEY (SEQ ID NO 47), GLKPGEEY (SEQ ID NO 48), DLVPGTEY (SEQ ID NO 49), ELVPETEY (SEQ ID NO 50), NLQPDTSY (SEQ ID NO 51), GLLPNTEY (SEQ ID NO 52), GLEPGTEY (SEQ ID NO 53), DLIPWMEY (SEQ ID NO 54), ELNPWVEY (SEQ ID NO 55), ELVPGRLY (SEQ ID NO 56), DLTPGKKY (SEQ ID NO 57) GLVPGRKY (SEQ ID NO 58) and NLKPYTKY (SEQ ID NO 77).

8. Use according to claim 5 wherein the peptide is of sequence selected from the group comprising TDSTSAPASNLQPASEY (SEQ ID NO 59) STDSTSAPASNLQPASEYS (SEQ ID NO 60), TETTSAPASGLQPGTDY (SEQ ID NO 61) and STETTSAPASGLQPGTDYS (SEQ ID NO 62)

9. Use according to any one of the preceding claim wherein the disease involves at least one chemokine or heparin binding soluble factor, preferably at least one chemokine or heparin binding soluble factor involved in inflammation, angiogenesis and/or fibrosis.

10. Use according to any one of the preceding claim wherein the disease involves at least one chemokine selected from the group comprising CXCL12, TRAIL, CCL21 and TGFβ.

11. Use according to any one of the preceding claim wherein the disease is selected from the group comprising cancer, infections, tumor, inflammatory disease, angiogenesis, fibrosis disease, immunological disorders, disease involving fibronectin.

12. Use according to any one of the preceding claim wherein the compound in combination with anticancer drug, anti-inflammatory drug, anti-fibrotic drug, anti-angiogenic drug, anti-hypertensive drug, anti-oxidative drug.


SEQUENCE LISTING

<110>  Université de Strasbourg
       INSERM- Institut National de la Santé et de la Recherche
       Médicale

<120>  Compounds binding tenascin-C (TNC) for use in the treatment of
       diseases

<130>  BNT229474EP01

<150>  EP20315253.3
<151>  2020-05-20

<160>  112

<170>  PatentIn version 3.5

<210>  1
<211>  55
<212>  PRT
<213>  Artificial Sequence

<220>
<223>  Matrix Binding Motif (M-motif)

<400>  1

Thr Asp Thr Thr Ala Leu Ile Thr Trp Phe Lys Pro Leu Ala Glu Ile
1               5                   10                  15

Asp Gly Ile Glu Leu Thr Tyr Gly Ile Lys Asp Val Pro Gly Asp Arg
            20                  25                  30

Thr Thr Ile Asp Leu Thr Glu Asp Glu Asn Gln Tyr Ser Ile Gly Asn
        35                  40                  45

Leu Lys Pro Asp Thr Glu Tyr
    50                  55

<210>  2
<211>  57
<212>  PRT
<213>  Artificial Sequence

<220>
<223>  Matrix Binding Motif (M-motif)

<400>  2

Thr Asp Asn Ser Ile Thr Leu Glu Trp Arg Asn Gly Lys Ala Ala Ile
1               5                   10                  15

Asp Ser Tyr Arg Ile Lys Tyr Ala Pro Ile Ser Gly Gly Asp His Ala
            20                  25                  30

Glu Val Asp Val Pro Lys Ser Gln Gln Ala Thr Thr Lys Thr Thr Leu

35                  40                  45

Thr Gly Leu Arg Pro Gly Thr Glu Tyr
    50                  55

<210>  3
<211>  53
<212>  PRT
<213>  Artificial Sequence

<220>
<223>  Matrix Binding Motif (M-motif)

<400>  3

Ala Glu Thr Ser Leu Thr Leu Leu Trp Lys Thr Pro Leu Ala Lys Phe
1               5                   10                  15

Asp Arg Tyr Arg Leu Asn Tyr Ser Leu Pro Thr Gly Gln Trp Val Gly
            20                  25                  30

Val Gln Leu Pro Arg Asn Thr Thr Ser Tyr Val Leu Arg Gly Leu Glu
        35                  40                  45

Pro Gly Gln Glu Tyr
    50

<210>  4
<211>  4
<212>  PRT
<213>  Artificial Sequence

<220>
<223>  A1 fragment of peptide of general formula A1-B1-C1 (I)

<220>
<221>  MISC_FEATURE
<222>  (2)..(2)
<223>  Xaa can be D or E or T or A

<220>
<221>  MISC_FEATURE
<222>  (3)..(3)
<223>  Xaa can be S or T or N,

<400>  4

Thr Xaa Xaa Thr
1

<210>  5
<211>  5
<212>  PRT
<213>  Artificial Sequence

<220>
<223>  A1 fragment of peptide of general formula A1-B1-C1


<220>
<221>  MISC_FEATURE
<222>  (3)..(3)
<223>  Xaa can be D or E or T or A

<220>
<221>  MISC_FEATURE
<222>  (4)..(4)
<223>  Xaa can be S or T or N

<400>  5

Ser Thr Xaa Xaa Thr
1               5


<210>  6
<211>  4
<212>  PRT
<213>  Artificial Sequence

<220>
<223>  A fragment of peptide of general formula A-B-C (I)


<220>
<221>  MISC_FEATURE
<222>  (1)..(1)
<223>  Xaa can be T or A or D or S or R or G

<220>
<221>  MISC_FEATURE
<222>  (2)..(2)
<223>  Xaa can be D or E or T or A or L or S or N or P

<220>
<221>  MISC_FEATURE
<222>  (3)..(3)
<223>  Xaa can be S or T or N or D or Y or H or R

<220>
<221>  MISC_FEATURE
<222>  (4)..(4)
<223>  Xaa can be T or S or Q or K

<400>  6

Xaa Xaa Xaa Xaa
1


<210>  7
<211>  5
<212>  PRT
<213>  Artificial Sequence

<220>
<223>  A1 fragment of peptide of general formula A1-B1-C1 (I)

<220>
<221>  MISC_FEATURE
<222>  (2)..(2)
<223>  Xaa can be T or A or D or S or R or G

<220>
<221>  MISC_FEATURE
<222>  (3)..(3)
<223>  Xaa can be D or E or T or A or L or S or N or P

<220>
<221>  MISC_FEATURE
<222>  (4)..(4)
<223>  Xaa can be S or T or N or D or Y or H or R

<220>
<221>  MISC_FEATURE
<222>  (5)..(5)
<223>  Xaa can be T or S or Q or K

<400>  7

Ser Xaa Xaa Xaa Xaa
1               5


<210>  8
<211>  9
<212>  PRT
<213>  Artificial Sequence

<220>
<223>  C1 fragment of peptide of general formula A1-B1-C1 (I)


<220>
<221>  MISC_FEATURE
<222>  (1)..(1)
<223>  Xaa can be N or G or E or D

<220>
<221>  MISC_FEATURE
<222>  (5)..(5)
<223>  Xaa can be G or A

<220>
<221>  MISC_FEATURE
<222>  (6)..(6)
<223>  Xaa can be S or T or Q or V or M

<220>
<221>  MISC_FEATURE
<222>  (7)..(7)
<223>  Xaa can be E or D

<400>  8

Xaa Leu Gln Pro Xaa Xaa Xaa Tyr Ser
1               5


<210>  9

<211>  8
<212>  PRT
<213>  Artificial Sequence

<220>
<223>  C1 fragment of peptide of general formula A1-B1-C1 (I)


<220>
<221>  MISC_FEATURE
<222>  (1)..(1)
<223>  Xaa can be N or G or E or D

<220>
<221>  MISC_FEATURE
<222>  (5)..(5)
<223>  Xaa can be G or A

<220>
<221>  MISC_FEATURE
<222>  (6)..(6)
<223>  Xaa can be S or T or Q or V or M

<220>
<221>  MISC_FEATURE
<222>  (7)..(7)
<223>  Xaa can be E or D

<400>  9

Xaa Leu Gln Pro Xaa Xaa Xaa Tyr
1               5


<210>  10
<211>  8
<212>  PRT
<213>  Artificial Sequence

<220>
<223>  C1 fragment of peptide of general formula A1-B1-C1 (I)


<220>
<221>  MISC_FEATURE
<222>  (1)..(1)
<223>  Xaa can be N or G or E or D

<220>
<221>  MISC_FEATURE
<222>  (3)..(3)
<223>  Xaa can be Q or E or R or H or V or L or P

<220>
<221>  MISC_FEATURE
<222>  (5)..(5)
<223>  Xaa can be G or A or E or D or N or W or Y or F

<220>
<221>  MISC_FEATURE
<222>  (6)..(6)
<223>  Xaa can be G or A or E or D or N or W or Y or F

<220>
<221>  MISC_FEATURE
<222>  (7)..(7)
<223>  Xaa can be E or D or S or L or K

<400>  10

Xaa Leu Xaa Pro Xaa Xaa Xaa Tyr
1               5


<210>  11
<211>  9
<212>  PRT
<213>  Artificial Sequence

<220>
<223>  C1 fragment of peptide of general formula A1-B1-C1 (I)


<220>
<221>  MISC_FEATURE
<222>  (1)..(1)
<223>  Xaa can be N or G or E or D

<220>
<221>  MISC_FEATURE
<222>  (3)..(3)
<223>  Xaa can be Q or E or R or H or V or L or P

<220>
<221>  MISC_FEATURE
<222>  (5)..(5)
<223>  Xaa can be G or A or E or D or N or W or Y or F

<220>
<221>  MISC_FEATURE
<222>  (6)..(6)
<223>  Xaa can be S or T or Q or V or E or M or R or K or C

<220>
<221>  MISC_FEATURE
<222>  (7)..(7)
<223>  Xaa can be E or D or S or L or K

<400>  11

Xaa Leu Xaa Pro Xaa Xaa Xaa Tyr Ser
1               5


<210>  12
<211>  4
<212>  PRT
<213>  Artificial Sequence

<220>
<223>  fragment A1 of peptide of general formula A1-B1-C1 (I)


<220>
<221>  MISC_FEATURE
<222>  (1)..(1)

<223>  Xaa can be T or A or D or S or R

<220>
<221>  MISC_FEATURE
<222>  (2)..(2)
<223>  Xaa can be D or E or T or A

<220>
<221>  MISC_FEATURE
<222>  (3)..(3)
<223>  Xaa can be S or T or N

<220>
<221>  MISC_FEATURE
<222>  (4)..(4)
<223>  Xaa can be T or S

<400>  12

Xaa Xaa Xaa Xaa
1


<210>  13
<211>  5
<212>  PRT
<213>  Artificial Sequence

<220>
<223>  fragment A1 of peptide of general formula A1-B1-C1 (I)


<220>
<221>  MISC_FEATURE
<222>  (2)..(2)
<223>  Xaa can be T or A or D or S or R

<220>
<221>  MISC_FEATURE
<222>  (3)..(3)
<223>  Xaa can be D or E or T or A

<220>
<221>  MISC_FEATURE
<222>  (4)..(4)
<223>  Xaa can be S or T or N

<220>
<221>  MISC_FEATURE
<222>  (5)..(5)
<223>  Xaa can be T or S

<400>  13

Ser Xaa Xaa Xaa Xaa
1               5


<210>  14
<211>  4
<212>  PRT
<213>  Artificial Sequence

<220>
<223>  fragment A1 of peptide of general formula A1-B1-C1 (I)

<400>  14

Thr Asp Ser Thr
1


<210>  15
<211>  5
<212>  PRT
<213>  Artificial Sequence

<220>
<223>  Fragment A1 of peptide of general formula A1-B1-C1

<400>  15

Ser Thr Asp Ser Thr
1               5


<210>  16
<211>  4
<212>  PRT
<213>  Artificial Sequence

<220>
<223>  fragment A1 of peptide of general formula A1-B1-C1

<400>  16

Thr Glu Thr Thr
1


<210>  17
<211>  5
<212>  PRT
<213>  Artificial Sequence

<220>
<223>  fragment A1 of peptide of general formula A1-B1-C1

<400>  17

Ser Thr Glu Thr Thr
1               5


<210>  18
<211>  4
<212>  PRT
<213>  Artificial Sequence

<220>
<223>  fragment A1 of peptide of general formula A1-B1-C1 (I)

<400>  18

Thr Glu Thr Ser
1

<210>  19
<211>  4
<212>  PRT
<213>  Artificial Sequence

<220>
<223>  fragment A1 of peptide of general formula A1-B1-C1 (I)

<400>  19

Ala Glu Thr Ser
1


<210>  20
<211>  4
<212>  PRT
<213>  Artificial Sequence

<220>
<223>  fragment A1 of peptide of general formula A1-B1-C1 (I)

<400>  20

Thr Asp Asn Ser
1


<210>  21
<211>  4
<212>  PRT
<213>  Artificial Sequence

<220>
<223>  fragment A1 of peptide of general formula A1-B1-C1 (I)

<400>  21

Thr Glu Asn Ser
1


<210>  22
<211>  4
<212>  PRT
<213>  Artificial Sequence

<220>
<223>  Fragment A1 of peptide of general formula A1-B1-C1 (I)

<400>  22

Thr Glu Asn Thr
1


<210>  23
<211>  4
<212>  PRT
<213>  Artificial Sequence

<220>
<223>  Fragment A1 of peptide of general formula A1-B1-C1 (I)

<400>  23

Thr Ala Thr Ser
1



<210>  24
<211>  4
<212>  PRT
<213>  Artificial Sequence

<220>
<223>  Fragment A1 of peptide of general formula A1-B1-C1 (I)

<400>  24

Asp Glu Asn Thr
1



<210>  25
<211>  4
<212>  PRT
<213>  Artificial Sequence

<220>
<223>  fragment A1 of peptide of general formula A1-B1-C1 (I)

<400>  25

Ser Thr Ser Thr
1



<210>  26
<211>  4
<212>  PRT
<213>  Artificial Sequence

<220>
<223>  fragment A1 of peptide of general formula A1-B1-C1 (I)

<400>  26

Thr Thr Asp Ser
1



<210>  27
<211>  4
<212>  PRT
<213>  Artificial Sequence

<220>
<223>  Fragment A of peptide of general formula A-B-C (I)

<400>  27

Arg Ala Thr Ser
1

<210>  28
<211>  4
<212>  PRT
<213>  Artificial Sequence

<220>
<223>  fragment A1 of peptide of general formula A1-B1-C1 (I)

<400>  28

Thr Asp Thr Thr
1


<210>  29
<211>  4
<212>  PRT
<213>  Artificial Sequence

<220>
<223>  fragment A1 of peptide of general formula A1-B1-C1 (I)

<400>  29

Ser Leu Thr Ser
1


<210>  30
<211>  4
<212>  PRT
<213>  Artificial Sequence

<220>
<223>  fragment A1 of peptide of general formula A1-B1-C1 (I)

<400>  30

Ser Ser Tyr Ser
1


<210>  31
<211>  4
<212>  PRT
<213>  Artificial Sequence

<220>
<223>  fragment A1 of peptide of general formula A1-B1-C1 (I)

<400>  31

Ala Asn Thr Ser
1


<210>  32
<211>  4
<212>  PRT
<213>  Artificial Sequence

<220>
<223>  Fragment A1 of peptide of general formula A1-B1-C1 (I)

<400>  32

Ala Ala His Gln
1

<210>  33
<211>  4
<212>  PRT
<213>  Artificial Sequence

<220>
<223>  fragment A1 of peptide of general formula A1-B1-C1 (I)

<400>  33

Gly Ser Arg Lys
1

<210>  34
<211>  42
<212>  PRT
<213>  Artificial Sequence

<220>
<223>  fragment B of peptide of general formula A-B-C (I)

<400>  34

Val Leu Val Arg Trp Thr Pro Pro Arg Ala Gln Ile Thr Gly Tyr Arg
1               5                   10                  15

Leu Thr Val Gly Leu Thr Arg Arg Gly Gln Pro Arg Gln Tyr Asn Val
            20                  25                  30

Gly Pro Ser Val Ser Lys Tyr Pro Leu Arg
        35                  40

<210>  35
<211>  42
<212>  PRT
<213>  Artificial Sequence

<220>
<223>  Fragment B of peptide of general formula A-B-C (I)

<400>  35

Ile Thr Ile Ser Trp Arg Thr Lys Thr Glu Thr Ile Thr Gly Phe Gln
1               5                   10                  15

Val Asp Ala Val Pro Ala Asn Gly Gln Thr Pro Ile Gln Arg Thr Ile
            20                  25                  30

Lys Pro Asp Val Arg Ser Tyr Thr Ile Thr
        35                  40

<210>  36
<211>  43
<212>  PRT
<213>  Artificial Sequence

<220>
<223>  fragment B1 of peptide of general formula A1-B1-C1 (I)

<400>  36

Ile Thr Ile Thr Trp Arg Thr Lys Thr Glu Thr Ile Thr Gly Phe Gln
1               5                   10                  15

Ile Asp Ala Ile Pro Ala Ala Ser Gly Gln Asn Pro Ile Gln Arg Thr
            20                  25                  30

Ile Ser Pro Asp Val Arg Thr Tyr Thr Ile Thr
        35                  40

<210>  37
<211>  5
<212>  PRT
<213>  Artificial Sequence

<220>
<223>  fragment B1 of peptide of general formula A1-B1-C1 (I)

<400>  37

Ser Ala Pro Ala Ser
1               5

<210>  38
<211>  8
<212>  PRT
<213>  Artificial Sequence

<220>
<223>  Fragment C1 of peptide of general formula A1-B1-C1 (I)

<220>
<221>  MISC_FEATURE
<222>  (1)..(1)
<223>  Xaa can be N or G or E or D

<220>
<221>  MISC_FEATURE
<222>  (3)..(3)
<223>  Xaa can be Q or E or R or H or V or I or P

<220>
<221>  MISC_FEATURE
<222>  (5)..(5)
<223>  Xaa can be G or A

<220>
<221>  MISC_FEATURE
<222>  (6)..(6)
<223>  Xaa can be S or T or Q or V or M

<220>
<221>  MISC_FEATURE
<222>  (7)..(7)
<223>  Xaa can be E or D

<400>  38

Xaa Leu Xaa Pro Xaa Xaa Xaa Tyr
1               5


<210>  39
<211>  9
<212>  PRT
<213>  Artificial Sequence

<220>
<223>  fragment C of peptide of general formula A-B-C (I)


<220>
<221>  MISC_FEATURE
<222>  (1)..(1)
<223>  Xaa can be N or G or E or D

<220>
<221>  MISC_FEATURE
<222>  (3)..(3)
<223>  Xaa can be Q or E or R or H or V or I or P

<220>
<221>  MISC_FEATURE
<222>  (5)..(5)
<223>  Xaa can be G or A

<220>
<221>  MISC_FEATURE
<222>  (6)..(6)
<223>  Xaa can be S or T or Q or V or M

<220>
<221>  MISC_FEATURE
<222>  (7)..(7)
<223>  Xaa can be E or D

<400>  39

Xaa Leu Xaa Pro Xaa Xaa Xaa Tyr Ser
1               5


<210>  40
<211>  8
<212>  PRT
<213>  Artificial Sequence

<220>

<223>  fragment C1 of peptide of general formula A1-B1-C1 (I)

<400>  40

Asn Leu Gln Pro Ala Ser Glu Tyr
1               5


<210>  41
<211>  9
<212>  PRT
<213>  Artificial Sequence

<220>
<223>  fragment C1 of peptide of general formula A1-B1-C1 (I)

<400>  41

Asn Leu Gln Pro Ala Ser Glu Tyr Ser
1               5


<210>  42
<211>  8
<212>  PRT
<213>  Artificial Sequence

<220>
<223>  Fragment C1 of peptide of general formula A1-B1-C1 (I)

<400>  42

Gly Leu Gln Pro Gly Thr Asp Tyr
1               5


<210>  43
<211>  9
<212>  PRT
<213>  Artificial Sequence

<220>
<223>  fragment C of peptide of general formula A-B-C (I)

<400>  43

Gly Leu Gln Pro Gly Thr Asp Tyr Ser
1               5


<210>  44
<211>  8
<212>  PRT
<213>  Artificial Sequence

<220>
<223>  Fragment C of peptide of general formula A-B-C

<400>  44

Gly Leu Glu Pro Gly Gln Glu Tyr
1               5

<210>  45
<211>  8
<212>  PRT
<213>  Artificial Sequence

<220>
<223>  fragment C of peptide of general formula A-B-C (I)

<400>  45

Gly Leu Arg Pro Gly Thr Glu Tyr
1               5


<210>  46
<211>  8
<212>  PRT
<213>  Artificial Sequence

<220>
<223>  Fragment C of general formula A-B-C

<400>  46

Gly Leu His Pro Gly Thr Glu Tyr
1               5


<210>  47
<211>  8
<212>  PRT
<213>  Artificial Sequence

<220>
<223>  Fragment C of peptide of general formula A-B-C

<400>  47

Gly Leu Arg Pro Gly Val Glu Tyr
1               5


<210>  48
<211>  8
<212>  PRT
<213>  Artificial Sequence

<220>
<223>  fragment C1 of peptide of general formula A1-B1-C1

<400>  48

Gly Leu Lys Pro Gly Glu Glu Tyr
1               5


<210>  49
<211>  8
<212>  PRT
<213>  Artificial Sequence

<220>

<223>  Fragment C1 of peptide of general formula A1-B1-C1

<400>  49

Asp Leu Val Pro Gly Thr Glu Tyr
1               5


<210>  50
<211>  8
<212>  PRT
<213>  Artificial Sequence

<220>
<223>  Fragment C of peptide of general formula A-B-C

<400>  50

Glu Leu Val Pro Glu Thr Glu Tyr
1               5


<210>  51
<211>  8
<212>  PRT
<213>  Artificial Sequence

<220>
<223>  Fragment C1 of peptide of general formula A1-B1-C1 (I)

<400>  51

Asn Leu Gln Pro Asp Thr Ser Tyr
1               5


<210>  52
<211>  8
<212>  PRT
<213>  Artificial Sequence

<220>
<223>  Fragment C1 of peptide of general formula A1-B1-C1 (I)

<400>  52

Gly Leu Leu Pro Asn Thr Glu Tyr
1               5


<210>  53
<211>  8
<212>  PRT
<213>  Artificial Sequence

<220>
<223>  Fragment C1 of peptide of general formula A1-B1-C1 (I)

<400>  53

Gly Leu Glu Pro Gly Thr Glu Tyr
1               5

<210>  54
<211>  8
<212>  PRT
<213>  Artificial Sequence

<220>
<223>  Fragment C1 of peptide of general formula A1-B1-C1

<400>  54

Asp Leu Ile Pro Trp Met Glu Tyr
1               5


<210>  55
<211>  8
<212>  PRT
<213>  Artificial Sequence

<220>
<223>  Fragment C of general A-B-C

<400>  55

Glu Leu Asn Pro Trp Val Glu Tyr
1               5


<210>  56
<211>  8
<212>  PRT
<213>  Artificial Sequence

<220>
<223>  Fragment C1 of general formula A1-B1-C1

<400>  56

Glu Leu Val Pro Gly Arg Leu Tyr
1               5


<210>  57
<211>  8
<212>  PRT
<213>  Artificial Sequence

<220>
<223>  fragment C opf general formula A-B-C (I)

<400>  57

Asp Leu Thr Pro Gly Lys Lys Tyr
1               5


<210>  58
<211>  8
<212>  PRT
<213>  Artificial Sequence

<220>

<223>  fragment C1 of general formula A1-B1-C1 (I)

<400>  58

Gly Leu Val Pro Gly Arg Lys Tyr
1               5


<210>  59
<211>  17
<212>  PRT
<213>  Artificial Sequence

<220>
<223>  peptide P5

<400>  59

Thr Asp Ser Thr Ser Ala Pro Ala Ser Asn Leu Gln Pro Ala Ser Glu
1               5                   10                  15


Tyr


<210>  60
<211>  19
<212>  PRT
<213>  Artificial Sequence

<220>
<223>  S-peptide P5-S

<400>  60

Ser Thr Asp Ser Thr Ser Ala Pro Ala Ser Asn Leu Gln Pro Ala Ser
1               5                   10                  15


Glu Tyr Ser


<210>  61
<211>  17
<212>  PRT
<213>  Artificial Sequence

<220>
<223>  peptide P13

<400>  61

Thr Glu Thr Thr Ser Ala Pro Ala Ser Gly Leu Gln Pro Gly Thr Asp
1               5                   10                  15


Tyr


<210>  62

<211>  19
<212>  PRT
<213>  Artificial Sequence

<220>
<223>  S-Peptide P13-S

<400>  62

Ser Thr Glu Thr Thr Ser Ala Pro Ala Ser Gly Leu Gln Pro Gly Thr
1               5                   10                  15

Asp Tyr Ser

<210>  63
<211>  19
<212>  PRT
<213>  Artificial Sequence

<220>
<223>  peptide S5

<400>  63

Ser Thr Thr Asp Ser Ser Ala Pro Ala Ser Tyr Glu Ser Ala Pro Gly
1               5                   10                  15

Leu Asn Ser

<210>  64
<211>  19
<212>  PRT
<213>  Artificial Sequence

<220>
<223>  peptide S13

<400>  64

Ser Thr Thr Glu Thr Ser Ala Pro Ala Ser Tyr Asp Thr Gly Pro Gln
1               5                   10                  15

Leu Gly Ser

<210>  65
<211>  18
<212>  DNA
<213>  Artificial Sequence

<220>
<223>  primer Ccl21 foward mCCL21_F

<400>  65
tccaagggct gcaagaga                                                    18

<210>  66
<211>  19
<212>  DNA
<213>  Artificial Sequence

<220>
<223>  primer CCl21 reverse mCCL21_R

<400>  66
tgaagttcgt gggggatct                                                    19


<210>  67
<211>  21
<212>  DNA
<213>  Artificial Sequence

<220>
<223>  primer Ccr7 forward mCCR7_F

<400>  67
ctccttgtca ttttccaggt g                                                 21


<210>  68
<211>  21
<212>  DNA
<213>  Artificial Sequence

<220>
<223>  primer Ccr7 reverse mCCR7_R

<400>  68
tggtattctc gccgatgtag t                                                 21


<210>  69
<211>  20
<212>  DNA
<213>  Artificial Sequence

<220>
<223>  primer Cd80 Forward mCD80_F

<400>  69
ccatgtccaa ggctcattct                                                   20


<210>  70
<211>  20
<212>  DNA
<213>  Artificial Sequence

<220>
<223>  primer Cd80 reverse mCD80_R

<400>  70
ggcaaggcag caatacctta                                                   20


<210>  71

<211>  20
<212>  DNA
<213>  Artificial Sequence

<220>
<223>  primer forward Cd86 mCD86_F

<400>  71
catgggcttg gcaatcctta                                                   20


<210>  72
<211>  20
<212>  DNA
<213>  Artificial Sequence

<220>
<223>  primer reverse Cd86 mCD86_R

<400>  72
aaatgggcac gggagatatg                                                   20


<210>  73
<211>  7
<212>  PRT
<213>  Artificial Sequence

<220>
<223>  peptide

<400>  73

Lys Leu Ala Lys Leu Ala Lys
1               5


<210>  74
<211>  4
<212>  PRT
<213>  Artificial Sequence

<220>
<223>  loop I of fibronectine type protein


<220>
<221>  misc_feature
<222>  (1)..(1)
<223>  Xaa can be any naturally occurring amino acid

<220>
<221>  MISC_FEATURE
<222>  (2)..(2)
<223>  Xaa can be D or E

<220>
<221>  MISC_FEATURE
<222>  (3)..(3)
<223>  Xaa can be T or N or S

<220>
<221>  MISC_FEATURE

<222>  (4)..(4)
<223>  Xaa can be T or S

<400>  74

Xaa Xaa Xaa Xaa
1


<210>  75
<211>  8
<212>  PRT
<213>  Artificial Sequence

<220>
<223>  similar loop II consensus sequence


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<220>
<221>  MISC_FEATURE
<222>  (7)..(7)
<223>  Xaa can be E or D

<400>  75

Xaa Leu Xaa Pro Xaa Xaa Xaa Tyr
1               5


<210>  76
<211>  8
<212>  PRT
<213>  Artificial Sequence

<220>
<223>  similar loop II consensus sequence


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<400>  76

Xaa Leu Xaa Pro Xaa Xaa Lys Tyr
1               5


<210>  77
<211>  8
<212>  PRT
<213>  Artificial Sequence

<220>
<223>  similar loop II of M-Motif of PTPRC (CD45)

<400>  77

Asn Leu Lys Pro Tyr Thr Lys Tyr
1               5


<210>  78
<211>  84
<212>  PRT
<213>  Artificial Sequence

<220>
<223>  Human FN5 sequence

<400>  78

Lys Leu Asp Ala Pro Thr Asn Leu Gln Phe Val Asn Glu Thr Asp Ser
1               5                   10                  15


Thr Val Leu Val Arg Trp Thr Pro Pro Arg Ala Gln Ile Thr Gly Tyr
            20                  25                  30


Arg Leu Thr Val Gly Leu Thr Arg Arg Gly Gln Pro Arg Gln Tyr Asn
        35                  40                  45


Val Gly Pro Ser Val Ser Lys Tyr Pro Leu Arg Asn Leu Gln Pro Ala
    50                  55                  60


Ser Glu Tyr Thr Val Ser Leu Val Ala Ile Lys Gly Asn Gln Glu Ser
65                  70                  75                  80


Pro Lys Ala Thr



<210>  79
<211>  84
<212>  PRT
<213>  Artificial Sequence

<220>
<223>  mouse FN5 sequence

<400>  79

Lys Leu Asp Ala Pro Thr Asn Leu Gln Phe Val Asn Glu Thr Asp Arg
1               5                   10                  15

Thr Val Leu Val Thr Trp Thr Pro Pro Arg Ala Arg Ile Ala Gly Tyr
            20                  25                  30

Arg Leu Thr Ala Gly Leu Thr Arg Gly Gly Gln Pro Lys Gln Tyr Asn
        35                  40                  45

Val Gly Pro Leu Ala Ser Lys Tyr Pro Leu Arg Asn Leu Gln Pro Gly
    50                  55                  60

Ser Glu Tyr Thr Val Thr Leu Val Ala Val Lys Gly Asn Gln Gln Ser
65                  70                  75                  80

Pro Lys Ala Thr

<210>  80
<211>  84
<212>  PRT
<213>  Artificial Sequence

<220>
<223>  Rat FN5 sequence

<400>  80

Lys Leu Asp Ala Pro Thr Asn Leu Gln Phe Val Asn Glu Thr Asp Arg
1               5                   10                  15

Thr Val Leu Val Thr Trp Thr Pro Pro Arg Ala Arg Ile Ala Gly Tyr
            20                  25                  30

Arg Leu Thr Val Gly Leu Thr Arg Gly Gly Gln Pro Lys Gln Tyr Asn
        35                  40                  45

Val Gly Pro Met Ala Ser Lys Tyr Pro Leu Arg Asn Leu Gln Pro Gly
    50                  55                  60

Ser Glu Tyr Thr Val Thr Leu Met Ala Val Lys Gly Asn Gln Gln Ser
65                  70                  75                  80

Pro Lys Ala Thr

<210>  81
<211>  84
<212>  PRT
<213>  Artificial Sequence

<220>
<223>  bovin FN5 sequence

<400>  81

Lys Leu Asp Ala Pro Thr Asn Leu Gln Phe Ile Asn Glu Thr Asp Thr
1               5                   10                  15

Thr Val Ile Val Thr Trp Thr Pro Pro Arg Ala Arg Ile Val Gly Tyr
            20                  25                  30

Arg Leu Thr Val Gly Leu Thr Arg Gly Gly Gln Pro Lys Gln Tyr Asn
        35                  40                  45

Val Gly Pro Ala Ala Ser Gln Tyr Pro Leu Arg Asn Leu Gln Pro Gly
    50                  55                  60

Ser Glu Tyr Ala Val Ser Leu Val Ala Val Lys Gly Asn Gln Gln Ser
65                  70                  75                  80

Pro Arg Val Thr



<210>  82
<211>  83
<212>  PRT
<213>  Artificial Sequence

<220>
<223>  human FN13 sequence

<400>  82

Asn Val Ser Pro Pro Arg Arg Ala Arg Val Thr Asp Ala Thr Glu Thr
1               5                   10                  15

Thr Ile Thr Ile Ser Trp Arg Thr Lys Thr Glu Thr Ile Thr Gly Phe
            20                  25                  30

Gln Val Asp Ala Val Pro Ala Asn Gly Gln Thr Pro Ile Gln Arg Thr
        35                  40                  45

Ile Lys Pro Asp Val Arg Ser Tyr Thr Ile Thr Gly Leu Gln Pro Gly
    50                  55                  60

Thr Asp Tyr Lys Ile Tyr Leu Tyr Thr Leu Asn Asp Asn Ala Arg Ser
65                  70                  75                  80

Ser Pro Val

<210> 83
<211> 83
<212> PRT
<213> Artificial Sequence

<220>
<223> mouse FN13 sequence

<400> 83

Asn Val Ser Pro Pro Arg Arg Ala Arg Val Thr Asp Ala Thr Glu Thr
1               5                   10                  15

Thr Ile Thr Ile Ser Trp Arg Thr Lys Thr Glu Thr Ile Thr Gly Phe
            20                  25                  30

Gln Val Asp Ala Ile Pro Ala Asn Gly Gln Thr Pro Val Gln Arg Ser
        35                  40                  45

Ile Ser Pro Asp Val Arg Ser Tyr Thr Ile Thr Gly Leu Gln Pro Gly
    50                  55                  60

Thr Asp Tyr Lys Ile His Leu Tyr Thr Leu Asn Asp Asn Ala Arg Ser
65                  70                  75                  80

Ser Pro Val

<210> 84
<211> 83
<212> PRT
<213> Artificial Sequence

<220>
<223> Rat FN13 sequence

<400> 84

Asn Val Ser Pro Pro Arg Arg Ala Arg Val Thr Asp Ala Thr Glu Thr
1               5                   10                  15

Thr Ile Thr Ile Ser Trp Arg Thr Lys Thr Glu Thr Ile Thr Gly Phe
            20                  25                  30

Gln Val Asp Ala Ile Pro Ala Asn Gly Gln Thr Pro Val Gln Arg Thr
        35                  40                  45

Ile Ser Pro Asp Val Arg Ser Tyr Thr Ile Thr Gly Leu Gln Pro Gly

50                  55                  60

Thr Asp Tyr Lys Ile His Leu Tyr Thr Leu Asn Asp Asn Ala Arg Ser
65                  70                  75                  80

Ser Pro Val

<210>  85
<211>  83
<212>  PRT
<213>  Artificial Sequence

<220>
<223>  bovin FN13 sequence

<400>  85

Asn Val Ser Pro Pro Arg Arg Ala Arg Val Thr Asp Ala Thr Glu Thr
1               5                   10                  15

Thr Ile Thr Ile Ser Trp Arg Thr Lys Thr Glu Thr Ile Thr Gly Phe
            20                  25                  30

Gln Val Asp Ala Ile Pro Ala Asn Gly Gln Thr Pro Ile Gln Arg Thr
        35                  40                  45

Ile Arg Pro Asp Val Arg Ser Tyr Thr Ile Thr Gly Leu Gln Pro Gly
    50                  55                  60

Thr Asp Tyr Lys Ile His Leu Tyr Thr Leu Asn Asp Asn Ala Arg Ser
65                  70                  75                  80

Ser Pro Val

<210>  86
<211>  83
<212>  PRT
<213>  Artificial Sequence

<220>
<223>  chick FN13 sequence

<400>  86

Asn Val Ser Pro Pro Arg Arg Ala Arg Val Thr Asp Ala Thr Glu Thr
1               5                   10                  15

Thr Ile Thr Ile Ser Trp Arg Thr Lys Thr Glu Thr Ile Thr Gly Phe
            20                  25                  30

Gln Val Asp Ala Ile Pro Ala Asn Gly Gln Asn Pro Ile Gln Arg Thr
        35                  40                  45

Ile Arg Pro Asp Val Arg Ser Tyr Thr Ile Thr Gly Leu Gln Pro Gly
    50                  55                  60

Thr Asp Tyr Lys Ile Tyr Leu Tyr Thr Leu Asn Asp Asn Ala Arg Ser
65                  70                  75                  80

Ser Pro Val

<210>  87
<211>  83
<212>  PRT
<213>  Artificial Sequence

<220>
<223>  horse FN13 sequence

<400>  87

Asn Val Ser Pro Pro Arg Arg Ala Arg Val Thr Asp Ala Thr Glu Thr
1               5                   10                  15

Thr Ile Thr Ile Ser Trp Arg Thr Lys Thr Glu Thr Ile Thr Gly Phe
            20                  25                  30

Gln Val Asp Ala Val Pro Ala Asn Gly Gln Pro Pro Ile Gln Arg Thr
        35                  40                  45

Ile Lys Pro Asp Val Arg Ser Tyr Thr Ile Thr Gly Leu Gln Pro Gly
    50                  55                  60

Thr Asp Tyr Lys Ile Tyr Leu Tyr Thr Leu Asn Asp Asn Ala Arg Ser
65                  70                  75                  80

Ser Pro Val

<210>  88
<211>  84
<212>  PRT
<213>  Artificial Sequence

<220>
<223>  Canfl FN13 sequence

<400>  88

Asn Val Ser Pro Pro Arg Arg Ala Arg Val Thr Asp Ala Thr Glu Thr
1               5                   10                  15

Thr Ile Thr Ile Thr Trp Arg Thr Lys Thr Glu Thr Ile Thr Gly Phe
            20                  25                  30

Gln Ile Asp Ala Ile Pro Ala Ala Ser Gly Gln Asn Pro Ile Gln Arg
        35                  40                  45

Thr Ile Ser Pro Asp Val Arg Thr Tyr Thr Ile Thr Gly Leu Gln Pro
    50                  55                  60

Gly Asn Asp Tyr Lys Ile Tyr Leu Tyr Thr Leu Asn Glu Asn Ala Arg
65                  70                  75                  80

Ser Ser Pro Val

<210>  89
<211>  83
<212>  PRT
<213>  Artificial Sequence

<220>
<223>  Xenla FN13 sequence

<400>  89

Asn Val Ser Pro Pro Arg Arg Pro Arg Ile Gln Asp Val Thr Glu Thr
1               5                   10                  15

Thr Val Thr Leu Ser Trp Arg Thr Lys Thr Glu Thr Ile Thr Gly Phe
            20                  25                  30

Gln Ile Asp Ala Ile Pro Ala Asp Gly Gln Asn Pro Ile Arg Arg Thr
        35                  40                  45

Val Asp Ala Asp Leu Arg Thr Phe Thr Ile Thr Gly Leu Gln Pro Gly
    50                  55                  60

Thr Asp Tyr Lys Ile Tyr Leu Tyr Thr Leu Asn Asp Asn Ala Arg Ser
65                  70                  75                  80

Ser Pro Val

<210>  90
<211>  77
<212>  PRT
<213>  Artificial Sequence

<220>
<223>  TN3 sequence

<400>  90

Ala Pro Ser Gln Ile Glu Val Lys Asp Val Thr Asp Thr Thr Ala Leu
1               5                   10                  15

Ile Thr Trp Phe Lys Pro Leu Ala Glu Ile Asp Gly Ile Glu Leu Thr
            20                  25                  30

Tyr Gly Ile Lys Asp Val Pro Gly Asp Arg Thr Thr Ile Asp Leu Thr
        35                  40                  45

Glu Asp Glu Asn Gln Tyr Ser Ile Gly Asn Leu Lys Pro Asp Thr Glu
    50                  55                  60

Tyr Glu Val Ser Leu Ile Ser Arg Arg Gly Asp Met Ser
65                  70                  75

<210>  91
<211>  79
<212>  PRT
<213>  Artificial Sequence

<220>
<223>  TN4 sequence

<400>  91

Ala Pro Arg Asn Leu Arg Arg Val Ser Gln Thr Asp Asn Ser Ile Thr
1               5                   10                  15

Leu Glu Trp Arg Asn Gly Lys Ala Ala Ile Asp Ser Tyr Arg Ile Lys
            20                  25                  30

Tyr Ala Pro Ile Ser Gly Gly Asp His Ala Glu Val Asp Val Pro Lys
        35                  40                  45

Ser Gln Gln Ala Thr Thr Lys Thr Thr Leu Thr Gly Leu Arg Pro Gly
    50                  55                  60

Thr Glu Tyr Gly Ile Gly Val Ser Ala Val Lys Glu Asp Lys Glu
65                  70                  75

<210>  92
<211>  71
<212>  PRT
<213>  Artificial Sequence

<220>
<223>  TN5 sequence

<400>  92

Leu Gln Val Ser Glu Thr Ala Glu Thr Ser Leu Thr Leu Leu Trp Lys

1               5                   10                  15

Thr Pro Leu Ala Lys Phe Asp Arg Tyr Arg Leu Asn Tyr Ser Leu Pro
            20                  25                  30

Thr Gly Gln Trp Val Gly Val Gln Leu Pro Arg Asn Thr Thr Ser Tyr
        35                  40                  45

Val Leu Arg Gly Leu Glu Pro Gly Gln Glu Tyr Asn Val Leu Leu Thr
    50                  55                  60

Ala Glu Lys Gly Arg His Lys
65                  70


<210>  93
<211>  8
<212>  PRT
<213>  Artificial Sequence

<220>
<223>  loop II sequence

<400>  93

Gly Leu Arg Pro Gly Arg Ser Tyr
1               5


<210>  94
<211>  8
<212>  PRT
<213>  Artificial Sequence

<220>
<223>  loop II sequence

<400>  94

Asn Leu Lys Pro Tyr Thr Lys Tyr
1               5


<210>  95
<211>  8
<212>  PRT
<213>  Artificial Sequence

<220>
<223>  loop II sequence

<400>  95

Gly Leu Ser Pro Tyr Ser Asp Tyr
1               5


<210>  96
<211>  8

<212>  PRT
<213>  Artificial Sequence

<220>
<223>  Loop II sequence

<400>  96

Gly Leu Ser Pro Phe Ser Glu Tyr
1               5


<210>  97
<211>  8
<212>  PRT
<213>  Artificial Sequence

<220>
<223>  loop II sequence

<400>  97

Asn Leu Tyr Pro Tyr Cys Asp Tyr
1               5


<210>  98
<211>  8
<212>  PRT
<213>  Artificial Sequence

<220>
<223>  Loop II sequence

<400>  98

Asn Leu Lys Pro Tyr Thr Lys Tyr
1               5


<210>  99
<211>  19
<212>  PRT
<213>  Artificial Sequence

<220>
<223>  putative HBS sequence


<220>
<221>  misc_feature
<222>  (2)..(5)
<223>  Xaa can be any naturally occurring amino acid

<220>
<221>  misc_feature
<222>  (7)..(12)
<223>  Xaa can be any naturally occurring amino acid

<220>
<221>  misc_feature
<222>  (14)..(18)
<223>  Xaa can be any naturally occurring amino acid

<400>  99

Arg Xaa Xaa Xaa Xaa Arg Xaa Xaa Xaa Xaa Xaa Xaa Arg Xaa Xaa Xaa
1               5                   10                  15

Xaa Xaa Arg

<210>  100
<211>  11
<212>  PRT
<213>  Artificial Sequence

<220>
<223>  Putative HBS sequence

<220>
<221>  misc_feature
<222>  (2)..(2)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(10)
<223>  Xaa can be any naturally occurring amino acid

<400>  100

Arg Xaa Lys Arg Xaa Xaa Lys Xaa Xaa Xaa Arg
1               5                   10

<210>  101
<211>  11
<212>  PRT
<213>  Artificial Sequence

<220>
<223>  putative HBS sequence

<220>
<221>  misc_feature
<222>  (2)..(5)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)

<223>  Xaa can be any naturally occurring amino acid

<400>  101

Lys Xaa Xaa Xaa Xaa Lys Xaa Xaa Arg Xaa Arg
1               5                   10


<210>  102
<211>  7
<212>  PRT
<213>  Artificial Sequence

<220>
<223>  putative HBS sequence


<220>
<221>  misc_feature
<222>  (2)..(3)
<223>  Xaa can be any naturally occurring amino acid

<220>
<221>  misc_feature
<222>  (6)..(6)
<223>  Xaa can be any naturally occurring amino acid

<400>  102

Arg Xaa Xaa Lys Arg Xaa Lys
1               5


<210>  103
<211>  6
<212>  PRT
<213>  Artificial Sequence

<220>
<223>  putative HBS sequence


<220>
<221>  misc_feature
<222>  (2)..(2)
<223>  Xaa can be any naturally occurring amino acid

<220>
<221>  misc_feature
<222>  (5)..(5)
<223>  Xaa can be any naturally occurring amino acid

<400>  103

Lys Xaa Arg Lys Xaa Arg
1               5


<210>  104
<211>  12
<212>  PRT
<213>  Artificial Sequence

<220>
<223>  Putative HBS sequence


<220>
<221>  misc_feature
<222>  (2)..(4)
<223>  Xaa can be any naturally occurring amino acid

<220>
<221>  misc_feature
<222>  (7)..(11)
<223>  Xaa can be any naturally occurring amino acid

<400>  104

Lys Xaa Xaa Xaa Arg Arg Xaa Xaa Xaa Xaa Xaa Lys
1               5                   10


<210>  105
<211>  23
<212>  PRT
<213>  Artificial Sequence

<220>
<223>  Putative HBS sequence


<220>
<221>  misc_feature
<222>  (2)..(9)
<223>  Xaa can be any naturally occurring amino acid

<220>
<221>  misc_feature
<222>  (11)..(22)
<223>  Xaa can be any naturally occurring amino acid

<400>  105

Lys Xaa Xaa Xaa Xaa Xaa Xaa Xaa Xaa Arg Xaa Xaa Xaa Xaa Xaa Xaa
1               5                   10                  15


Xaa Xaa Xaa Xaa Xaa Xaa Lys
            20


<210>  106
<211>  4
<212>  PRT
<213>  Artificial Sequence

<220>
<223>  Putative HBS sequence


<220>
<221>  misc_feature
<222>  (2)..(3)
<223>  Xaa can be any naturally occurring amino acid

<400>  106

Arg Xaa Xaa Lys
1


<210>  107
<211>  7
<212>  PRT
<213>  Artificial Sequence

<220>
<223>  Putative HBS sequence


<220>
<221>  misc_feature
<222>  (2)..(2)
<223>  Xaa can be any naturally occurring amino acid

<220>
<221>  misc_feature
<222>  (5)..(6)
<223>  Xaa can be any naturally occurring amino acid

<400>  107

Arg Xaa Lys Lys Xaa Xaa Lys
1               5


<210>  108
<211>  11
<212>  PRT
<213>  Artificial Sequence

<220>
<223>  Putative HBS sequence


<220>
<221>  misc_feature
<222>  (2)..(6)
<223>  Xaa can be any naturally occurring amino acid

<220>
<221>  misc_feature
<222>  (9)..(10)
<223>  Xaa can be any naturally occurring amino acid

<400>  108

Lys Xaa Xaa Xaa Xaa Xaa Lys Lys Xaa Xaa Lys
1               5                   10


<210>  109
<211>  4
<212>  PRT
<213>  Artificial Sequence

<220>
<223>  Putative HBS sequence

<220>
<221>  misc_feature
<222>  (2)..(3)
<223>  Xaa can be any naturally occurring amino acid

<400>  109

Lys Xaa Xaa Lys
1


<210>  110
<211>  5
<212>  PRT
<213>  Artificial Sequence

<220>
<223>  Putative HBS sequence


<220>
<221>  misc_feature
<222>  (2)..(4)
<223>  Xaa can be any naturally occurring amino acid

<400>  110

Lys Xaa Xaa Xaa Lys
1               5


<210>  111
<211>  10
<212>  PRT
<213>  Artificial Sequence

<220>
<223>  Putative HBS sequence


<220>
<221>  misc_feature
<222>  (2)..(4)
<223>  Xaa can be any naturally occurring amino acid

<220>
<221>  misc_feature
<222>  (7)..(9)
<223>  Xaa can be any naturally occurring amino acid

<400>  111

Lys Xaa Xaa Xaa Lys Lys Xaa Xaa Xaa Lys
1               5                   10


<210>  112
<211>  2201
<212>  PRT
<213>  Homo sapiens

<400>  112